# EUROPEAN PATENT APPLICATION

(11) **EP 3 199 139 A1**
(43) Date of publication of application: **02.08.2017**
(21) Application number: 15843749.1
(22) Date of filing: 17.09.2015
(51) Int. Cl.: A61J 3/00, G06Q 50/24

(54) **INSPECTION ASSISTANCE SYSTEM AND TABLET PACKAGING DEVICE**

(30) Priority: 25.09.2014 JP 2014195760; 10.10.2014 JP 2014209496; 28.05.2015 JP 2015109232; 01.07.2015 JP 2015133109
(71) Applicant: Yuyama Mfg. Co., Ltd., Toyonaka-shi Osaka 561-0841 (JP)
(72) Inventor: YUYAMA, Shoji, Toyonaka-shi Osaka 561-0841 (JP); KOIKE, Naoki, Toyonaka-shi Osaka 561-0841 (JP); TANAKA, Tooru, Toyonaka-shi Osaka 561-0841 (JP); SUGIMOTO, TOMOHIRO, Toyonaka-shi Osaka 561-0841 (JP); FUKADA, Masao, Toyonaka-shi Osaka 561-0841 (JP); NISHIO, Masanao, Toyonaka-shi Osaka 561-0841 (JP)
(74) Representative: McWilliams, David John
(86) International application number: PCT/JP2015/076587
(87) International publication number: WO 2016/047569

(57) **Abstract**

An inspection assistance system (1) includes an inspection display processing part (211) which is configured to display, per unit of packing, a capture image of a tablet which is captured before the tablet dispensed from one or both of a tablet cassette (41) and a manual distribution unit (42) based on prescription data is packed into a packing material in a tablet packing device (4), and is configured to display a result of an inspection process which is executed based on the prescription data and identification information of the tablet included in the capture image of the tablet. Thus, it is possible to assist a pharmacist's inspection task carried out with respect to the tablets packed by the tablet packing device (4).

## Description

### TECHNICAL FIELD

The present invention relates to an inspection assistance system and a tablet packing device which assists in the inspection of medicine preparation performed based on prescription data.

### BACKGROUND

In general, there is known a tablet packing device which is provided with a plurality of tablet cassettes for receiving predetermined types of tablets, and which is capable of packing the tablets received in the respective tablet cassettes per dosage unit based on prescription data (*see,* e.g., Patent Document 1). Further, there may be a case where the tablet packing device is provided with a manual distribution unit for dispensing tablets, which are put into a plurality of cells arranged in a matrix shape per dosage unit, on a cell-by-cell basis (*see,* e.g., Patent Document 1).

Patent Document 1: Japanese Patent Application Publication No. 2011-104077

### SUMMARY

In a medical facility such as a hospital or a pharmacy, a pharmacist conducts an inspection task for checking whether the tablets packed by a tablet packing device accurately correspond to the prescription data.

It is an object of the present invention to provide an inspection assistance system and a tablet packing device which are capable of assisting an inspection task of a pharmacist.

An inspection assistance system according to the present invention includes an inspection display processing part which is configured to display, per unit of packing (per unit of one pack), a capture image of a tablet which is captured before the tablet dispensed from one or both of a tablet cassette and a manual distribution unit based on prescription data is packed into a packing material in a tablet packing device, and is configured to display a result of an inspection process which is executed based on the prescription data and identification information of the tablet included in the capture image of the tablet.

According to the present invention, it is possible to assist an inspection task of a pharmacist.

### BRIEF DESCRIPTION OF THE DRAWINGS

FIG. 1 shows the configuration of an inspection assistance system according to an embodiment of the present invention.
FIG. 2 is a schematic diagram showing the configuration of a tablet packing device according to an embodiment of the present invention.
FIG. 3 is a schematic diagram showing the configuration of a tablet packing device according to an embodiment of the present invention.
FIG. 4 shows one example of a tablet rotating part of a tablet packing device according to an embodiment of the present invention.
FIG. 5 shows one example of a tablet rotating part of a tablet packing device according to an embodiment of the present invention.
FIG. 6 shows one example of a packing result performed by a tablet packing device according to an embodiment of the present invention.
FIG. 7 is a flowchart showing one example of a procedure of an inspection assistance process performed by an inspection assistance system according to an embodiment of the present invention.
FIG. 8 shows one example of a display screen displayed by an inspection assistance system according to an embodiment of the present invention.
FIG. 9 shows one example of a display screen displayed by an inspection assistance system according to an embodiment of the present invention.
FIG. 10 shows one example of a display screen displayed by an inspection assistance system according to an embodiment of the present invention.
FIG. 11 shows one example of a display screen displayed by an inspection assistance system according to an embodiment of the present invention.
FIG. 12 shows one example of a display screen displayed by an inspection assistance system according to an embodiment of the present invention.
FIG. 13 shows one example of a display screen displayed by an inspection assistance system according to an embodiment of the present invention.
FIG. 14 shows one example of a display screen displayed by an inspection assistance system according to an embodiment of the present invention.
FIG. 15 shows one example of a display screen displayed by an inspection assistance system according to an embodiment of the present invention.
FIG. 16 shows one example of a display screen displayed by an inspection assistance system according to an embodiment of the present invention.
FIG. 17 shows one example of a display screen displayed by an inspection assistance system according to an embodiment of the present invention.
FIG. 18 shows one example of a display screen displayed by an inspection assistance system according to an embodiment of the present invention.
FIG. 19 shows one example of a display screen displayed by an inspection assistance system according to an embodiment of the present invention.
FIG. 20 shows one example of a display screen displayed by an inspection assistance system according to an embodiment of the present invention.
FIG. 21 shows one example of a display screen displayed by an inspection assistance system according to an embodiment of the present invention.
FIG. 22 shows one example of a display screen displayed by an inspection assistance system according to an embodiment of the present invention.
FIG. 23 shows one example of a display screen displayed by an inspection assistance system according to an embodiment of the present invention.
FIG. 24A shows transition of areas of tablet in capture images captured by an inspection assistance system according to an embodiment of the present invention.
FIG. 24B shows transition of areas of tablet in capture images captured by an inspection assistance system according to an embodiment of the present invention.
FIG. 25 shows one example of a display screen displayed by an inspection assistance system according to an embodiment of the present invention.
FIG. 26 shows one example of a display screen displayed by an inspection assistance system according to an embodiment of the present invention.
FIG. 27 shows one example of a display screen displayed by an inspection assistance system according to an embodiment of the present invention.
FIG. 28 shows one example of a display screen displayed by an inspection assistance system according to an embodiment of the present invention.
FIG. 29 shows one example of a display screen displayed by an inspection assistance system according to an embodiment of the present invention.
FIG. 30 is a flowchart showing one example of a procedure of an information registration process performed by an inspection assistance system according to an embodiment of the present invention.
FIG. 31 shows one example of selection setting information used in an inspection assistance system according to an embodiment of the present invention.
FIG. 32 is a flowchart showing one example of a procedure of a device selection process performed by an inspection assistance system according to an embodiment of the present invention.
FIG. 33A shows one example of a medicine pack dispensed by an inspection assistance system according to an embodiment of the present invention.
FIG. 33B shows one example of a medicine pack dispensed by an inspection assistance system according to an embodiment of the present invention.
FIG. 33C shows one example of a medicine pack dispensed by an inspection assistance system according to an embodiment of the present invention.
FIG. 34 is a flowchart showing one example of a procedure of an automatic inspection process performed by an inspection assistance system according to an embodiment of the present invention.
FIG. 35A shows one example of a display screen displayed by an inspection assistance system according to an embodiment of the present invention.
FIG. 35B shows one example of a display screen displayed by an inspection assistance system according to an embodiment of the present invention.
FIG. 36 shows one example of a display screen displayed by an inspection assistance system according to an embodiment of the present invention.
FIG. 37A shows one example of a display screen displayed by an inspection assistance system according to an embodiment of the present invention.
FIG. 37B shows one example of a display screen displayed by an inspection assistance system according to an embodiment of the present invention.
FIG. 38 shows one example of a display screen displayed by an inspection assistance system according to an embodiment of the present invention.
FIG. 39A shows one example of a display screen displayed by an inspection assistance system according to an embodiment of the present invention.
FIG. 39B shows one example of a display screen displayed by an inspection assistance system according to an embodiment of the present invention.
FIG. 40 shows one example of a display screen displayed by an inspection assistance system according to an embodiment of the present invention.
FIG. 41 shows one example of a display screen displayed by an inspection assistance system according to an embodiment of the present invention.
FIG. 42A shows one example of a display screen displayed by an inspection assistance system according to an embodiment of the present invention.
FIG. 42B shows one example of a display screen displayed by an inspection assistance system according to an embodiment of the present invention.
FIG. 43 shows the configuration of a packing unit of a tablet packing device according to an embodiment of the present invention.
FIG. 44 shows the configuration of a sealing device of a packing unit of a tablet packing device according to an embodiment of the present invention.
FIG. 45 shows the configuration of a sealing device of a packing unit of a tablet packing device according to an embodiment of the present invention.
FIG. 46 shows the configuration of a sealing device of a packing unit of a tablet packing device according to an embodiment of the present invention.
FIG. 47 shows one example of a medicine pack dispensed from a tablet packing device according to an embodiment of the present invention.
FIG. 48 shows the configuration of a packing unit of a tablet packing device according to an embodiment of the present invention.
FIG. 49 shows the configuration of a packing unit of a tablet packing device according to an embodiment of the present invention.
FIG. 50A shows one example of a capture image captured by a tablet packing device according to an embodiment of the present invention.
FIG. 50B shows one example of a capture image captured by a tablet packing device according to an embodiment of the present invention.
FIG. 51 is a flowchart showing one example of a procedure of a packing bag formation method performed by a tablet packing device according to an embodiment of the present invention.
FIG. 52 is a flowchart showing one example of a procedure of a subroutine related to a second transversal seal formation steps performed by a tablet packing device according to an embodiment of the present invention.
FIG. 53 shows one example of a display screen displayed by an inspection assistance system according to an embodiment of the present invention.
FIG. 54 shows one example of a display screen displayed by an inspection assistance system according to an embodiment of the present invention.
FIG. 55 is a diagram for explaining one example of a pattern matching process.
FIG. 56 is a flowchart showing one example of a procedure of an image inspection process performed by an inspection assistance system according to an embodiment of the present invention.
FIG. 57 is a flowchart showing one example of a procedure of a similar medicine registration process performed by an inspection assistance system according to an embodiment of the present invention.
FIG. 58 shows one example of a display screen displayed by an inspection assistance system according to an embodiment of the present invention.
FIG. 59 shows one example of a correct image used in an inspection assistance system according to an embodiment of the present invention.
FIG. 60 shows one example of a correct image used in an inspection assistance system according to an embodiment of the present invention.
FIG. 61A is a diagram for explaining a capsule image processing function of an inspection assistance system according to an embodiment of the present invention.
FIG. 61B is a diagram for explaining a capsule image processing function of an inspection assistance system according to an embodiment of the present invention.
FIG. 61C is a diagram for explaining a capsule image processing function of an inspection assistance system according to an embodiment of the present invention.
FIG. 61D is a diagram for explaining a capsule image processing function of an inspection assistance system according to an embodiment of the present invention.

### DETAILED DESCRIPTION

Hereinafter, descriptions are made as embodiments of the present invention with reference to the accompanying drawings so that the present invention can be understood. It should be noted that the embodiments described below are examples of embodying the present invention and are not intended to limit the technical scope of the present invention. Further, it will be possible to select and arbitrarily combine the configurations and the process functions of the embodiments described below.

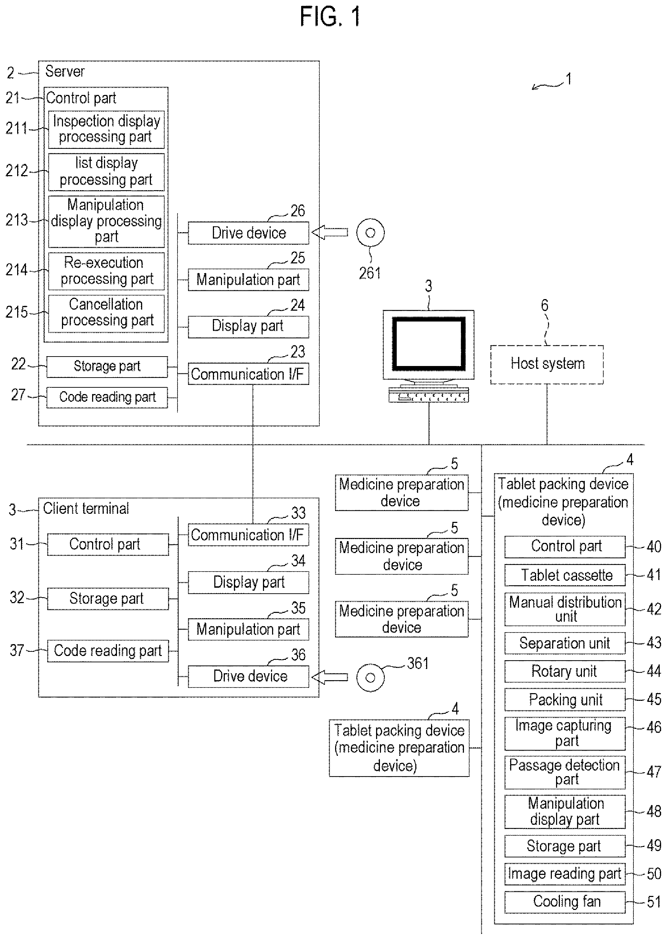

[First Embodiment] As shown in FIG. 1, an inspection assistance system 1 according to an embodiment of the present invention includes a server 2, one or more client terminals 3, one or more tablet packing devices 4 and one or more medicine preparation devices 5. Further, the server 2 alone may be comprehended as an inspection assistance system according to the present invention.

The server 2, the client terminal 3, the tablet packing device 4 and the medicine preparation device 5 are respectively connected via a communication network N1 such as a LAN or the Internet such that they can communicate wiredly or wirelessly. Further, a host system 6 such as an electronic medical record system or a prescription input terminal for inputting prescription data to the server 2 is connected to the server 2 via the communication network N1. It is also conceivable that the server 2 can read prescription data from a prescription slip, or that prescription data can be inputted by a user's manipulation on the server 2.

[Server 2] The server 2 is a personal computer which includes a control part 21, a storage part 22, a communication I/F 23, a display part 24, a manipulation part 25, a drive device 26, a code reading part 27, etc. The server 2 is disposed inside or outside a medical institution in which the inspection assistance system 1 is used.

The control part 21 includes a control device such as a CPU, a ROM, a RAM and an EEPROM (registered trademark). The CPU is a processor that executes various computing processes. The ROM is a non-volatile storage in which information such as a control program for causing the CPU to execute various processes is stored in advance. The RAM is a volatile storage, and the EEPROM is a non-volatile storage. The RAM and the EEPROM are used as temporary storage memories (work areas) of various processes executed by the CPU. Using the CPU, the control part 21 executes various processes according to various control programs stored in advance in the ROM, the EEPROM or the storage part 22.

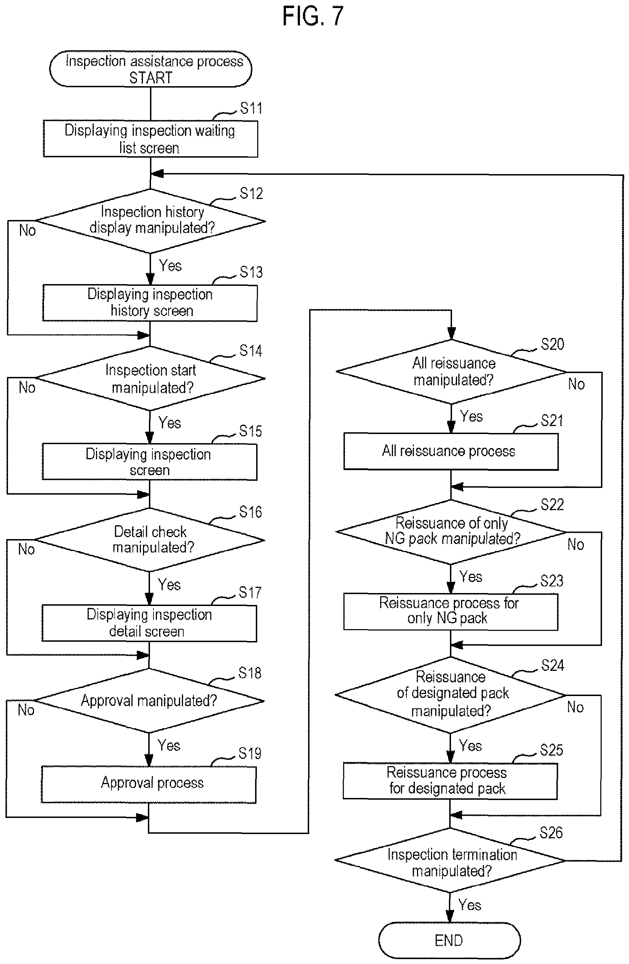

The storage part 22 is a storage such as a hard disk device or a SSD (Solid State Drive) that stores various types of data. Specifically, an inspection assistance program for causing the computer such as the control part 21 or the like to execute an inspection assistance process *(see* FIG. 7), which is described below, is stored in the storage part 22 in advance.

In the storage part 22, for example, various databases such as a medicine master, a patient master, a cassette master and a pharmacy master are also stored. The medicine master includes information on an individual medicine such as a medicine ID, a medicine code, a medicine name, a YJ code, a JAN code (or an RSS code), a medicine bottle code, a classification (dosing type: a powder medicine, a tablet, a liquid medicine, an external use medicine, etc.), a tablet shape (a capsule tablet, a spherical tablet, a flat tablet (disc-shaped tablet), etc.), a tablet size, a specific gravity, a medicine type (ordinary medicines, poisons, narcotics, dangerous medicines, antipsychotics, therapeutic medicines, etc.), a mix change, an excipient, a precaution, etc. The patient master includes information on patients such as a patient ID, a name, a gender, an age, a medical history, a prescribed medicine history, a family information, a clinical department, a ward, a hospital room, etc. The pharmacy master includes information on pharmacies such as a pharmacy name, a pharmacist name, a pharmacist ID, etc. The cassette master is information indicative of a correspondence relationship between a cassette identification information of each of below-described medicine cassettes 41 of the tablet packing device 4 and tablets allocated to each of the medicine cassettes 41.

Further, in the storage part 22, a medicine database, in which information such as a medicine code, a medicine name, a JAN code, an RSS code, a medicine bottle code, a dosing type, a unit, a specific gravity, a medicine type, a mix change, an excipient, precautions, an allergy information, attached document information, etc. is associated with each medicine, is stored separately from the medicine master. Particularly, in the medicine database, information such as a tablet identification information formed in a tablet, a shape of a tablet, an appearance image of a tablet (front and back surfaces), etc. is stored with regard to the tablet. Further, the medicine database is read from a recording medium such as a CD or a DVD by the drive device 26 or received from an external device via the communication network N1, and is stored in the data storage part 22. The medicine database is used in the inspection assistance system 1 when information is inputted to various masters such as the medicine master or the like or when to the attached document information of each medicine is referred to. For example, in the inspection assistance system 1, the medicine database can be displayed in response to a user's manipulation on the server 2, the client terminal 3 or the like. Further, the control part 21 may be configured to read out the medicine database from an external device or a website via the communication network N1 when necessary.

The communication I/F 23 is a communication interface having a network card which executes wired or wireless data communication with external devices such as the client terminal 2, the tablet packing device 4 and the medicine preparation device 5 via the communication network N1 according to a predetermined communication protocol.

The display part 24 is a display such as a liquid crystal monitor or the like which displays various types of information and manipulation screens in response to a control instruction from the control part 21. The manipulation part 25 is a manipulation part such as a keyboard, a mouse, a touch panel or the like which receives a user's manipulation. The manipulation part 25 inputs a manipulation signal corresponding to the user's manipulation to the control part 21. The manipulation part 25 receives various manipulation inputs, such as a prescription data selection manipulation, a prescription data issuance manipulation for requesting the start of medicine preparation of the prescription data, etc., for example, on a display screen displayed in the display part 24.

The drive device 26 is capable of reading the inspection assistance program from a computer-readable recording medium 261 in which the inspection assistance program is recorded. The recording medium 261 is a CD, a DVD, a BD, a USB memory or the like. The drive device 26 is a CD drive, a DVD drive, a BD drive, a USB port or the like. In the server 2, the inspection assistance program read from the recording medium 261 by means of the drive device 26 is stored in the storage part 22 by the control part 21.

The code reading part 27 is a bar code reader which is capable of reading code information (a bar code or a two-dimensional code, etc.). For example, the code reading part 27 is used for reading prescription data from the code information written on a prescription slip. The prescription data read from the prescription slip is stored in the storage part 22 by the control part 21.

In the server 2 configured as described above, the control part 21 includes an inspection display processing part 211, a list display processing part 212, a manipulation display processing part 213 and a re-execution processing part 214. Specifically, the control part 21 executes various types of processes according to the inspection assistance program, and functions as the inspection display processing part 211, the list display processing part 212, the manipulation display processing part 213 and the re-execution processing part 214. Further, the control part 21 also has a function of creating prescription data for medicine preparation (medicine preparation data) for executing a medicine preparation process such as a packing process or the like by the tablet packing device 4 and the medicine preparation device 5 based on the prescription data, and a function of inputting the prescription data to the tablet packing device 4 and the medicine preparation device 5. Thus, in the tablet packing device 4 and the medicine preparation device 5, the medicine preparation process such as a packing process or the like is executed based on the prescription data.

The inspection display processing part 211 displays capture images of a tablet which are captured before the tablet dispensed from the below-described tablet cassette 41 or the below-described manual distribution unit 42 based on the prescription data is packed into a packing material such as a packing paper or the like in the tablet packing device 4, on the client terminal 3 or the like per unit of packing (per unit of one pack). Further, the inspection display processing part 211 displays a result of an inspection process, which is executed based on the prescription data and an identification information (a character or a symbol) of the tablet included in the capture images of the tablet, on the client terminal 3 or the like. For example, in the inspection process, the identification information recognized from the capture images of the tablet by a character recognition process is collated with an identification information of the tablet included in the prescription data. Further, in the inspection process, images of the identification information included in the capture images of the tablet may be collated with a correct image preregistered in association with the identification information of the tablet included in the prescription data.

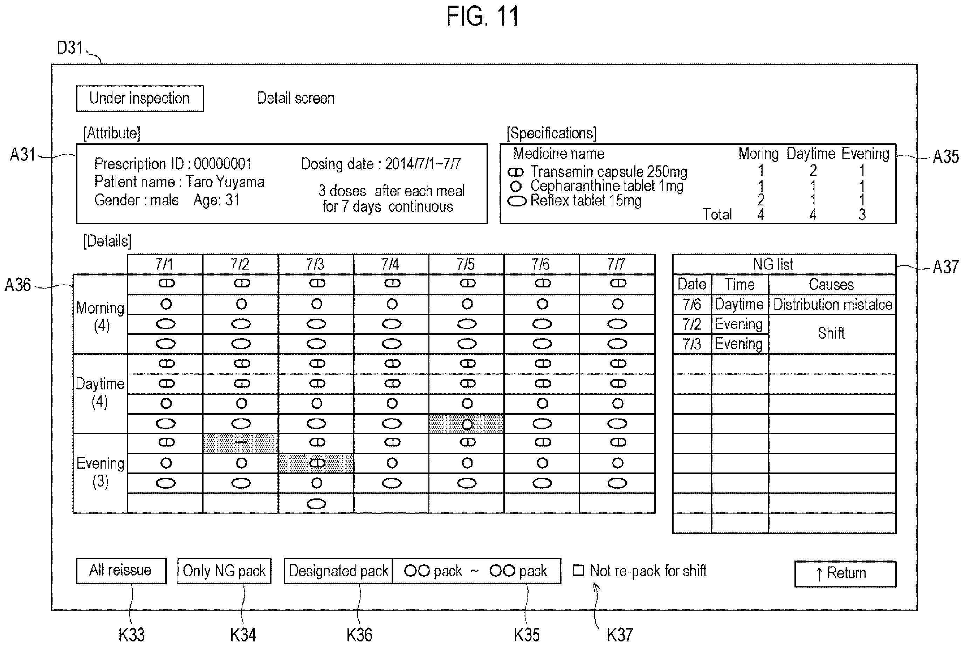

When the result of the inspection process is an error, the list display processing part 212 displays, by a list, a cause of the error together with a dosing date and a dosing time of the tablet which causes the error. For example, the list display processing part 212 causes the client terminal 3 or the like to display an NG list area A37 (*see* FIG. 11) of an inspection detail screen D31 which is described below.

The manipulation display processing part 213 displays a manipulation section for individually re-executing a portion or all of the medicine preparation processes, which are executed by the tablet packing device 4 and the medicine preparation device 5 based on the prescription data, on a first display screen, in which the inspection result of the medicine prepared by the tablet packing device 4 and the medicine preparation device 5 is displayed, or on a second display screen, which is displayed after a predetermined manipulation is made on the first screen. More specifically, in the present embodiment, the manipulation display processing part 213 can display a manipulation key K33, a manipulation key K34, an input field K35 and a manipulation key K36, which are described below, as the manipulation section for re-executing a portion of the entirety of the packing process executed by the tablet packing device 4, on the below-described inspection detail screen D31 (*see* FIG. 11) which is the first display screen in which the result of the inspection process is displayed by the inspection display processing part.

In response to the user's manipulation on the manipulation section displayed by the manipulation display processing part 213, the re-execution processing part 214 causes the tablet packing device 4 and the medicine preparation device 5 to re-execute a portion or all of the medicine preparation process executed by the tablet packing device 4 and the medicine preparation device 5. More specifically, in the present embodiment, in response to the user's manipulation on the manipulation section displayed by the manipulation display processing part 213, the re-execution processing part 214 can cause the tablet packing device 4 to re-execute a portion of the entirety of the packing process executed by the tablet packing device 4.

[Client Terminal 3] Further, the client terminal 3 is a personal computer which includes a control part 31, a storage part 32, a communication I/F 33, a display part 34, a manipulation part 35, a drive device 36, a code reading part 37, etc. Each client terminal 3 is a manipulation terminal which is disposed in a medical institution where the inspection assistance system 1 is used, and which is manipulated by a user such as a pharmacist.

The control part 31 includes control devices such as a CPU, a ROM, a RAM, an EEPROM and the like. The CPU is a processor that executes various computing processes. The ROM is a non-volatile storage in which information such as a control program for causing the CPU to execute various processes is stored in advance. The RAM is a volatile storage, and the EEPROM is a non-volatile storage. The RAM and the EEPROM are used as temporary storage memories (work areas) for various processes executed by the CPU. Using the CPU, the control part 31 executes various processes according to various control programs stored in advance in the ROM, the EEPROM or the storage part 32.

The storage part 32 is a non-volatile storage such as a hard disk or an SSD in which various application programs to be executed by the control part 31 and various data are stored. Specifically, an operating system (OS) and an application program such as browser software or the like are stored in the storage part 32. The browser software is an application software for causing the display part 34 to display various display screens by accessing to the server 2 via the communication network N1 and for transmitting an input manipulation made on the manipulation screens by means of the manipulation part 35 to the server 2. Specifically, when address information such as a URL (Universal Resource Locator) or the like corresponding to the server 2 is inputted to a predetermined location on the manipulation screen displayed by the browser software, the control part 31 accesses to the server 2 based on the address information.

The communication I/F 33 is a communication interface including a network card which executes wired or wireless data communication with external devices such as the server 2 or the like via the communication network N1 according to a predetermined communication protocol.

The display part 34 is a display such as a liquid crystal display or an organic EL display which displays various types of information in response to a control instruction from the control part 31. The manipulation part 35 is a manipulation part which is manipulated by a user to input various types of information to the client terminal 3. Specifically, the manipulation part 35 includes a keyboard and a mouse (pointing device) which receive input manipulations made on various manipulation screens displayed in the display part 34. Further, the manipulation part 35 may include a touch panel which receives touch manipulations made on various manipulation screens displayed in the display part 34, or a voice input device which receives input of various types of information by voice recognition.

The drive device 36 is capable of reading the OS or the browser software from a computer-readable recording medium 361 in which the OS or the browser software is recorded. The recording medium 361 is a CD, a DVD, a BD, a USB memory, or the like. The drive device 36 is a CD drive, a DVD drive, a BD drive, a USB port, or the like. In the client terminal 3, the OS, the browser software or the like read from the recording medium 361 by means of the drive device 36 is stored in the storage part 32 by the control part 31.

The code reading part 37 is a bar code reader capable of reading code information (a bar code or a two-dimensional code, etc.). For example, the code reading part 37 is used to read the code information written in a medicine pack 451.

In the inspection assistance system 1, the server 2 and the client terminal 3 constitutes a sever client system. Descriptions are made as to that the server 2 executes various processes in response to the user's manipulation on the client terminal 3. Therefore, "display", "manipulation", "selection", "input", etc, which are described below, are performed by using the display part 34 and the manipulation part 35 of the client terminal 3. For example, the control part 21 of the server 2 causes the client terminal 3 to display various screens by transmitting data described with a page description language such as a HTML to the client terminal 3.

Further, it is conceivable that a portion or all of the inspection assistance programs is installed in any one or more of the server 2, the client terminal 3 and the tablet packing device 4, and that the below-described inspection assistance process is performed through the cooperation of the server 2, the client terminal 3, the tablet packing device 4 and the like.

Further, in another embodiment, it is also conceivable that an inspection assistance system having an inspection assistance function for executing the below-described inspection assistance process (*see* FIG. 7) is provided in the inspection assistance system 1 separately from the server 2. Such an inspection assistance system is an information processing device such as, for example, a personal computer or a tablet terminal, and is capable of making communication with the server 2, the client terminal 3, the tablet packing device 4, the medicine preparation device 5 and the like provided in the inspection assistance system 1. Further, it is also conceivable that such an inspection assistance system is installed in a medicine preparation device such as the tablet packing device 4.

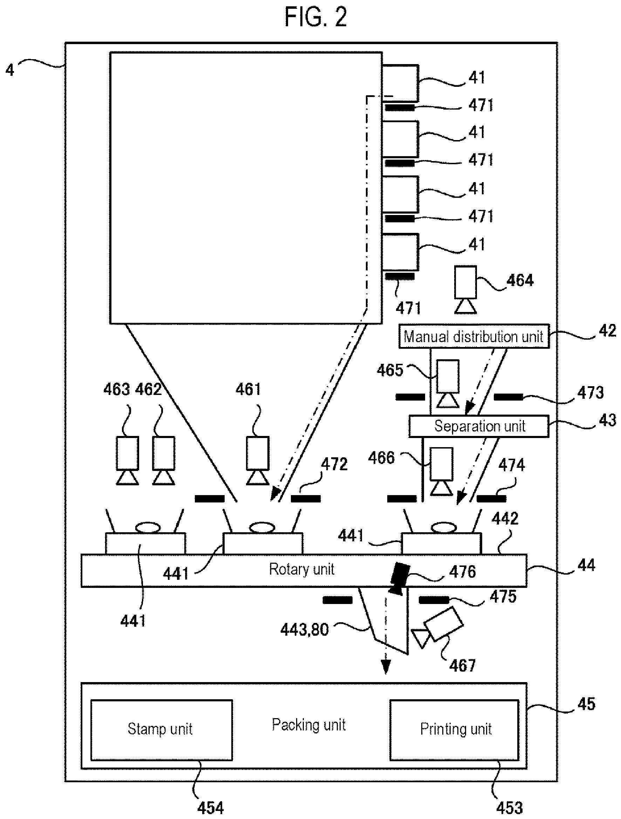

[Tablet Packing Device 4] The tablet packing device 4 is a medicine preparation device used for preparing a medicine. Specifically, as shown in FIGS. 1 and 2, the tablet packing device 4 includes a control part 40, a tablet cassette 41, a manual distribution unit 42, a separation unit 43, a rotary unit 44, a packing unit 45, an image capturing part 46, a passage detection part 47, etc. The tablet packing device 4 is capable of automatically dispensing tablets from either or both of the tablet cassette 41 and the manual distribution unit 42 based on the prescription data and packing the tablets per unit of one dosage. In FIG. 2, a one-dot chain line shows a movement path of tablets.

The image capturing part 46 includes cameras 461 to 467 which are provided at a tablet movement path from the tablet cassette 41 to the packing unit 45 and at a tablet movement path from the manual distribution unit 42 to the packing unit 45. The cameras 461 to 467 are used for capturing an image of one tablet or each of a plurality of tablets before the tablet dispensed from the tablet cassette 41 or the manual distribution unit 42 is packed into a packing paper by the packing unit 45. Further, the images captured by the cameras 461 to 467 are color or monochrome. By the control part 40, the capture images of the tablet obtained by the cameras 461 to 467 are stored in the storage part 49, which is a storage device such as a hard disk provided in the tablet packing device 4, and are transmitted to the server 2.

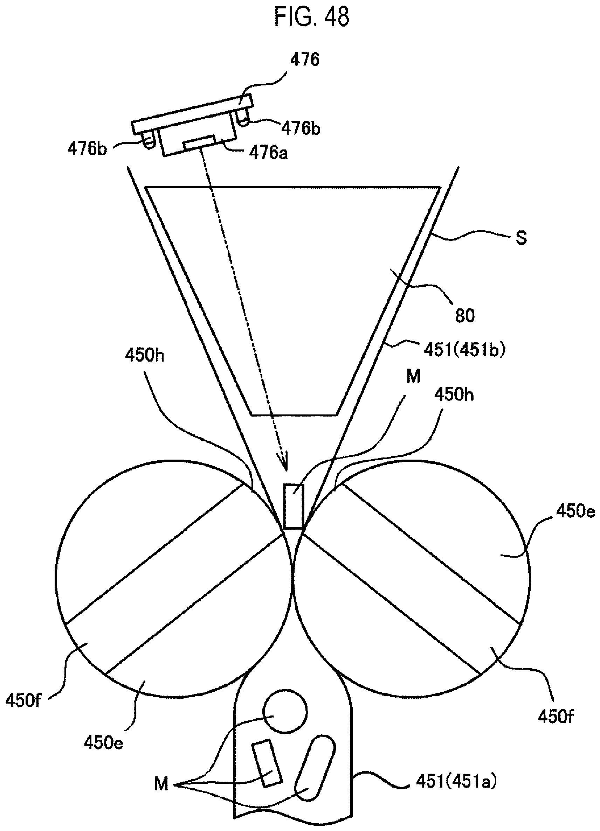

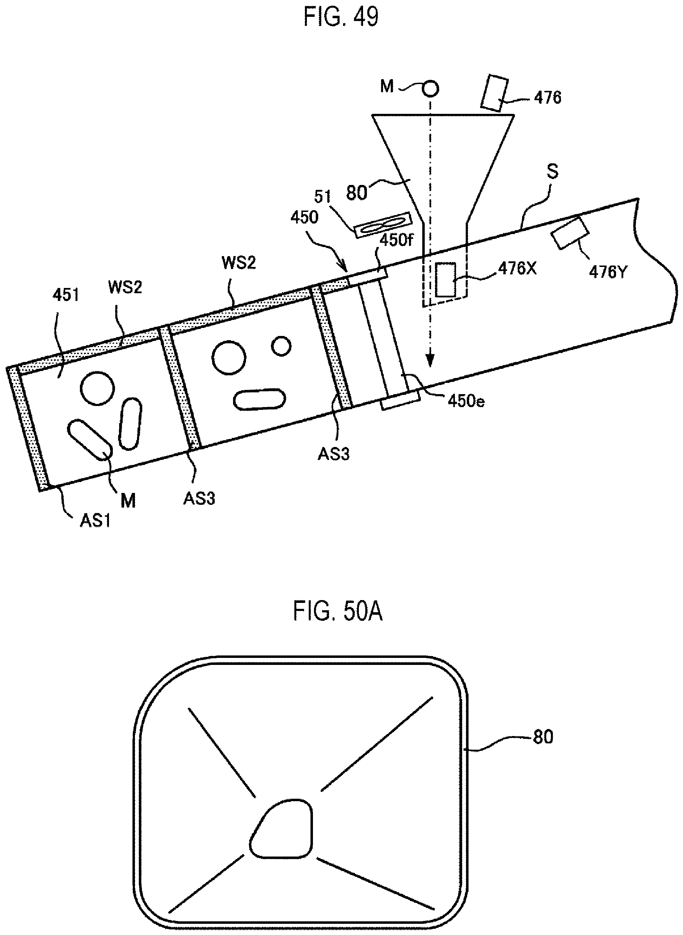

As shown in FIG. 2, the camera 461 is used to capture images of the tablets supplied from the tablet cassette 41 to the rotation unit 44. The camera 462 and the camera 463 are used to capture images of a plurality of different regions (for example, front and back surfaces) of an outer periphery of the tablet from the tablet which is rotated in a below-described tablet rotation part 441 provided in the rotary unit 44. The camera 464 is used to capture images of the tablets received in the manual distribution unit 42. The camera 465 is used to capture images of the tablets supplied from the manual distribution unit 42 to the separation unit 43. The camera 466 is used to capture images of the tablets supplied from the separation unit 43 to the rotary unit 44. The camera 467 is used to capture images of the tablets of a unit of one dosage (a unit of packing) which are retained in a below-described retention part 443 provided in the rotary unit 44. Further, in case where the tablet packing device 4 does not include the below-described retention part 443, the camera 467 is used to capture images of the tablets dropping from the rotary unit 44 during the dropping of the tablets. Further, instead of the retention part 443, a medicine introduction part 80 *(see* FIGS. 48 and 49) may be provided for guiding the tablets dropping from the rotary unit 44 into the packing material located therebelow. In this case, it is conceivable that the camera 467 is disposed in a position where the camera 467 is capable of capturing an image of an image capturing range including the medicine introduction part 80, the tablets, and the packing material, instead of the tablets retained in the retention part 443.

Further, the passage detection part 47 includes passage detection sensors 471 to 475 such as an optical sensor which are configured to detect passage of the tablets in the tablet movement path from the tablet cassette 41 to the packing unit 45 and in the tablet movement path from the manual distribution unit 42 to the packing unit 45. Tablet detection signals generated by the passage detection sensors 471 to 475 are inputted to the control part 40.

As shown in FIG. 2, the passage detection sensor 471 detects the tablets dispensed from the tablet cassette 41, and the passage detection sensor 472 detects the tablet dropping from the tablet cassette 41 to the rotary unit 44. Further, the passage detection sensor 473 detects the tablet dispensed from the manual distribution unit 42, and the passage detection sensor 474 detects the tablet dropping from the separation unit 43 to the rotary unit 44. Further, the passage detection sensor 475 detects the tablet dropping from the below-described tablet rotation part 441 into the below-described retention part 443 in the rotary unit 44.

The control part 40 includes a processor such as a CPU and a storage part such as a RAM and an EEPROM, and globally controls the tablet packing device 4. Specifically, the control part 40 controls the operation of the tablet packing device 4 based on the prescription data inputted from the server 2, thereby causing the tablet packing device 4 to perform a packing process in which one or more types of tablets corresponding to the prescription data are packed per each dosing time.

Further, the control part 40 executes an image capturing process in which an image is captured by the image capturing part 46 in response to a tablet detection timing of the passage detection part 47. For example, when the tablet dropping from the tablet cassette 41 into the tablet rotation part 441 is detected by the passage detecting sensor 472, image capturing is performed by the camera 461. Further, the image capturing of the camera 462 and the camera 463 is performed at a pre-set image capturing interval (several milliseconds) during the execution of the packing process in the tablet packing device 4. On the other hand, the image capturing of the camera 464 is performed, for example, when a manipulation input is made to the effect that a task of manually distributing tablets to the manual distribution unit 42 is completed. Further, when the tablet dispensed from the manual distribution unit 42 is detected by the passage detection sensor 473, image capturing is performed by the camera 465. When the tablet dropping from the separation unit 43 into the tablet rotation part 441 is detected by the passage detection sensor 474, image capturing is performed by the camera 466. Further, when the tablets corresponding to one dosage are detected by the passage detection sensor 475, image capturing is performed by the camera 467. Further, in case where the tablet packing device 4 does not include the retention part 443, whenever the tablet dropping from the rotary unit 44 is detected by the passage detection sensor 475, image capturing is performed by the camera 467 and the tablet dropping from the rotary unit 44 is captured. Further, the image capturing timings of the cameras 461 to 467 are not limited the aforementioned timings and may be set in advance depending upon the use of the respective capture images or the like. Further, in the tablet packing device 4, the control part 40 can determine the progress of the tablets dispensed from the tablet cassette 41 or the manual distribution unit 42 toward the packing unit 45 based on the detection results obtained by the passage detecting sensors 471 to 475. Therefore, for example, when the dispensing of the tablet is not performed normally, the control part 40 can decide a position where the tablet has moved, and can notify (indicate) the user of the position of the tablet. Further, in the tablet packing device 4, it is conceivable that an exterior member on the tablet movement path is formed of transparent or translucent member such that the movement situation of the tablet can be visually recognized from the outside.

The control part 40 can execute an automatic inspection process based on the prescription data and the capture images of the tablet captured by the camera 462 or the camera 463. In the automatic inspection process, when the identification information of the tablet included in the capture images of the tablet is recognized, it is checked whether the identification information of the tablet is matched with the medicine information included in the prescription data. As such, the control part 40 inspects whether the packing process has been properly performed, based on the capture images captured by the camera 462 or the camera 463 before the tablets are packed into the medicine pack 451 in the packing process. In this regard, when the image inspection process of collating the identification information of the tablet included in the capture images with the prescription data is executed as the automatic inspection process of automatically inspecting whether the packing process is proper or not, the control part 40 is one example of an image inspection processing part. Further, as a method of acquiring the identification information of the tablet from the capture images of the tablet in the automatic inspection process, various conventional techniques such as, for example, a technique of reading the identification information by a pattern matching process are used. If a collation result is coincidence, it is decided that the packing process is proper. If the collation result is non-coincidence, it is decided that the packing process is an error.

Further, when the automatic inspection process is executed using the identification information of the tablet acquired from the capture images of the tablet, the control part 40 transmits an original image, in which the identification information is read from the tablet among the capture images of the tablet captured by the camera 462 or the camera 463, to the server 2 together with the result of the automatic inspection process (coincidence or an error). Further, in the automatic inspection process, it is conceivable that the control part 40 extracts a region of the tablet from the capture images captured by the camera 462 or 463 and reads the identification information of the tablet based on extracted trimming images. In this case, the control part 40 transmits the trimming images to the server 2 as the original images, instead of or together with the capture images.

The tablet cassette 41 is a cassette in which a predetermined type of tablet is received. The tablet packing device 4 is provided with a plurality of tablet cassettes 41. In the tablet packing device 4, the tablets included in the prescription data are automatically dispensed from the tablet cassettes 41.

The manual distribution unit 42 includes cells provided in a matrix shape. The tablets such as a half-tablet, which are unsuitable for being dispensed from the tablet cassette 41, are put into each of the cells. The manual distribution unit 42 can dispense the tablets received in the respective cells on a cell-by-cell basis. The separation unit 43 can separate the tablets dispensed from the manual distribution unit 42 one tablet by one tablet, and can supply the tablet to the rotary unit 44. Further, various configurations of the separation unit 43 are conceivable. For example, the separation unit may be configured such that tablets placed on a V-shaped groove portion in an aligned state are supplied one tablet by one tablet from a distal end of the groove portion by vibrating the groove portion and conveying the tablet.

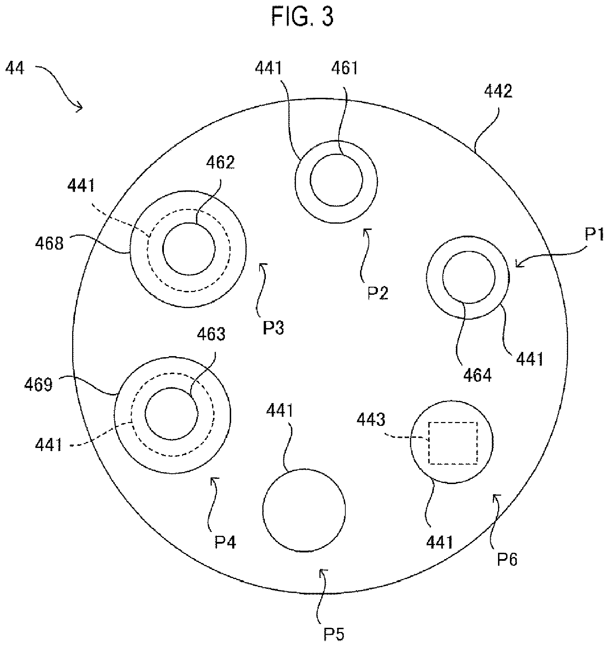

As shown in FIGS. 2 and 3, the rotary unit 44 includes six tablet rotation parts 441, a unit rotation part 442 and a retention part 443. The unit rotation part 442 is rotatably supported by a base (not shown). FIG. 3 is a schematic diagram showing the rotary unit 44 which is viewed from above.

Each of the tablet rotation parts 441 can change a position of the tablet by rotating one tablet supplied from the tablet cassette 41 or the manual distribution unit 42. In the unit rotation part 442, the six tablet rotation parts 441 are arranged at a spacing of 60° around a predetermined rotation axis. The unit rotation part 442 can rotate the tablet rotation parts 441 about a predetermined rotation axis. Specifically, the unit rotation part 442 can sequentially move each of the tablet rotation parts 441 to the following six locations: a drop position P1 where the tablet is dropped from the separation unit 43; a drop position P2 where the tablet is dropped from the tablet cassette 41; an image-capturable position P3 where image capturing can be performed by the camera 462; an image-capturable position P4 where image capturing can be performed by the camera 463; a preliminary position P5; and a drop position P6 where the tablet is dropped into the retention part 443.

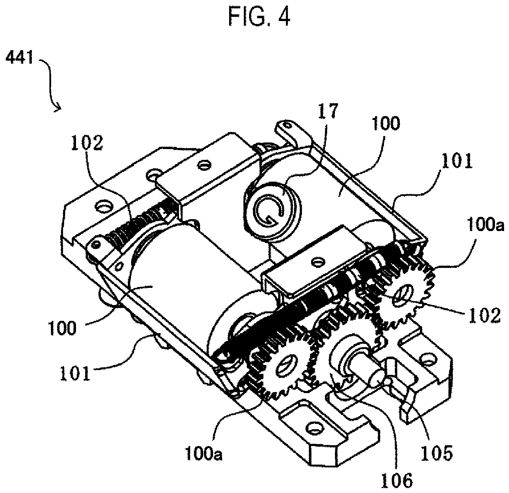

In the tablet packing device 4, as shown in FIG. 4, when one tablet 17 dispensed from the tablet cassette 41 drops into the tablet rotation part 441, or when one tablet 17 dispensed from the separation unit 43 drops into the tablet rotation part 441, the tablet rotation part 441 is rotated by 60° by the unit rotation part 442. Thus, each tablet dispensed from the tablet cassette 41 or the manual distribution unit 42 is sequentially moved from the drop position P1 or the drop position P2 toward the drop position P6 in the state where the tablet is individually placed on the tablet rotation part 441. Then, in the image-capturable position P3 and the image-capturable position P4, the tablet 17 is captured by the camera 462 and the camera 463.

Thereafter, the tablet 17 placed on the tablet rotation part 441 is dropped from the drop position P6 into the retention part 443. The retention part 443 is used to temporarily retain the tablets of a unit of one dosage during the dropping of the tablets from the rotary unit 44 to the packing unit 45. Then, after the tablets of a unit of one dosage are retained in the retention part 443, the bottom surface of the retention part 443 is opened and the tablets of a unit of one dosage received in the retention part 443 are supplied to the packing unit 45.

As shown in FIG. 3, an illumination device 468 and an illumination device 469 for illuminating the tablets in the image-capturable positions of the camera 462 and the camera 463 are fixed above the rotary unit 44. The illumination device 468 and the illumination device 469 irradiate light to the tablet rotation parts 441 at different angles or with different illumination intensities such that images with different illumination environments are captured by the camera 462 and the camera 463. For example, the illumination device 468 performs illumination suitable for reading the identification information of the tablet formed on the tablet by an engraving. For example, it is conceivable that the illumination device 468 irradiates light on the tablet from a lateral side or an oblique upper side so that the identification information of the tablet is clearly captured. Further, the illumination device 468 may irradiate light on the tablet in a plurality of directions. This is because when the identification information of the tablet formed on the tablet is an engraving, concavities and convexities are created on the surface of the tablet due to the engraving and the side at which the identification information is captured in the tablet capture image is changed depending on a light irradiation angle on concavities and convexities. In this regard, the camera 462 and the illumination device 468 are one example of a first image capturing unit. Further, the illumination device 469 performs illumination suitable for reading the identification information of the tablet formed on the tablet by printing. For example, it is conceivable that the illumination device 469 irradiates light on the tablet from above so that the identification information of the tablet is clearly captured. This is because, when the identification information of the tablet formed on the tablet is a print, the identification information is formed on the surface of the tablet with a paint or a developer, and because concavities and convexities are not created on the tablet and the side at which the identification information is captured in the tablet capture image hardly changed. In this regard, the camera 463 and the illumination device 469 are one example of a second image capturing unit. Further, it is pre-registered in the medicine master or the like whether the identification information of the tablet such as a character or symbol formed on the tablet is an engraving or a print. It is conceivable that the medicine master is stored not only in the storage part 22 of the server 2 but also in the storage part 49 provided in the tablet packing device 4. For example, in the medicine master, it is conceivable that an engraving flag is set to "1" with respect to the tablet whose identification information is an engraving, and that a print flag is set to "1" with respect to the tablet whose identification information is a print. Further, in the medicine master, the engraving flag may be set to "1" with respect to the tablet whose identification information is an engraving, and the printing flag may be set to "0" with respect to the tablet whose identification information is a print. Then, in the tablet packing device 4, the capture images which are suitable for the method of forming the identification information of the tablet (engraving or printing) among the images captured by the camera 462 and the camera 463 are used for the automatic inspection process. Further, as described above, the method of forming the identification information includes, for example, an engraving method wherein the identification information is formed on the surface of the tablet through concavities and convexities and a printing method wherein the identification information is formed by coating a paint or a developer on the surface of the tablet as described above. Further, among the images captured by the camera 462 and the camera 463, images having a high coincidence degree in a pattern matching when the identification information of the tablet is recognized, or images having the clearly-captured tablet identification information may be used for the automatic inspection process.

Further, in the storage part 49, similar to the server 2, a medicine database, in which information such as a medicine code, a medicine name, a JAN code, an RSS code, a medicine bottle code, a dosing type, a unit, a specific gravity, a medicine type, a mix change, an excipient, precautions, allergy information, attached document information and the like is associated with each medicine, is stored separately from the medicine master. Particularly, as for a tablet, information such as a tablet identification information formed in the tablet, a shape of the tablet and appearance images of the tablet (front and back surfaces) are stored in the medicine database. Further, the medicine database is read from a recording medium such as a CD or a DVD, for example, by a drive device (not shown) provided in the tablet packing device, or is received from an external device such as the server 2 via the communication network N1, and is stored in the storage part 49. Further, the control part 40 may be configured to read out the medicine database from an external device such as the server 2 or a website via the communication network N1 in response to need.

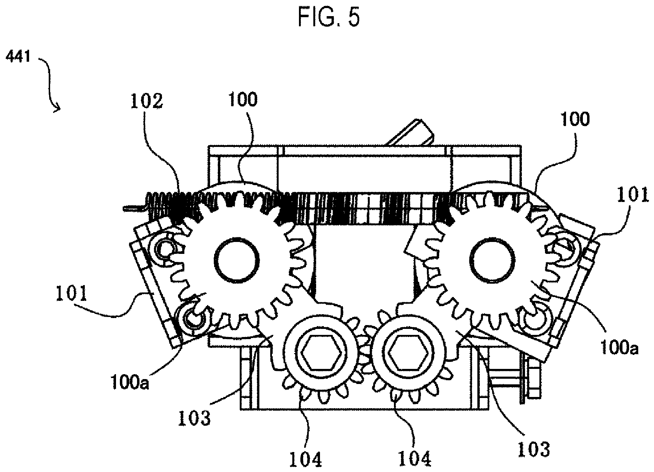

Descriptions are made as to one example of the tablet rotation part 441 with reference to FIGS. 4 and 5. As shown in FIGS. 4 and 5, the tablet rotation part 441 includes a pair of rotating rollers 100, a pair of support plates 101 supporting the rotating rollers 100 respectively, and a spring 102 biasing the support plates 101 in such a direction that the support plates 101 move toward each other. Further, in the tablet rotation part 441, arms 103 extend from end portions of the respective support plates 101, and gears 104 meshing with each other are formed in leading end portions of the arms. Thus, in a normal state, the rotating rollers 100 are kept in an approached state and the tablet 17 can be supported by the rotating rollers 100. Further, the rotating rollers 100 are synchronously moved toward or away from each other by rotation of the gears 104. In a state where the rotating rollers 100 are in contact with or close to each other, the medicine 17 is rotatably supported by the rotating rollers 100. In a state where the rotating rollers 100 are spaced apart from each other, the medicine 17 is dropped from the rotating rollers 100 toward the retention part 443.

Further, a driven gear 100a is integrally formed with one end portion of a rotation shaft of each of the rotating rollers 100. A driving gear 106 integrally formed with one end of a drive shaft 105 meshes with each driven gear 100a. A driven roller (not shown) is integrally formed with the other end of the drive shaft 105. The driven roller is comprised of a magnet gear. Further, in the rotary unit 44, when the tablet rotation part 441 is moved to the image-capturable position P3 of the camera 462 or the image-capturable position P4 of the camera 463, a coupling magnet gear (not shown) connected to a predetermined drive motor is provided in a position located immediately below the tablet rotation part 441 and spaced apart from the tablet rotation part 441.

When the tablet rotation part 441 is moved to the image-capturable position P3 of the camera 462 or the image-capturable position P4 of the camera 463, a drive force of the predetermined drive motor is transmitted to the driven roller through the coupling magnet gear. Thus, the drive force of the predetermined drive motor is transmitted to a pair of the rotating rollers 100 through the driven roller and the drive shaft 105, whereby the rotating rollers 100 are synchronously rotated in the same direction. Therefore, when the tablet 17 is placed on the rotating rollers 100, the tablet 17 is rotated. Accordingly, in the tablet rotation part 441, the position of the tablet 17 to be captured by the camera 462 and the camera 463 can be changed and the outer peripheral surface of the tablet 17 can be captured in different directions. That is to say, the tablet 17 rotated by the tablet rotation part 441 is intermittently or continuously captured by the camera 462 and the camera 463, and therefore an image of the outer peripheral surface, which includes the identification information formed on the tablet 17 by an engraving or the like, can be captured.

The above-described configuration of the tablet rotation part 441 is only illustrative. The tablet rotation part 441 may have any configuration as long as it can displace the tablet into a state where images with the readable tablet identification information can be captured by the camera 462, the camera 463, etc. However, it is preferable that those images are captured such that the identification information of the tablet clearly appears in the capture images. Further, the tablet packing device 4 may be configured such that an image of a tablet is captured from a front surface and a back surface of a transparent loading plate in a state where the tablet is placed on the loading plate, as long as the image of the outer peripheral surface of the tablet can be captured in different directions.

Further, it is conceivable that the control part 40 compares all of the capture images with the correct image associated with the tablet, or reads the identification information of the tablet from all of the capture images. However, there is a concern that such a process increases the load or reduces the reading accuracy of the identification information of the tablet. On the other hand, in case the tablet is a flat tablet, if the capture images of the front and the back surfaces of the flat tablet, which are captured by the camera 462 or the camera 463 in a direction perpendicular to the front surface and the back surface of the flat tablet, are compared with the correct image, or if the identification information of the tablet is read from the capture images, then it is possible to enhance the accuracy of the automatic inspection process. This is because the identification information of the tablet is located on one or both of the front and back surfaces of the tablet in case the tablet is the flat tablet. Thus, in case the tablet is the flat tablet, it is conceivable that the control part 40 specifies a capture image to be compared with the correct image or a capture image to be used for acquiring the identification information of the tablet, among a plurality of the capture images, according to an area of the tablet included in a plurality of the capture images.

First, the control part 40 determines whether the tablet is the flat tablet, according to transition of the areas of tablet in the respective capture images. For example, it is conceivable that the tablet is determined to be a flat tablet when a ratio of a maximum value and a minimum value of the areas of the tablet in the respective capture images is equal to or greater than a preset threshold value, i.e., when a variation of the areas of the tablet is large. Further, the control part 40 may determine whether the tablet is a flat tablet, based on the medicine master or the medicine database. When it is determined that the tablet is a flat tablet, the control part 40 specifies a capture image, which is captured between two capture images having the largest area of the tablet and which has the area of the tablet equal to or greater than the predetermined threshold value, among a plurality of the capture images.

For example, it is conceivable that the control part 40 specifies the capture image, which precedes, by one image, the capture image in which the transition (inclination) of the areas of the tablets changes from an increment to a decrement when the areas of the tablet in the respective capture images are arranged in an image capturing order of the respective capture images, as the capture image having the largest area of the tablet. Thus, two capture images having the largest area of the tablet are specified from a plurality of the capture images corresponding to one turn of the outer peripheral surface of a tablet. In other words, the two capture images having the largest area of the tablet are the images having two high-ranked areas of the tablet among the capture images which are captured at a predetermined image capturing interval and correspond to the one turn of the outer peripheral surface of a tablet. Further, between the two capture images having the largest area of the tablet, the capture image, which has the area of the tablet equal to or greater than the threshold value and which precedes, by one image, the capture image in which the transition (inclination) of the areas of the tablet changes from a decrement to an increment, is specified as an image obtained by capturing the front or back surface of the tablet from a front side.

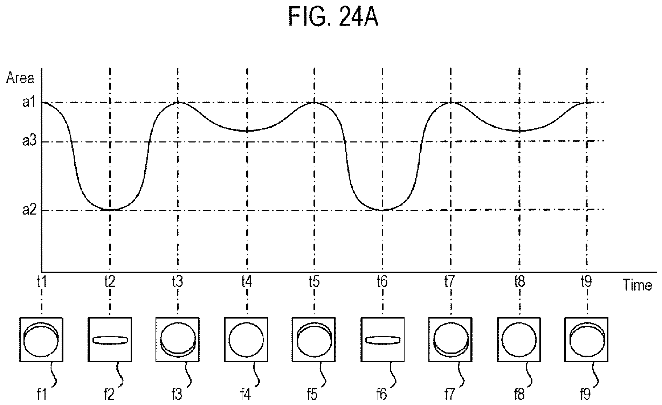

FIG. 24A shows the transition of the areas of the tablet in case where the flat tablet (the tablet) rotated by the tablet rotation part 441 is continuously captured. As shown in FIG. 24A, the areas of the tablet in the respective capture images transit between an area a1 having a maximum value and an area a2 having a minimum value along with the rotation of the tablet on the tablet rotation part 441. Specifically, when the front or back surface to which the identification information of the tablet is added is slightly inclined with respect to the camera 462 or the camera 463 of the image capturing part 46, and when the front or back surface of the tablet and the side surface of the tablet are captured together, the area of the tablet in the capture image becomes the maximum area a1. Further, when only the side surface of the tablet is captured, the area of the tablet in the capture image becomes the minimum area a2. While capture images f1 to f9 shown in FIG. 24A are taken as examples in the descriptions made herein, one or a plurality of images may be also captured between the respective capture images f1 to f9 in the tablet packing device 4.

As shown in FIG. 24A, the front or back surface of the tablet and a portion of the side surface of the tablet are included in the capture images f1, f3, f5, f7 and f9 corresponding to the times t1, t3, t5, t7 and t9 at which the area of the tablet becomes the maximum area al. Further, only the side surface of the tablet is included in the capture images f2 and f6 corresponding to the times t2 and t6 at which the area of the tablet becomes the minimum area a2. Further, the front or back surface of the tablet is included in the capture images f4 and f8 corresponding to the times t4 and t8 at which the area of the tablet is less than the area a1 and is equal to or greater than an area a3 predetermined with respect to each type of tablet. That is to say, one of the capture images f4 and f8 is an image obtained by capturing the front surface of the tablet from a front side and the other is an image obtained by capturing the back surface of the tablet from a front side. The area a3 is a value less than the area a1 and greater than the area a2, and is a value set in advance as a value serving as an index for distinguishing the area of the front or back surface of the tablet from the area of the side surface of the tablet. For example, the area a3 is a value which is less by a predetermined value than the area obtained by capturing the front or back surface of the tablet from a front side.

Then, the control part 40 extracts a capture image having the smallest area of the tablet among the capture images which are captured between the two capture images having the area of the tablet of the maximum area a1, and whose areas of tablet are less than the area a1 and equal to or greater than the area a3. In this regard, the control part 40 specifies the odd-numbered capture image among the extracted capture images as the capture image f4, and specifies the even-numbered capture image among the extracted capture images as the capture image f8. Further, the control part 40 specifies the capture image f4 as a front surface image of the tablet and specifies the capture image f8 as a back surface of the tablet.

Thus, the control part 40 can compare the capture images f4 and f8, which are obtained by capturing the front and back surfaces of the tablet from a front side, with the correct image, or can read the identification information of the tablet from the capture images f4 and f8 with high accuracy. Further, it is conceivable that, for example, based on a tablet having a longest required time between the times t1 to t9 among various tablets registered in the medicinal master, an image capturing time per one tablet for the tablets, which are captured by the camera 462 and the camera 463 in the packing process, is determined to be equal to or longer than the required time. Thus, the capture image f4 and the capture image f8 are captured by at least one image as the front surface capture image and the back surface capture image of the tablet.

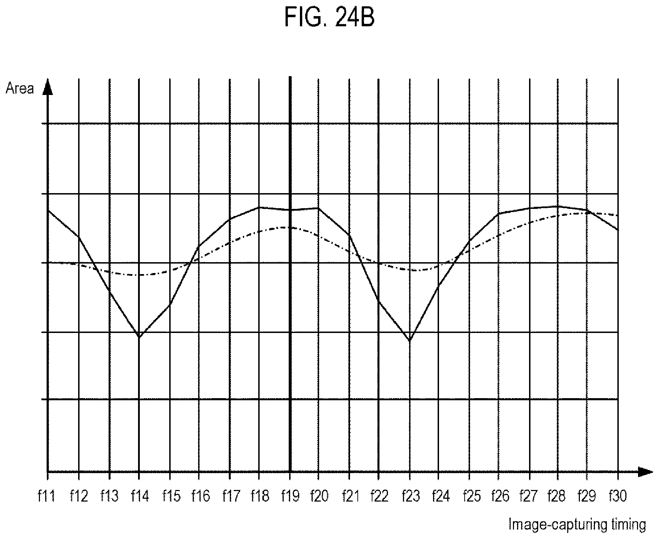

Further, it is also conceivable that the control part 40 specifies a capture image, which is captured at a timing when a smoothened value of the areas of tablet becomes a peak when the areas of tablet in the respective capture images are arranged in a capturing order of the respective capture images, as a front surface capture image or back surface capture image of the tablet. For example, the smoothened value is a simple moving average value of the areas of tablet in a plurality of the capture images. In this regard, FIG. 24B shows an example of the transition of the areas of tablet in case where images of the flat tablet (the tablet) rotated by the tablet rotation part 441 are captured continuously. FIG. 24B shows that image capturing has been performed at total twenty times of image capturing timings t11 to t30. In FIG. 24B, a solid line indicates a measured value of the area of the tablet in the capture image captured at each image capturing timing t11 to t30, and a one-dot chain line indicates the smoothened value of the area of the tablet in the capture image captured at each image capturing timing t11 to t30. In the example shown in FIG. 24B, the smoothened value is a peak value at the image capturing timing t19. Thus, the capture image captured at the image capturing timing t19 is specified as the front surface capture image or the back surface capture image of the tablet. More specifically, the odd-numbered peak value of the smoothened values is specified as the front surface capture image of the tablet and the even-numbered peak value is specified as the back surface capture image of the tablet.

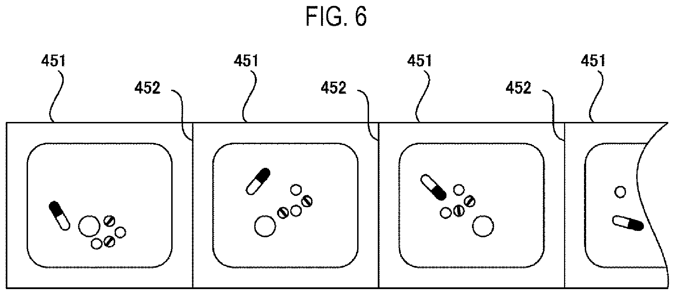

The packing unit 45 can pack the tablets of a unit of one dosage, which are supplied from either or both of the tablet cassette 41 and the manual distribution unit 42 via the rotary unit 44 into a medicine pack 451 (a packing material). In this regard, FIG. 6 shows an example of the medicine packs 451 dispensed from the tablet packing device 4. As shown in FIG. 6, a plurality of tablets are packed into each of the medicine packs 451 per unit of one dosage. Tear-off lines (perforations) for easily separating the respective medicine packs 451 are formed between the respective medicine packs 451.

Further, the medicine pack 451 or a packing paper (rolled sheet) for forming the medicine pack 451, which is used for packing in the packing unit 45, is an expendable article supplemented or replaced when necessary. The tablet packing device 4 has a function of detecting the residual quantity of the medicine packs 451 or the packing paper. The residual quantity is appropriately notified to the server 2 by the control part 40. Alternatively, in the tablet packing device 4, a blister pack or the like capable of accommodating tablets of a unit of one dosage may be used instead of the medicine packs 451. The blister pack is a packing material formed with a plurality of packing portions which are closed by bonding a packing member, which has a plurality of packing regions open at one side, to a flat plate-shaped member. Further, in the tablet packing device 4, the tablets received in the tablet cassette 41 are also an example of an expendable article. The tablet packing device 4 has a function of detecting the residual quantity of the tablets received in the tablet cassette 41, and the residual quantity of the tablets is appropriately notified to the server 2 by the control part 40.

Further, the packing unit 45 is provided with a printing unit 453 for printing information on each of the medicine packs 451. On a surface of each of the medicine packs 451, information such as a patient name, a dosing time, prescribed medicines, prescribed quantity, etc. can be printed by the printing unit 453. Further, an ink ribbon used for printing in the printing unit 453 is an expendable article supplemented or replaced when necessary. The tablet packing device 4 has a function of detecting the residual quantity of the ink ribbon, and the residual quantity of the ink ribbon is appropriately notified to the server 2 by the control part 40.

[Medicine preparation device 5] Similar to the tablet packing device 4, the medicine preparation device 5 is an apparatus used for preparing a medicine based on prescription data. In addition to the tablet packing device 4, the medicine preparation device 5 includes, for example, a powder medicine packing device, a liquid medicine dispensing device, a sheet dispensing device, a picking assisting device, etc. The powder medicine packing device includes a plurality of powder medicine cassettes for receiving a plurality of types of powder medicines. The powder medicine packing device can automatically pack, by a predetermined quantity, the powder medicines received in the powder medicine cassettes according to the prescription data. Further, the liquid medicine dispensing device includes a plurality of medicine bottles for containing a plurality of types of liquid medicines. The liquid medicine dispensing device dispenses a necessary quantity of liquid medicine from the medicine bottles according to the prescription data. The sheet dispensing device dispenses a sheet from a plurality of sheet cassettes in which a PTP sheet with pre-packed tablets or a heat seal is received. The picking assisting device is used when a pharmacist manually prepares medicines. The picking assisting device reads a medicine name from an identification information (barcode or the like) affixed a medicine rack, a medicine bottle or the like, and collates the read medicine name with a medicine name included in the prescription data.

In a medical facility such as a hospital or a pharmacy, a pharmacist conducts an inspection task to check whether the tablets packed by the tablet packing device 4 are proper ones corresponding to the prescription data. In this regard, in the above-described inspection assistance system 1, an inspection assistance process (*see* FIG. 7) which is described below is executed, assisting the inspection task of the pharmacist.

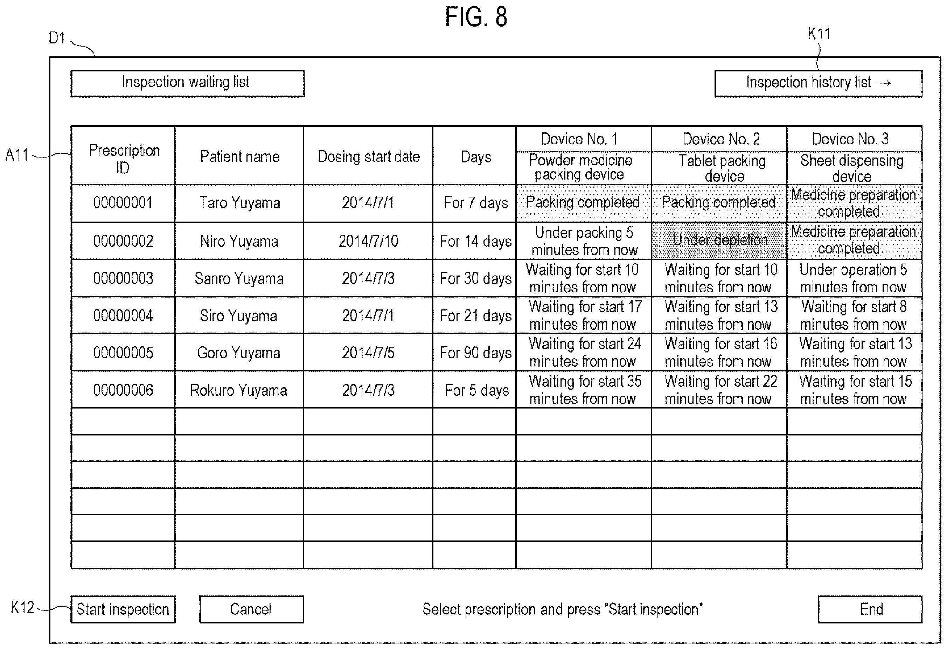

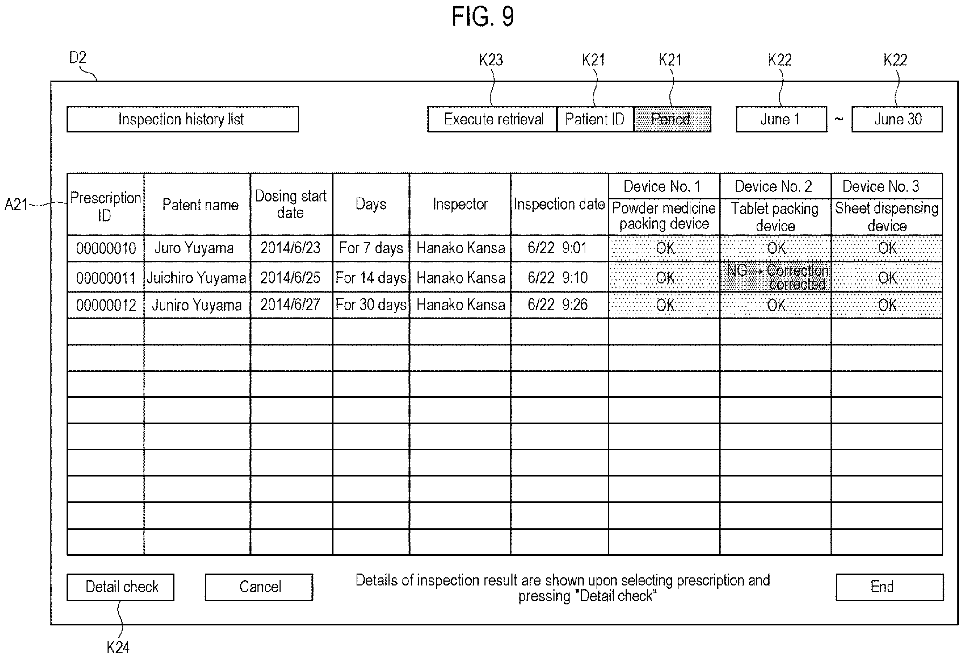

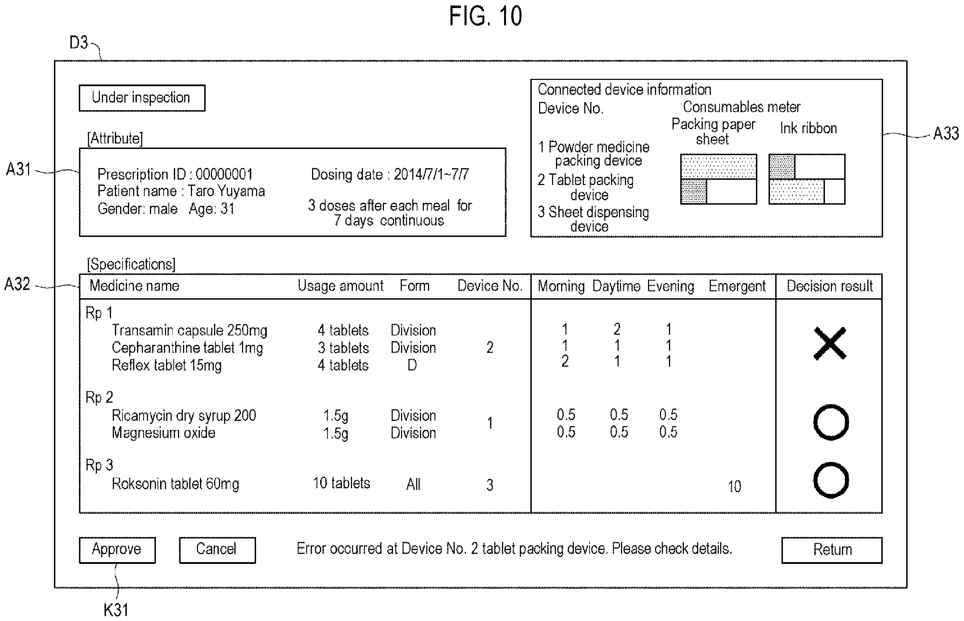

[Inspection Assistance Process] Hereinafter, descriptions are made with reference to FIG. 7 as to an example of a procedure of an inspection assistance process executed by the control part 21 of the server 2 in the inspection assistance system 1. The inspection assistance process is executed when a login manipulation is made on the client terminal 3 by a pharmacist having a pre-set final inspection authority or when an inspection start manipulation for performing a final inspection process is made after the login manipulation. Further, the "display", the "manipulation", etc. described below are performed using the display part 33 and the manipulation part 34 of the client terminal 3 on which the aforementioned login manipulation has been made.
<Step S11> First, in step S11, the control part 21 causes the client terminal 3 to display an inspection waiting list screen D1 for displaying a list of prescription data subject to inspection. In this regard, FIG. 8 shows an example of the inspection waiting list screen D1.
   As shown in FIG. 8, a list display area A11, in which a list of prescription data subject to inspection is displayed, is displayed in the inspection waiting list screen D1. Specifically, in the list display area A11, information on a prescription ID (prescription identification information), a patient name (patient identification information), a dosing start date and the number of days, which are included in the prescription data, is displayed together with a status information of a plurality of medicine preparation devices such as the tablet packing device 4 and the medicine preparation device 5 which are connected to the inspection assistance system 1.
   Herein, as shown in FIG. 8, it is assumed that, in the server 2, the powder medicine packing device of the medicine preparation device 5 is preregistered as Device No. 1, the sheet dispensing device is preregistered as Device No. 3, and the tablet packing device 4 is preregistered as Device No. 2. The status information is classified beforehand into "packing completed", "medicine preparation completed", "under packing", "under operation", "waiting for start", "under depletion", etc. The "packing completed" and the "medicine preparation completed" indicate that the medicine preparation has been completed, and a background or characters thereof are displayed in a predetermined first specific color such as a blue color. The "under packing" and the "under operation" indicate that the medicine preparation is being executed, and are displayed together with a waiting time until completion. The "waiting for start" is a standby state until start of medicine preparation and is displayed together with a waiting time until start. The "under depletion" indicates that the medicine preparation cannot start because expendable articles such as medicines used for medicine preparation based on the prescription data, the medicine packs 451, the packing paper and the ink ribbon are under a depletion state, and a background or characters thereof is displayed in a predetermined second specific color such as a red color.
   Further, manipulation keys K11 to K12 and the like for receiving user's manipulations are displayed in the inspection waiting list screen D1. The manipulation key K11 is a manipulation key for displaying an inspection history screen D2 which displays an inspection history in the server 2. The manipulation key K12 is a manipulation key for displaying an inspection screen D3 (an example of the first display screen) for executing an inspection process in the server 2. That is to say, in response to the manipulation on the inspection waiting list screen D1, the control part 21 can start the inspection of the medicine preparation performed by a plurality of medicine preparation devices such as the tablet packing device 4 and the medicine preparation device 5.
<Step S12> In step S12, the control part 21 determines whether a display manipulation has been made on the inspection history screen D2. Specifically, when the manipulation key K11 in the inspection waiting list screen D1 is manipulated, the control part 21 determines that the display manipulation on the inspection history screen D2 has been made. If it is determined that the display manipulation on the inspection history screen D2 has been made (S12: Yes), the control part 21 causes the process to proceed to step S 13. If the display manipulation on the inspection history screen D2 has not been made (S12: No), the control part 21 causes the process to proceed to step S 14.
<Step S13> In step S13, the control part 21 causes the client terminal 3 to display the inspection history screen D2. In this manner, in response to the user's manipulation on the inspection waiting list screen D1, the control part 21 can display the inspection history of the medicine preparation performed by a plurality of medicine preparation devices such as the tablet packing device 4 and the medicine preparation device 5.
   In this regard, FIG. 9 shows an example of the inspection history screen D2. As shown in FIG. 9, a list display area A21, in which a list of prescription data that has already been inspected is displayed, is displayed in the inspection history screen D2. In the list display area A21, information such as the prescription ID (prescription identification information), the patient name (patient identification information), the dosing start date, an inspector, inspection date and time, etc. included in the prescription data is displayed. Further, "OK" indicating that a decision result is proper or "NG" indicating that the decision result is an error is displayed in the list display area A21 as the inspection result for the medicines prepared by each of the tablet packing device 4 and the medicine preparation device 5 connected to the inspection assistance system 1. Specifically, the inspection result corresponding to the tablet packing device 4 is the result of the automatic inspection process. Further, a background or characters of the "OK" is displayed in a predetermined third specific color such as a blue color, and a background or characters of the "NG" is displayed in a predetermined fourth specific color such as a red color. Further, when the "NG" is displayed, what has been performed with respect to the decision result of an error (e.g., "correction completed" or the like) is displayed.
   Further, a manipulation key K21 for selecting one of "patient ID" and "period" as a retrieval item, an input field K22 for inputting the retrieval contents for an item selected by the manipulation key K21, a manipulation key K23 for executing a retrieval, etc. are displayed in the inspection history screen D2. When the retrieval contents are inputted to the input field K22 and the manipulation key of "retrieval execution" is manipulated, the control part 21 retrieves prescription data corresponding to the retrieval contents and displays the prescription data in the list display area A21. Further, a manipulation key K24 for displaying an inspection screen, which is similar to an inspecting screen D3 to be described below which displays the details of the inspection history of the prescription data displayed in the list display area A21, is displayed in the inspection history screen D2.
<Step S14> In step S14, the control part 21 determines whether an inspection start manipulation has been made. Specifically, when the manipulation key K12 is manipulated in a state where the prescription data is selected in the inspection waiting list screen D1, the control part 21 determines that the inspection start manipulation has been made. Then, if it is determined that the inspection start manipulation has been made (S14: Yes), the control part 21 causes the process to proceed to step S15. If the inspection start manipulation has not been made (S14: No), the control part 21 causes the process to proceed to step S16.
<Step S15> In step S15, the control part 21 causes the client terminal 3 to display an inspection screen D3 for performing an inspection on the prescription data selected in the inspection waiting list screen D1. In this regard, FIG. 10 shows an example of the inspection screen D3. As shown in FIG. 10, a basic information area A31, an inspection result area A32 and a device information area A33 are displayed in the inspection screen D3. Information such as the prescription ID (prescription identification information), the patient name (patient identification information), a gender, an age, the dosing date, a usage instruction, etc. included in the prescription data are displayed in the basic information area A31.
   At the inspection result area A32, the contents of the prescription data selected in the inspection waiting list screen D1 and the inspection result on the prescription data are displayed with respect to each of records (Rp1 to Rp3). Specifically, the medicine name, a dosage, a form, the number of the medicine preparation device, dose and decision result, etc. are displayed at the inspection result area A32. Further, similar to the automatic inspection process executed in the tablet packing device 4, the decision result is a decision result on the suitability of the automatic inspection process executed in each of the tablet packing device 4 and the medicine preparation device 5, and is appropriately inputted to the server 2 from each of the tablet packing device 4 and the medicine preparation device 5. Further, the automatic inspection process may be executed by the control part 21 of the server 2 which has acquired various types of information such as images from the tablet packing device 4 and the medicine preparation device 5. Specifically, in the server 2, the control part 21 causes the storage part 22 to store the inspection result of the suitability of the medicine preparation process executed by each of the tablet packing device 4 and the medicine preparation device 5 in association with a prescription identification information such as the prescription ID of the prescription. Further, the control part 21 can collectively display the inspection results of each of the tablet packing device 4 and the medicine preparation device 5 associated with the prescription ID of the prescription data in a single screen such as the inspection result area A32 of the inspection screen D3. Thus, a user can collectively check the inspection results of a plurality of medicine preparation devices 5 which include the tablet packing device 4 performing the medicine preparation based on the prescription data. For example, in the inspection screen D3 shown in FIG. 10, it is conceivable that Rp1 is a medicine prepared by the tablet packing device 4, Rp2 is a medicine prepared by the powder medicine packing device of the medicine preparation devices 5, and Rp3 is a medicine prepared by the sheet dispensing device of the medicine preparation devices 5.
   A residual quantity information on the expendable articles in the tablet packing device 4 and the medicine preparation device 5 connected to the server 2 is displayed at the device information area A33. The residual quantity information displayed at the device information area A33 is appropriately inputted to the server 2 from each of the tablet packing device 4 and the medicine preparation device 5, or is read out from each of the tablet packing device 4 and the medicine preparation device 5 by the server 2. Thus, a user can easily check the residual quantity of the expendable articles in the tablet packing device 4 and the medicine preparation device 5. For example, the example shown in FIG. 10 shows, as the information on the expendable articles of the tablet packing device 4 of "Device No. 2", that the residual quantity of the "packing paper" is small and the residual quantity of the "ink ribbon" is sufficient. Similarly, as the information on the expendable articles of the powder medicine packing device of "Device No. 1", the example shows that the residual quantity of the "packing paper" is sufficient and the residual quantity of the "ink ribbon" is small. In this manner, in the inspection screen D3, the control part 21 can display, by a list, the information on the expendable articles in a plurality of medicine preparation devices such as the tablet packing device 4 and the medicine preparation device 5. Therefore, a user can easily perform management of the entire expendable articles of the inspection assistance system 1. Thus, it is possible to procure the expendable articles without omission and it is possible to continuously operate a plurality of medicine preparation devices such as the tablet packing device 4 and the medicine preparation device 5 by a small number of people.
<Step S16> In step S16, the control part 21 determines whether a detail check manipulation for checking the details of the decision result displayed in the inspection screen D3 has been made. Specifically, when a display location of the decision result corresponding to any one of the records included in the prescription data displayed in the inspection screen D3 is selected on the inspection screen D3, it is determined that the detail check manipulation has been made. If it is determined that the detail check manipulation has been made (S16: Yes), the control part 21 causes the process to proceed to step S 17. If the detail check manipulation has not been made (S16: No), the control part 21 causes the process to proceed to step S18.
<Step S17> In step S17, the control part 21 displays an inspection detail screen D31 (an example of the second display screen) in which the details of the decision result displayed in the inspection screen D3 is displayed. Further, the displaying of the inspection screen D3 and the inspection detail screen D31 is executed by the inspection display processing part 211 of the control part 21.
   In this regard, FIG. 11 shows an example of the inspection detail screen D31. As shown in FIG. 11, the basic information area A31, a prescription information area A35, a medicine preparation detail area A36, an NG list area A37, etc. are displayed in the inspection detail screen D31. At the prescription information area A35, the medicine name (medicine identification information), the number of tablets for each dosing time, a medicine image, etc. are displayed as the prescription information of the record corresponding to the decision result.
   The contents of the tablets, which are packed into the medicine pack 451 corresponding to the dosing time of each dosing day (the number of tablets for each dosing time), are displayed at the medicine preparation detail area A36. More specifically, at the medicine preparation detail area A36 of the inspection detail screen D31, the control part 21 displays the capture images of the tablets in an arrangement format in which dosing dates are displayed in one of a column and a row and dosing times are displayed in the other of a column and a row.
   In particular, at the display locations corresponding to the respective combinations of the dosing dates and the dosing times, the control part 21 arranges and displays the capture images of the tablets of a unit of packing, which correspond to the respective combinations, in a display order predetermined in a direction parallel to the arrangement order of the dosing times. For example, the display order of the capture images of the tablet is the same as the order of the tablets displayed in the prescription information section A35. At the medicine preparation detail area A36 displayed as described above, when the decision result of the automatic inspection process is proper, the same type of the capture images of the tablet are arranged in the arrangement direction of the dosing dates (a left-right direction in FIG. 11). Accordingly, a pharmacist can easily check the suitability of each of the capture images of the tablet.
   Further, at the medicine preparation detail area A36, when the result of the automatic inspection process is an error, a background of an area displaying the capture image of the tablet which has caused the error is displayed in a predetermined fifth specific color such as a red color. That is to say, the control part 21 distinguishably displays the capture image of the error-causing tablet at the medicine preparation detail area A36. Thus, the capture images of the tablet are displayed per unit of packing and the result of the automatic inspection process is displayed, thereby promoting the efficiency of the pharmacist's inspection task. In particular, by referring to the display of the fifth specific color, the pharmacist can easily identify and check the capture image of the error-causing tablet.
   A list of errors occurring at the medicine preparation detail area A36 is displayed at the NG list area A37. Specifically, when the result of the automatic inspection process is an error, the control part 21 displays, by a list, a cause of the error together with the dosing date and the dosing time of the error-causing tablet. Such a process is executed by the list display processing part 212 of the control part 21. Thus, by referring to the displayed list, the pharmacist can easily understand the cause of the error and the dosing date and time of the tablet. Further, at one of the tablet packing device 4, the medicine preparation device 5 or the server 2, the cause of the error displayed at the NG list area A37 is specified based on the result of the automatic inspection process. For example, in case where a tablet different from the tablet to be packed into the medicine pack 451 according to the prescription data is received in the medicine pack 451, the cause of the error is specified as "distribution mistake". Further, in case where the tablets to be packed into the medicine pack 451 is deficient and instead tablets corresponding the number of deficient tablets are received in another medicine pack 451 continuously located ahead of and behind the medicine pack 451, the cause of the error is specified as "shift".
   Further, in the inspection detail screen D31, the capture images of the tablets received in all the medicine packs 451 are displayed at the medicine preparation detail area A36. However, for example, it is conceivable that, like the NG list area A37, the dosing date, the dosing time, the capture images of the tablet and the like corresponding only to the medicine pack 451 whose result of the automatic inspection process is an error are displayed, and the information of the medicine pack 451 whose result of the automatic inspection process is proper is not displayed. Thus, a user can easily grasp only the check-required locations by looking at the inspection detail screen D31. This makes it possible to improve the efficiency of the inspection task.
   Further, in the inspection detail screen D31, manipulation sections such as a manipulation key K33, a manipulation key K34, an input field K35, a manipulation key K36 and a check box K37 are displayed together with the medicine preparation detail area A36. The manipulation sections are an example of a manipulation section for individually re-executing a portion or all of the medicine preparation process based on the prescription data executed by the tablet packing device 4. The displaying of the manipulation sections is executed by the manipulation display processing part 213 of the control part 21. Specifically, in the tablet packing device 4, a packing process is performed as the medicine preparation process and the manipulation sections are a manipulation section for re-executing a portion of the entirety of the packing process.
   The manipulation key K33 is an example of a first manipulation section for re-executing all the medicine preparation processes (packing processes) corresponding to the above-described record. The manipulation key K34 is an example of a second manipulation section for re-executing a portion of the medicine preparation process (packing process) which is decided to be an error in the automatic inspection process among the medicine preparation processes (packing processes) corresponding to the above-described record. The input field K35 and the manipulation key K36 are an example of a third manipulation section for re-executing a portion of the medicine preparation process (packing process) which is selected by a user's manipulation on the input field K35 among the medicine preparation processes (packing processes) corresponding to the above-described record. In the input field K35, it is possible to designate a range of the medicine packs 451 (from a certain pack to a certain pack) subject to the re-execution of the medicine preparation process, or to designate only a specific one of the medicine packs 451. Thus, as described below, a pharmacist can cause the tablet packing device 4 to arbitrarily execute a portion or all of the packing process by selectively using any two or three of the manipulation key K33, the manipulation key K34, the input field K35 and the manipulation key K36.
   The check box K37 is a manipulation key for setting a process which does not re-execute the medicine preparation process for the medicine causing the error when the cause of the error is "shift". When the check box K37 is checked, even if the manipulation key K33, the manipulation key K34 or the manipulation key K36 is manipulated, the control part 21 does not perform re-execution for the medicine which has caused the error of "shift". Thus, for example, when the error of "shift" occurs in the tablet packing device 4, it is possible to cope with such an application that a pharmacist manually moves the tablets to the ahead-located medicine pack 451 or the behind-located medicine pack 451.
<Step S18> In step S18, the control part 21 determines whether an approval manipulation on the result of the automatic inspection process on the prescription data has been made. Specifically, the control part 21 determines whether an approval key K31 displayed in the inspection screen D3 has been manipulated. At this time, if it is determined that the approval manipulation has been made (S18: Yes), the control part 21 causes the process to proceed to step 19. If the approval manipulation has not been made (S18: No), the control part 21 causes the process to proceed to step 20.
<Step S19> In step S 19, the control part 21 executes an approval process of approving the result of the automatic inspection process on the prescription data. For example, in the approval process, the control part 21 causes the storage part 22 to store the approval of the result of the automatic inspection process on the prescription data, and an identification information (name or ID) of the user who is a pharmacist making the approval manipulation, together with the result of the automatic inspection process, in association with the prescription data.
<Step S20> In step S20, the control part 21 determines whether the all reissuance manipulation has been made in the inspection detail screen D31. Specifically, when the manipulation key K33 displayed in the inspection detail screen D31 is manipulated, it is determined that the all reissuance manipulation has been made. At this time, if it is determined that the all reissuance manipulation has been made (S20: Yes), the control part 21 causes the process to proceed to step S21. If the all reissuance manipulation has not been made (S20: No), the control part 21 causes the process to proceed to step S22.
<Step S21> In step S21, the control part 21 transmits a control instruction for re-executing all the medicine preparation processes corresponding to the record corresponding to the decision result, to the tablet packing device 4 or the medicine preparation device 5 that has executed the medicine preparation processes. For example, as the control instruction, a medicine preparation prescription data corresponding to the record is re-inputted to the tablet packing device 4 or the medicine preparation device 5. Thus, in the tablet packing device 4 or the medicine preparation device 5, all the medicine preparation processes corresponding to the record are re-executed. For example, in the tablet packing device 4, the entire packing process of the tablets corresponding to the record is re-executed.
<Step S22> In step S22, the control part 21 determines whether a reissuance manipulation for only the NG pack has been made on the inspection detail screen D31. Specifically, when the manipulation key K34 displayed in the inspection detail screen D31 is manipulated, it is determined that the reissuance manipulation for only the NG pack has been made. At this time, if it is determined that the reissuance manipulation for only the NG pack has been made (S22: Yes), the control part 21 causes the process to proceed to step S23. If the reissuance manipulation of only the NG pack has not been made (S22: No), the control part 21 causes the process to proceed to step S24.
<Step S23> In step S23, the control part 21 transmits a control instruction for re-executing only the medicine preparation process (NG pack), at which an error has occurred, among the medicine preparation processes corresponding to the record corresponding to the decision result, to the tablet packing device 4 or the medicine preparation device 5 that has executed the medicine preparation process. For example, as the control instruction, a portion of the prescription data corresponding to the medicine preparation process, at which the error has occurred, among the medicine preparation prescription data corresponding to the record, is re-inputted to the tablet packing device 4 or the medicine preparation device 5. Thus, in the tablet packing device 4 or the medicine preparation device 5, only a portion of the medicine preparation process at which the error has occurred is re-executed. For example, in the tablet packing device 4, a portion of the packing process for the tablets corresponding to the record is re-executed.
<Step S24> In step S24, the control part 21 determines whether a reissuance manipulation of only the designated pack has been made in the inspection detail screen D31. Specifically, when the manipulation key K36 displayed in the inspection detail screen D31 is manipulated, it is determined that the reissuance manipulation of only the designated pack has been made. At this time, if it is determined that the reissuance manipulation of only the designated pack has been made (S24: Yes), the control part 21 causes the process to proceed to step S25. If the reissuance manipulation of only the designated pack has not been made (S24: No), the control part 21 causes the process to proceed to step S26.
<Step S25> In step S25, the control part 21 transmits a control instruction for re-executing only the medicine preparation process (designated pack) designated in the input field K35 among the medicine preparation processes corresponding to the record corresponding to the decision result, to the tablet packing device 4 or the medicine preparation device 5 that has executed the medicine preparation process. For example, as the control instruction, a portion of the prescription data corresponding to the medicine preparation process designated in the input field K35 among the medicine preparation prescription data corresponding to the record is re-inputted to the tablet packing device 4 or the medicine preparation device 5. Thus, in the tablet packing device 4 or the medicine preparation device 5, only a portion of the medicine preparation process designated in the input field K35 is re-executed. For example, in the tablet packing device 4, a portion of the packing process for the tablets corresponding to the record is re-executed.
<Step S26> In step S26, the control part 21 determines whether a preset inspection termination manipulation for terminating the inspection assistance process has been made. For example, when the manipulation key corresponding to "end" in the inspection waiting list screen D1 is manipulated, the control part 21 determines that the inspection termination manipulation has been made. At this time, if it is determined that the inspection termination manipulation has been made (S26: Yes), the control part 21 terminates the inspection assistance process. If the inspection termination manipulation has not been made (S26: No), the control part 21 returns the process to the step S12.

As described above, in the inspection assistance system 1, the control part 21 executes the processes of steps S20 to S25, whereby a portion or all of the medicine preparation processes already executed in each of the tablet packing device 4 and the medicine preparation device 5 can be individually re-executed by each of the tablet packing device 4 and the medicine preparation device 5. Such a process is executed by the re-execution processing part 214 of the control part 21. Thus, it is possible to improve the task efficiency of a pharmacist in a hospital, a pharmacy or the like in which the inspection assistance system 1 is used.

For example, in the tablet packing device 4, the automatic inspection process based on the prescription data and the identification information of the tablet read from the capture image of the tablet captured by the image capturing part 46, or the automatic inspection process based on the correct image and the capture image of the tablet captured by the image capturing part 46 is executed. Also, the packing process can be re-executed per the medicine pack 451 in response to the user's arbitrary manipulation corresponding to the execution result of the automatic inspection process. Accordingly, it is possible to omit the labor of a pharmacist who conducts the final inspection for the medicine pack 451 dispensed from the tablet packing device 4, thereby improving the efficiency of the pharmacist's inspection task.

In the present embodiment, descriptions were mainly made as to the case where the control part 21 re-executes the medicine preparation process executed in the tablet packing device 4. However, the control part 21 can arbitrarily re-execute a portion or all of the medicine preparation processes performed in a plurality of medicine preparation devices such as the tablet packing device 4 and the medicine preparation device 5, in response to the user's manipulation on the inspection screen D3 or on the inspection detail screen D31 displayed in response to the user's manipulation on the inspection screen D3.

For example, in case the medicine preparation device 5 is the powder medicine packing device, the control part 21 can re-execute a portion or all of the powder medicine preparation processes performed by the powder medicine packing device. Further, the powder medicine packing device includes: a powder medicine cassette in which a powder medicine is received; a feeder for automatically dispensing a predetermined quantity of powder medicines from the powder medicine cassette; a distribution unit for distributing the powder medicine dispensed from the powder medicine cassette by the feeder per each dosing time; and a packing unit for packing the powder medicine of each dosing time distributed by the distribution unit into a packing paper. The distribution unit includes, for example, a rotary disc on which the powder medicine supplied from the feeder is placed, and a scooping member for scooping the powder medicine placed on the rotary disc by each predetermined quantity. In the powder medicine packing device, for example, a predetermined quantity of powder medicine is supplied from the feeder, in a state where the rotary disc is stopped, and is scooped out by the scooping member, whereby the packing process can be executed per unit of one pack.

[Other Embodiments] Hereinafter, descriptions are made as to other functions of the inspection assistance system 1 or other embodiments of the inspection assistance system 1.

[Re-pack Collation Function] As described in the above embodiment, in response to the user's manipulation on the inspection detail screen D31, the control part 21 may cause the tablet packing device 4 to re-execute a portion or all of the packing processes corresponding to the record of the prescription data. At this time, it is conceivable that the control part 21 has a re-pack collation function of collating the medicine pack 451 re-executed by the tablet packing device 4.

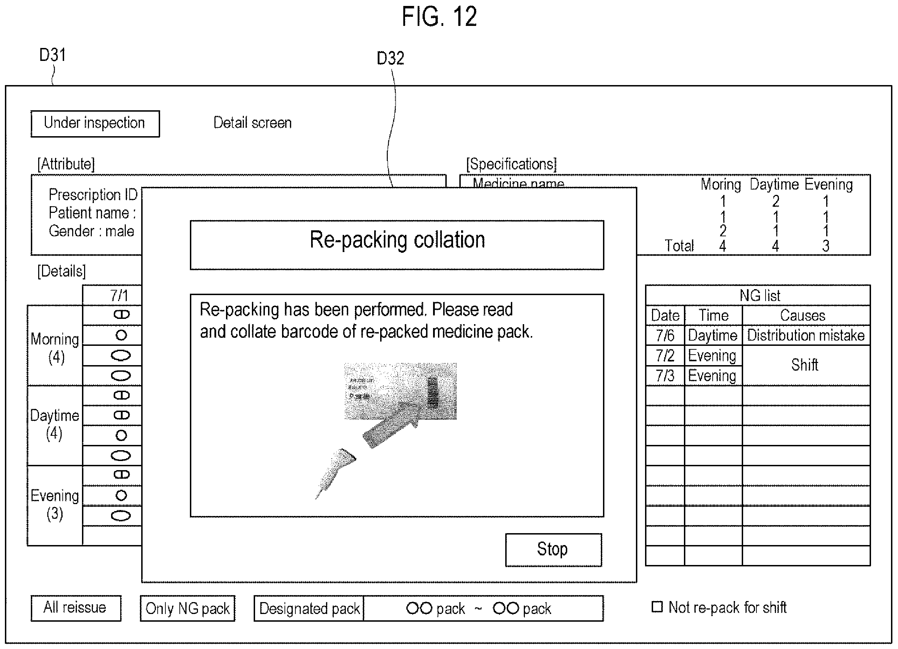

For example, as shown in FIG. 12, it is conceivable that, after requesting the tablet packing device 4 to re-execute the packing process, the control part 21 displays a collation screen D32, which urges a user to read a code information (one-dimensional code or two-dimensional code) written in the re-executed and prepared medicine pack 451, by superimposing the collation screen on the inspection detail screen D31. Then, when the code information, which is read from the medicine pack 451 by means of the code reading part 27 of the server 2, the code reading part 37 of the client terminal 3 or the like, is proper, the control part 21 changes the medicine preparation result of the record to "correction completed". As a result, when the inspection history screen D2 is displayed, "NG → correction completed" is displayed with respect to the record.

For example, the suitability of the code information is determined according to whether both of the code information of the medicine pack 451 before the re-execution and the code information of the medicine pack 451 after the re-execution have been read. Further, it is conceivable that the suitability of the code information is determined according to whether the code information of the medicine packs 451 located ahead of and behind the medicine pack 451 to be re-executed, among a plurality of the medicine packs 451 obtained as a packing result corresponding to the record, has been read. As a result, a mistake in replacement positions of the medicine pack 451 before and after re-execution is suppressed. It is also conceivable that the suitability of the code information is determined according to whether the code information printed on either or both of the foremost and last medicine packs 451 before re-execution and the code information printed on the medicine packs 451 after the re-execution have been read.

[Progress Monitoring Function] It is conceivable that the control part 21 has a function of appropriately checking the capture images of the tablet captured on the path of the tablets supplied from the tablet cassette 41 or the manual distribution unit 42 to the packing unit 45 by using the cameras 461 to 467 of the image capturing part 46 of the tablet packing device 4. Specifically, the capture images of the tablet captured by the image capturing part 46 are transmitted from the tablet packing device 4 to the server 2 and are stored in the storage part 22 of the server 2.

When a manipulation key (not shown), which is displayed in the manipulation screens such as the inspection screen D3 and the inspection detail screen D31 and corresponds to the progress monitoring function, is manipulated, the control part 21 can cause the client terminal 4 to display a progress monitoring screen D4 for displaying the capture images of the tablet captured by the cameras 461 to 467 of the image capturing part 46.

Thus, when an error occurs in the medicine preparation performed by the tablet packing device 4, a user can easily check the occurrence place and cause of the error by checking the capture images of the tablet which are captured by the image capturing part 46 at the respective time points with respect to the dispensing the tablets in the tablet packing device 4.

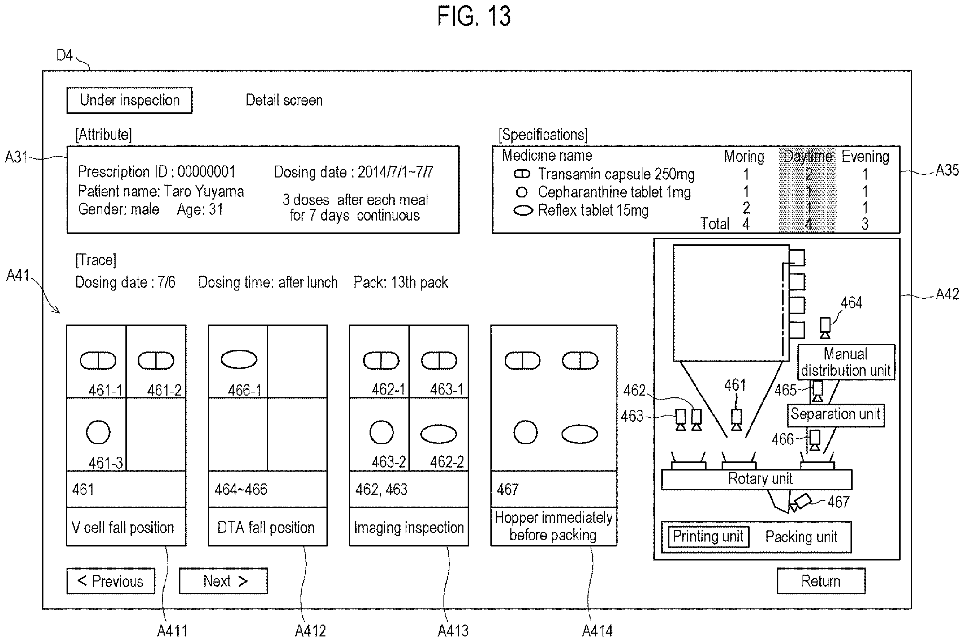

In this regard, FIG. 13 shows an example of the progress monitoring screen D4. In the progress monitoring screen D4 shown in FIG. 13, an image display area A41 and a guide display area A42 are displayed together with the basic information area A31 and the prescription information area A35. In the image display area A41, the capture images of the tablet captured by the cameras 461 to 467 of the image capturing part 46 are displayed in association with identification codes of the cameras 461 to 467. Specifically, the image display area A41 includes: a display area A411 in which the capture images of the tablet captured by the camera 461 are displayed; a display area A412 in which the capture images of the tablet captured by the cameras 464 to 466 are displayed; a display area A413 in which the capture images of the tablet captured by the cameras 462 and 463 are displayed; and a display area A414 in which the capture images of the tablet captured by the camera 467 are displayed. Further, a guide information for indicating the image capturing locations of the cameras 461 to 467 in the tablet packing device 4 is displayed in the guide display area A42. Thus, a user can easily grasp the positions of the images displayed in the image display area A41.

Further, when the capture images are stored in the storage part 22, the control part 21 causes the storage part 22 to store the respective capture images and the prescription identification information such as the prescription ID of the prescription data which was the target of the packing process when the respective capture images were captured, in association with each other. Thus, when the prescription ID of the prescription data is designated after terminating the packing process and the inspection assistance process, the control part 21 can display the respective capture images corresponding to the designated prescription ID.

[Manual Distribution Check Monitoring Function] Further, the control part 21 has a manual distribution check monitoring function of checking the state after a manual distribution task for tablets in the manual distribution unit 42. Specifically, when tablets are packed by using the manual distribution unit 42, the tablet packing device 4 causes the camera 464 to capture an image of the manual distribution unit 42 after the task of manually distributing tablets to the manual distribution unit 42.

For example, in the tablet packing device 4, when a manipulation key for inputting the completion of the task of manually distributing tablets to the manual distribution unit 42 is manipulated, the control part 40 captures the image of the manual distribution unit 42 using the camera 464. Further, in case where the manual distribution unit 42 is configured to be drawn out of the tablet packing device 4 when in use and to be retracted to the tablet packing device 4 after tablets have been supplied, it is also conceivable that the camera 464 is provided inside the tablet packing device 4 and the control part 40 causes the camera 464 to capture the image of the manual distribution unit 42 at the timing when the manual distribution unit 42 is retracted to the tablet packing device 4.

Then, the tablet packing device 4 transmits a manual distribution image captured by the camera 464 to the server 2 together with the information for identifying the prescription data. As a result, the manual distribution image is stored in the storage part 22 in association with the prescription data by the control part 21. Further, the medicine preparation management device 2 may be configured to read out the manual distribution image stored in the tablet packing device 4.

On the other hand, when a manipulation key (not shown), which is displayed in the manipulation screens such as the inspection screen D3 or the inspection detail screen D31 and corresponds to the manual distribution check monitoring function, is manipulated, the control part 21 reads out, from the storage part 22, the manual distribution image corresponding to the currently-displayed prescription data. Then, the control part 21 causes the client terminal 3 to display a manual distribution check screen D5 in which the manual distribution image is displayed. Thus, when an error occurs in the medicine preparation performed by the tablet packing device 4, a user can check the manual distribution image captured by the camera 464 with respect to the dispensing of the tablets in the tablet packing device 4 and can easily check whether a cause of the error is a mistake in distributing tablets to the manual distribution unit 42.

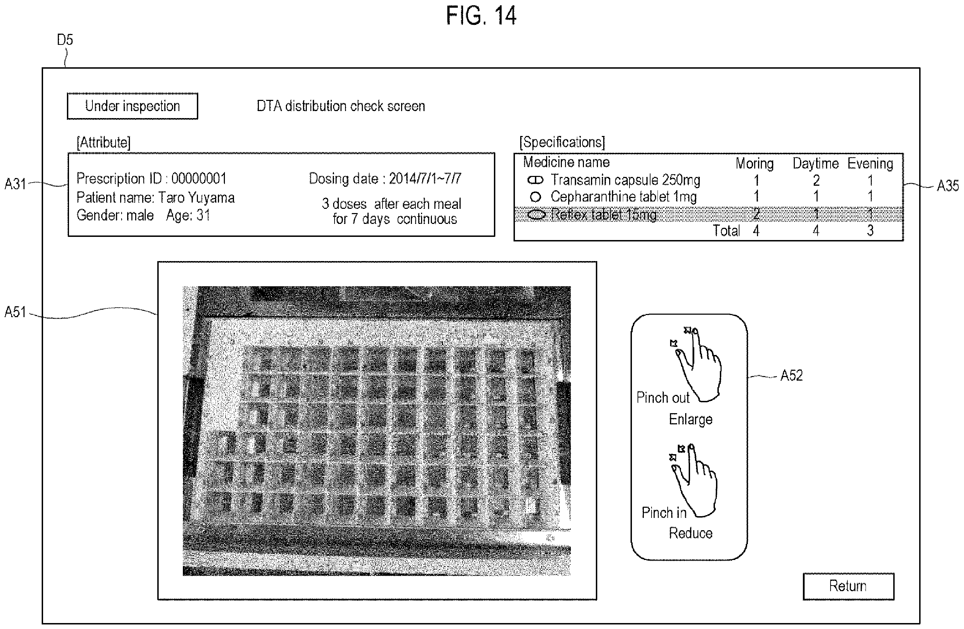

In this regard, FIG. 14 shows an example of the manual distribution check monitoring screen D5. A manual distribution unit image area A51 and a manipulation explanation area A52 are displayed together with the basic information area A31 and the prescription information area A35 in the manual distribution check monitoring screen D5 shown in FIG. 14. The manual distribution image obtained by capturing the manual distribution unit 42 from above by the camera 464 is displayed in the manual distribution unit image area A51. Thus, a user can check the state immediately after tablets are manually distributed to the manual distribution unit 42.

Further, an explanation on how to manipulate the manual distribution unit image area A51 is displayed in the manipulation explanation area A52. For example, in the manipulation explanation area A52, an indication that the image is enlarged by a pinch-out manipulation and is reduced by a pinch-in manipulation is displayed together with gestures and images of fingers.

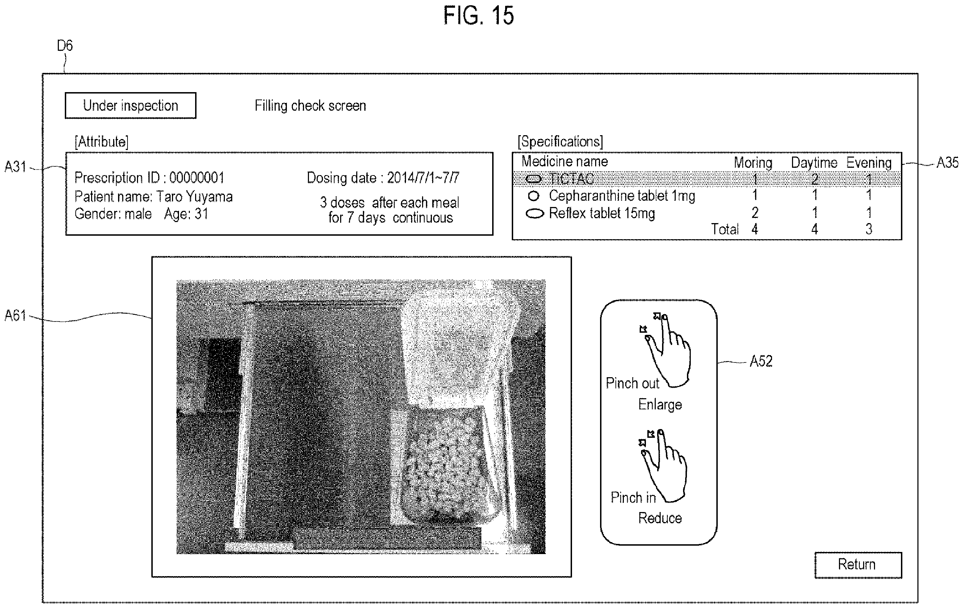

[Cassette Filling Monitoring Function] Further, the control part 21 has a cassette filling monitoring function of checking the state of a task of filling of the tablet cassette 41 with tablets. Specifically, it is conceivable that the tablet packing device 4 includes a worktable for performing the filling of the tablet cassette 41 with tablets and a camera for capturing an image of the tablet cassette 41 placed on the worktable. Further, the worktable is provided on a front surface of the tablet packing device 4. As shown in FIG. 15, the worktable can be drawn out of the tablet packing device 4 when the tablet cassette 41 is filled with tablets, and can be retracted in the tablet packing device 4 when not in use. Also, in the tablet packing device 4, when the tablet cassette 41 is filled with the tablets, the control part 40 captures an image of the tablet cassette 41 by using the camera.

For example, in the tablet packing device 4, when a manipulation key for inputting the completion of a task of filling of the tablet cassette 41 with tablets is manipulated, the control part 40 captures an image of the tablet cassette 41 by using the camera. Then, the tablet packing device 4 transmits a cassette image captured by the camera to the server 2 together with the medicine identification information such as a tablet name for identifying the tablets. As a result, in the server 2, the cassette image is stored in the storage part 22 in association with the tablet by the control part 21. Further, the medicine preparation management device 2 may be configured to read out the cassette image stored in the tablet packing device 4.

On the other hand, when the tablet subject to display is selected on the manipulation screen such as the inspection screen D3 or the inspection detail screen D31 and thereafter a manipulation key (not shown) corresponding to the cassette filling monitoring function displayed in the manipulation screen is manipulated, the control part 21 reads out the cassette image corresponding to the tablet from the storage part 22. Then, the control part 21 causes the client terminal 3 to display a cassette filling monitoring screen D6 in which the cassette image is displayed. Thus, when an error occurs in the packing process performed by the tablet packing device 4, a user can check the cassette image captured by the camera and can easily check whether a cause of the error is a mistake in filling of the tablet cassette 41 with tablets.

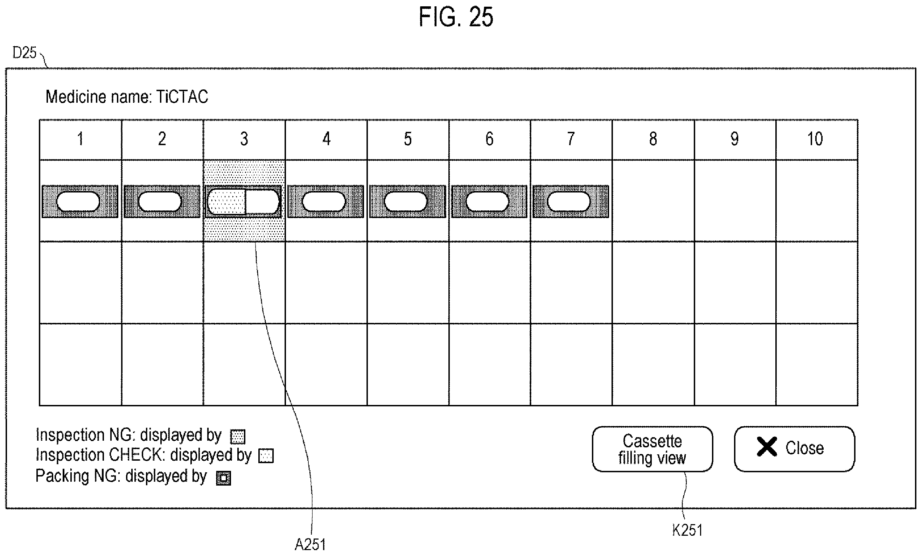

For example, if a specific type of tablet is selected on the manipulation screen such as the inspection screen D3 or the inspection detail screen D31, the control part 21 displays the inspection result of the selected type of tablet by a pop-up screen D25 shown in FIG. 25. Further, in the pop-up screen D25, the tablet whose inspection result is an error is displayed in a distinguishable manner. For example, a background area A251 of the tablet whose inspection result is an error is displayed in red. Then, when the manipulation key K251 corresponding to the cassette filling monitoring function is manipulated on the pop-up screen D25, the control part 21 displays the cassette filling monitoring screen D6 for displaying the cassette image corresponding to the tablet.

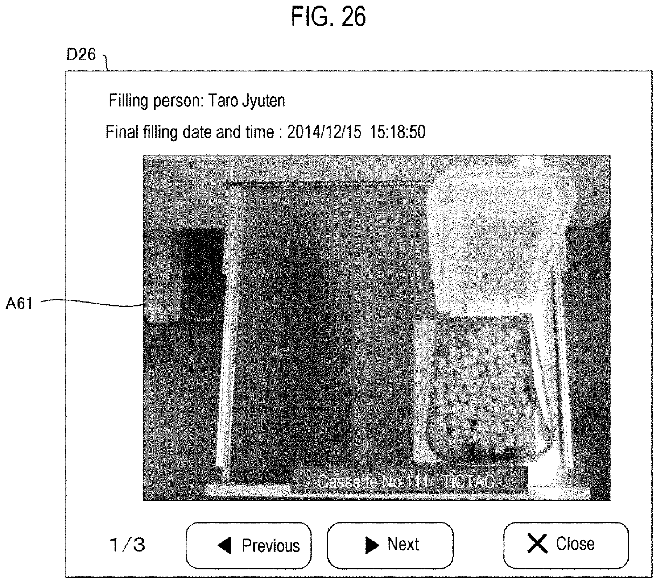

In this regard, FIG. 15 shows an example of the cassette filling monitoring screen D6. A cassette image area A61 is displayed together with the basic information area A31, the prescription information area A35 and the manipulation explanation area A52 in the cassette filling monitoring screen D6 shown in FIG. 15. The cassette image obtained by capturing the tablet cassette 41 from above with the camera is displayed in the cassette image area A61. Thus, a user can check the filling state of the tablet cassette 41 with tablets. Further, FIG. 26 shows a cassette filling monitoring screen D 26 which is another example of the cassette filling monitoring screen D6. In the cassette filling monitoring screen D26, similar to the cassette filling monitoring screen D6, the cassette image is displayed in the cassette image area A61, and information such as a person who conducted the filling task of the tablet cassette 41 and date and time of final filling is also displayed.

[Other Example of Inspection Screen] An inspection detail screen D301 related to a first display form and an inspection detail screen D302 related to a second display form are described as other examples of the inspection detail screen D31 displayed in the client terminal 3 by the control part 21.

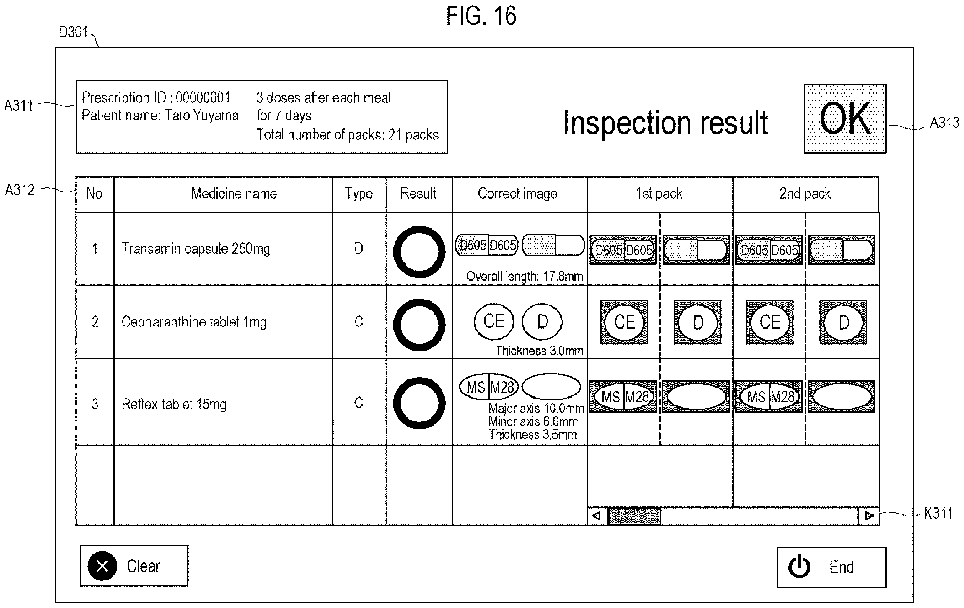

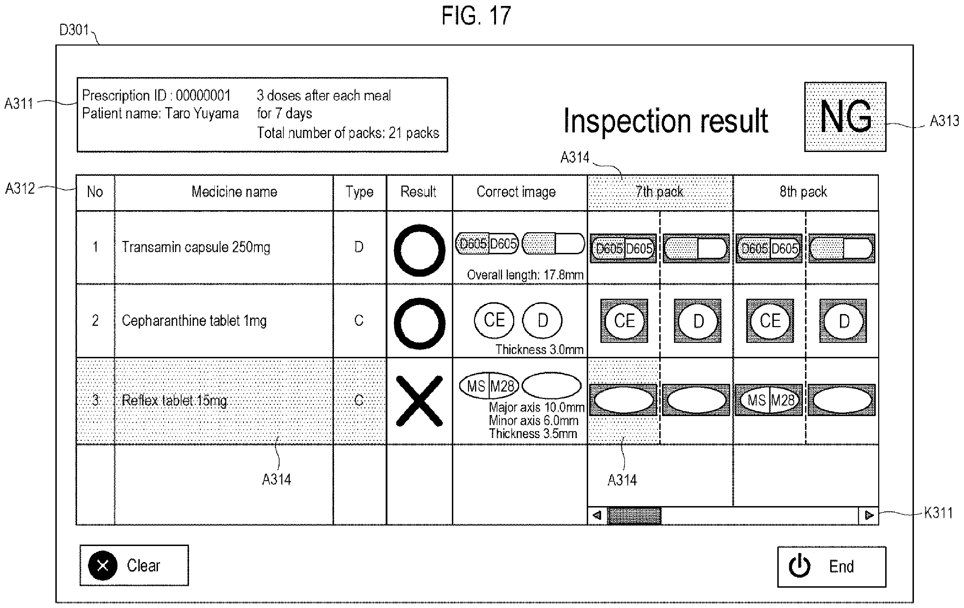

[First Display form of Inspection Screen] In this regard, FIGS. 16 and 17 show an inspection detail screen D301 which is another example of the inspection detail screen D31. FIG. 16 shows the inspection detail screen D301 wherein the inspection result is proper, and FIG. 17 shows the inspection detail screen D301 wherein the inspection result is an error.

As shown in FIGS. 16 and 17, a basic information area A311, a medicine preparation detail area A312 and an inspection result area A313 are displayed in the inspection detail screen D301. Similar to the basic information section A31, the basic information area A 31 is an area in which a patient information, a prescription data and the like are displayed. The medicine preparation detail area A312 is displayed in place of the medicine preparation detail area A36. The medicine preparation detail area A312 displays what tablets are packed into the medicine packs 451 per unit of the medicine pack 451.

More specifically, a medicine name, a type, a result and a correct image are displayed at the medicine preparation detail area A312 shown in FIGS. 16 and 17. Specifically, as to the aforementioned type, it is distinguishably displayed which one of the tablet cassette 41 and the manual distribution unit 42 the tablet corresponding to the medicine name is dispensed from. For example, in case the dispensing source of the tablet is the tablet cassette 41, "C" or "Cassette" is displayed as an identification symbol. In case the dispensing source of the tablet is the manual distribution unit (Detachable Tablet Adapter), "D" or "Manual" is displayed as an identification symbol. Further, as to the result, it is individually displayed whether the inspection result of the tablet corresponding to the medicine name is proper. The correct image is a registration image preregistered in the medicine master or the like as a correct image of the tablet. For example, in the medicine master, two images respectively corresponding to the front and back surfaces of the tablet are registered as the registration image in association with each tablet. Further, the registration image may be a single image wherein an area including the identification information of the tablet among an outer peripheral surface of the tablet is captured.

Further, at the medicine preparation detail area A312, the capture images of the tablet, which are captured by the image capturing part 46 before the tablets are packed into the respective medicine packs 451, are displayed in association with respective tablets. In this regard, the control part 21 displays the capture images of the tablet corresponding to the respective combinations of the tablet name and the packing order, in such an arrangement format that the tablet name (medicine name) is displayed in one of a row and a column and the packing order (the 1st pack, the 2nd pack, ···) is displayed in the other of a row and a column. Further, at the medicine preparation detail area A312 shown in FIGS. 16 and 17, the tablet names are arranged and displayed in an up-down direction and the packing order is displayed in a left-right direction. In this manner, since the same types of the capture images are arranged in an arrangement direction of the packing order, a pharmacist can easily check the suitability of the respective tablet capture images.

Further, the control part 21 displays, with respect to each tablet, a plurality of capture images, which include at least the original image whose tablet identification information is read in the tablet packing device 4 or the original image compared with the correct image, and which are captured with respect to each tablet in the tablet packing device 4 and correspond to different outer peripheral surfaces of the tablets. Thus, for example, when it is difficult for a pharmacist to determine the suitability of the tablet with only one tablet capture image, a pharmacist can determine the suitability of the tablet by referring to a plurality of the capture images of the tablet. Further, at the medicine preparation detail area A312, the correct image of the tablet and the capture images of the tablet are displayed side by side. Therefore, a pharmacist can easily determine the suitability of the tablet by comparing the registration image of the tablet and the capture images of the tablet.

For example, at the medicine preparation detail area A312 shown in FIG. 16, two capture images of each of three tablets, "Transamin capsule 250 mg", "Cepharanthine tablet 1 mg" and "Reflex tablet 15 mg", are displayed as the capture images of the tablets packed into the medicine pack 451 corresponding to a first pack. At this time, in case the tablet is a flat tablet, two images of the front and back surfaces of the tablet are displayed. In case the tablet is in the form of a capsule tablet or the like, two images of the surface including the identification information of the tablet and the surface not including the identification information of the tablet are displayed.

Further, the control part 21 acquires the identification information included in the capture image of the tablet and detects an inclination of the character included in the identification information. The control part 21 makes a rotation correction of the tablet capture image such that the top and bottom of the character included in the identification information coincide with the top and bottom of the inspection detail screen D301 and then displays the tablet capture image at the medicine preparation detail area A312. That is to say, the control part 21 displays the tablet capture image by aligning an orientation of the character of the identification information of the tablet. Thus, a user can easily check the identification information of the tablet together with the respective images of the tablets. Further, the rotation correction for the tablet capture image may be executed by the tablet packing device 4.

Further, when the number of the medicine packs 451 exceeds the number of medicine packs that can be simultaneously displayed at the medicine preparation detail area A312, a scroll key K311 is displayed at the medicine preparation detail area A312 and the control part 21 scroll-displays the information of the respective medicine packs 451 to be displayed at the medicine preparation detail area A312 in response to manipulation of the scroll key K311. Further, it is conceivable that, at the medicine preparation detail area A312, the dosing date and dosing time are displayed together with or in place of the pack number information such as the 1 st pack, the 2nd pack or the like which shows the pack number of the medicine packs 451.

In particular, as shown in FIG. 17, at the medicine preparation detail area A312, when the result of the automatic inspection process is an error in the medicines included in the record of the prescription data displayed in the inspection detail screen D301, a background of one or a plurality of areas A314 corresponding to such a medicine are displayed in a predetermined sixth specific color such as a red color. Thus, by checking the location of the area A314, a user can easily grasp the medicine whose result of the automatic inspection process is an error.

Specifically, the medicine preparation detail area A312 of FIG. 17 shows a case where the display areas of the medicine name, the tablet capture image and the number of the medicine pack 451 are set to the aforementioned area A314. Further, when the number of medicine packs 451 exceeds the number of medicine packs that can be simultaneously displayed at the medicine preparation detail area A312, and when there is a medicine whose result of the automatic inspection process is an error, the control part 21 automatically moves the scroll key K311 and displays the information corresponding to the medicine pack 451, at which the error has occurred, at the medicine preparation detail area A312.

Further, regarding the medicine preparation of all the medicines included in the record of the prescription data displayed in the inspection detail screen D301, the results of the automatic inspection process are displayed at the inspection result area A313. Specifically, in FIG. 16, "OK" indicating that the result of the automatic inspection process is proper is displayed together with a background of a predetermined seventh specific color such as a blue color. In FIG. 17, "NG" indicating that the result of the automatic inspection process is an error is displayed together with a background of a predetermined eighth specific color such as a red color different from the seventh specific color. Thus, a user can easily grasp the suitability of the inspection result on all the records by checking the inspection result area A313.

Particularly, at the medicine preparation detail area A312, the results of the inspection process are individually displayed with respect to the respective tablets included in the record of the prescription data. Specifically, in the example shown in FIG. 17, "O" indicating that the result of the inspection process is proper is displayed with respect to "Transamin capsule 250 mg" and "Cepharanthine tablet 1 mg", and "X" indicating that the result of the inspection process is an error is displayed with respect to "Reflex tablet 15 mg". Thus, a pharmacist can easily grasp, for example, the tablet which causes an error in the result of the inspection process.

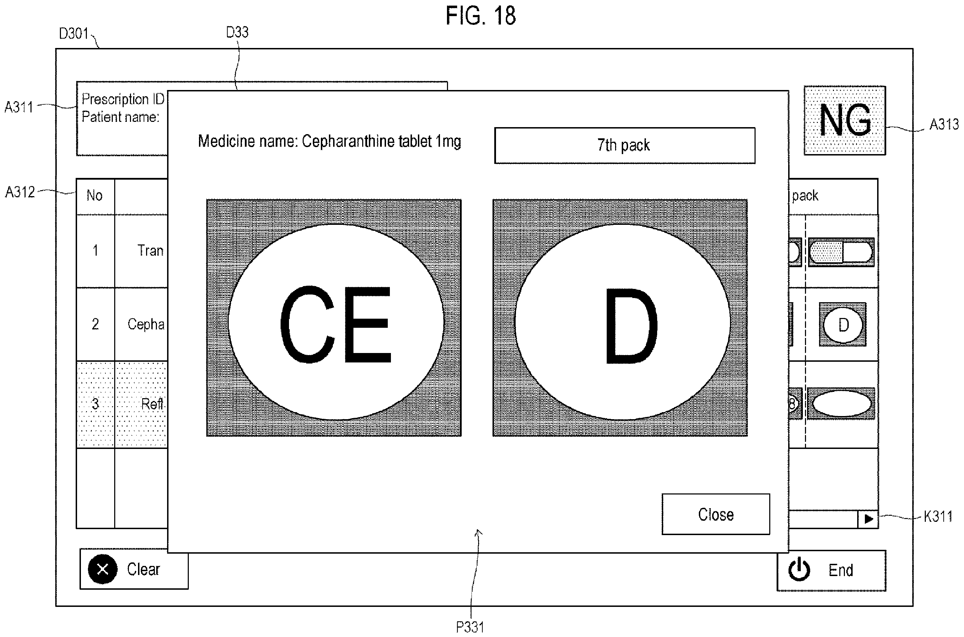

Further, when a selection manipulation for the tablet capture image displayed at the medicine preparation detail area A312 is made, as shown in FIG. 18, the control part 21 can cause the client terminal 3 to display an enlarged screen D33 in which the tablet capture image subject to the selection manipulation is enlarged and displayed as an enlarged image P331. Further, in the enlarged screen D33, the enlarged image P331 and an enlarged image, which enlarges the correct image corresponding to the tablet subject to the selection manipulation, may be displayed side by side up and down or left and right. Further, in the enlarged screen D33, the packing order (n-th pack) of the medicine pack 451 into which the tablet selected by the selection manipulation is packed is displayed. Thus, a user can easily grasp the medicine pack 451 into which the tablet under check are packed. Further, it is conceivable that, in response to a predetermined user's manipulation such as a flick manipulation on the enlarged screen D33 in an up-down direction or a left-right direction, the control part 21 changes the tablet displayed in the enlarged screen D33 according to the packing order of the medicine pack 451. For example, in case where the tablet packed into the medicine pack 451 of the seventh medicine pack is displayed in the enlarged screen D33, the tablet packed into the medicine pack 451 corresponding to the sixth or eighth medicine pack is displayed in response to the predetermined user's manipulation. Further, it is conceivable that the control part 21 sequentially displays only the error-causing tablets in response to the predetermined user's manipulation. Further, it is also conceivable that the control part 21 switches the medicine pack 451 to be displayed in response to the flick manipulation in one of the up-down direction and the left-right direction on the enlarged screen D33 and switches the type of the tablet to be displayed to another tablet packed in the same pack in response to the flick manipulation in the other of the up-down direction and the left-right direction.

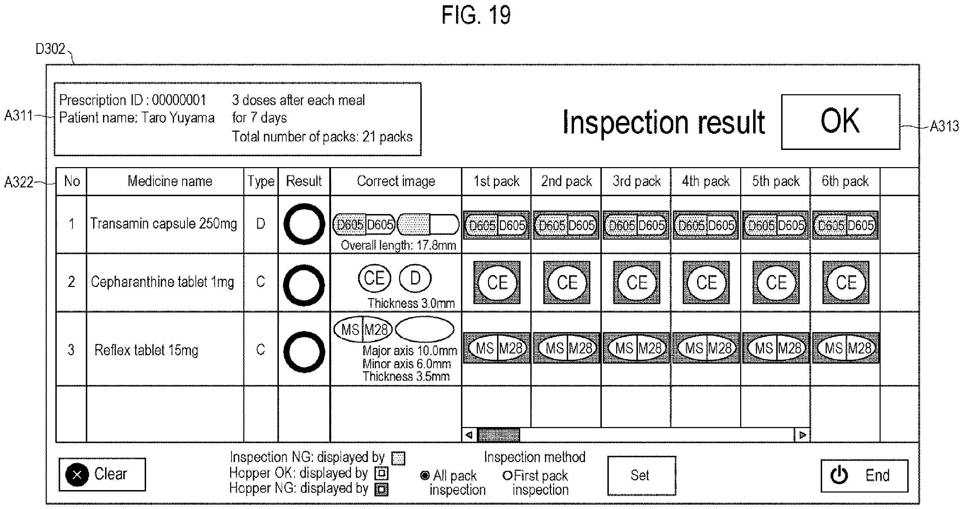

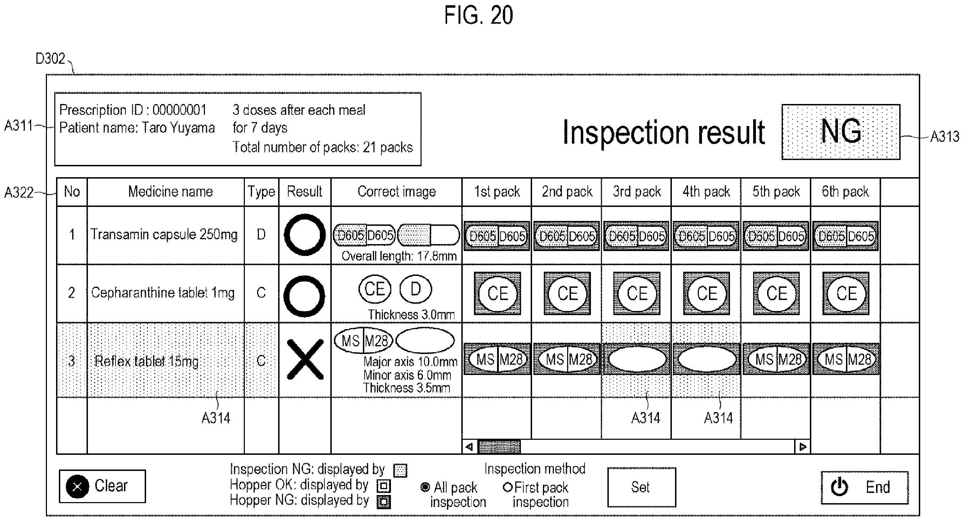

[Second Display Form of Inspection Screen] FIGS. 19 and 20 show an inspection detail screen D302 which is another example of the inspection detail screen D31. FIG. 19 shows the inspection detail screen D302 wherein the inspection result is proper, and FIG. 20 shows the inspection detail screen D302 wherein the inspection result is an error.

As shown in FIGS. 19 and 20, in the inspection detail screen D302, the basic information area A311 and the inspection result area A313 similar to those of the inspection detail screen D301 are displayed, and a medicine preparation detail area A322 is displayed in place of the medicine preparation detail area A312.

More specifically, at the medicine preparation detail area A322 shown in FIGS. 19 and 20, similar to the medicine preparation detail area A312, the medicine name, the type, the result and the correct image are displayed. On the other hand, at the dispensing detailed area A322, dissimilar to the medicine preparation detail area A312, only one image including the identification information of the tablet is displayed as the tablet capture image. Therefore, it is possible to increase the number of packs that can be simultaneously displayed at the medicine preparation detail area A322. This is because the identification information of the tablet can be generally checked when the outer peripheral surface of the medicine is viewed from one direction and thus the images of the outer peripheral surface other than that are not necessary in many cases.

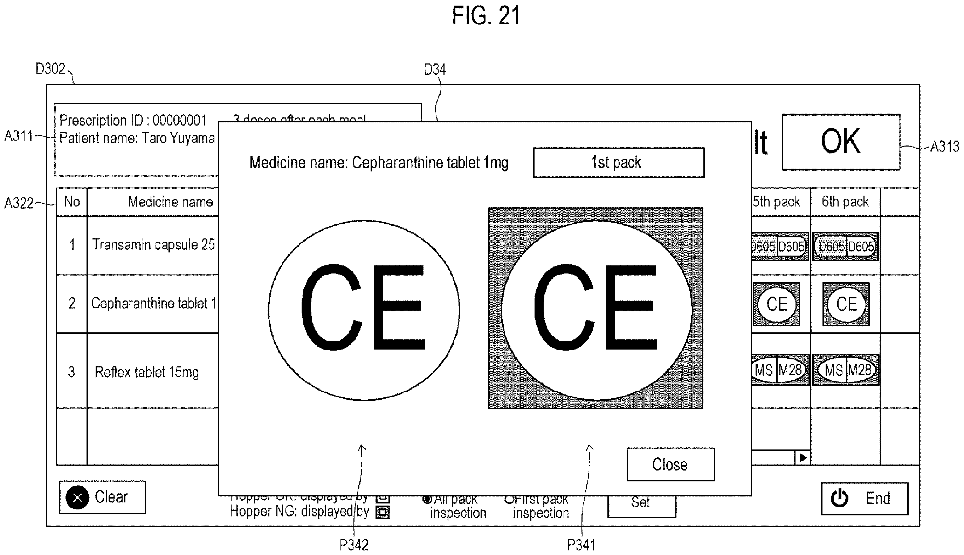

Further, when the selection manipulation of the tablet capture image displayed at the medicine preparation detail area A322 is made, as shown in FIG. 21, the control part 21 can cause the client terminal 3 to display an enlarged screen D34 in which the tablet capture image subject to the selection manipulation is enlarged and is displayed as an enlarged image P341. Further, in the enlarged screen D34, the control part 21 displays, together with the enlarged image P341, an enlarged image P342 in which the image including the identification information is enlarged among the correct images corresponding to the tablet to be manipulated as the selection manipulation. Further, an arrangement direction of the enlarged image P341 and the enlarged image P342 may be either the up-down direction or the left-right direction. Further, a plurality of images preregistered as the correct images corresponding to the tablet subject to the selection manipulation may be displayed as the enlarged image P342.

Further, it is conceivable that, at the medicine preparation detail area A322, the dosing date and dosing time are displayed together with or in place of the pack number information such as the 1 st pack, the 2nd pack and the like which shows the pack number of the medicine packs 451. Further, it is also conceivable that the control part 21 alternatively switches and displays a plurality of the capture images of the tablet (for example, a front surface image and a back surface image) in response to the selection manipulation of the tablet capture image on the medicine preparation detail area A322. Therefore, it is possible to check the state of the tablet viewed in a plurality of directions while suppressing a size of a display area at a normal time.

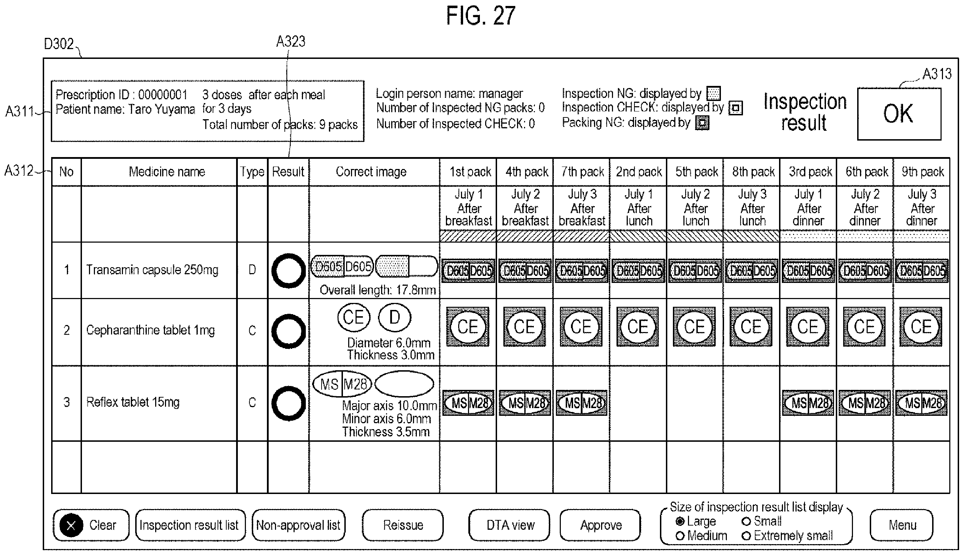

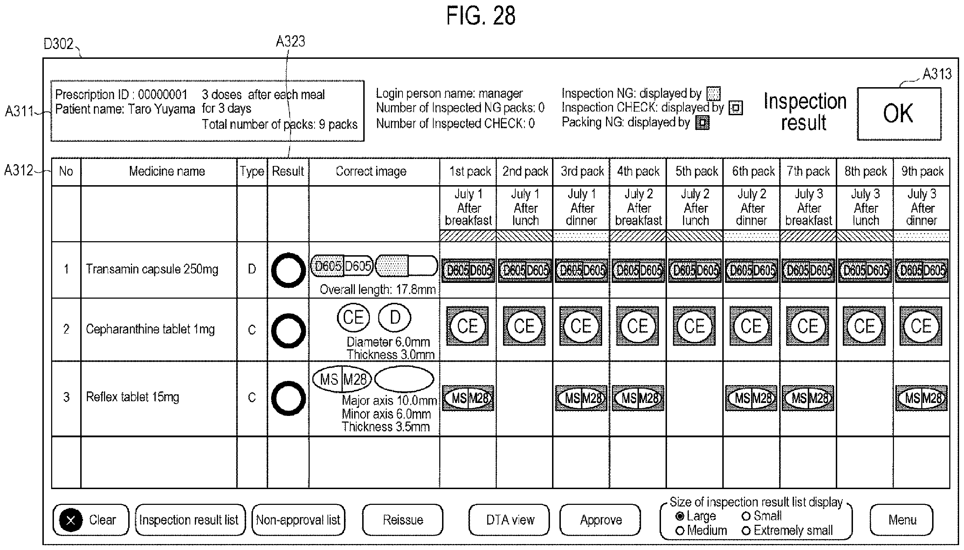

As shown in FIGS. 16, 17, 19 and 20, in the inspection detail screen D301 related to the first display form and the inspection detail screen D302 related to the second display form, the control part 21 arranges the capture images of the tablet corresponding to the plurality of medicine packs 451 dispensed in a series of packing processes in the order of the first pack, the second pack, etc., and displays the capture images of the tablet per unit of packing. In the regard, it is conceivable that the control part 21 can arbitrarily select the arrangement order of the medicine packs 451 to be displayed, from a first arrangement *(see* FIG. 27) in which the medicine packs 451 are arranged for each dosing time and a second arrangement *(see* FIG. 28) in which the medicine packs 451 are arranged in the order of the dosing date and the dosing time, in response to the user's manipulation or the like. Further, the control part 21 switches the first arrangement and the second arrangement in response to, for example, a setting manipulation in an initial setting of the server 2 or the tablet packing device 4.

For example, it is considered a case where the medicine packs 451 corresponding to 9 times of morning, daytime and evening during a period between July 1 and July 3 are dispensed based on one record of the prescription data. In this case, as shown in FIG. 27, according to the first arrangement, the capture images of the tablets packed into the medicine packs 451 are arranged and displayed in the order of the morning of July 1, the morning of July 2, the morning of July 3, the daytime of July 1, the daytime of July 2, the daytime of July 3, the evening of July 1, the evening of July 2 and the evening of July 3. On the other hand, according to the second arrangement, as shown in FIG. 28, the capture images of the tablets packed into the medicine packs 451 are arranged and displayed in the order of the morning of July 1, the daytime of July 1, the evening of July 1, the morning of July 2, the daytime of July 2, the evening of July 2, the morning of July 3, the daytime of July 3 and the evening of July 3. Thus, it is possible to change the display form of the inspection detail screen D301 or D302 in conformity with the application in the medical institution in which the inspection assistance system 1 is used, thereby realizing a system configuration which is excellent in versatility.

[Inspection Mode Switching function] Further, according to the embodiment described above, in the automatic inspection process of the tablet packing device 4, the suitability is determined with respect to the tablets of all the medicine packs 451 dispensed based on the prescription data. On the other hand, in the tablet packing device 4, it is conceivable that an inspection mode in the automatic inspection process includes a first inspection mode where the tablets of all the medicine packs 451 are inspected and a second inspection mode where the tablets of a predetermined portion of the medicine packs 451 are inspected. Further, in the initial setting of the tablet packing device 4 performed in response to the user's manipulation, the control part 21 switches the inspection mode in the automatic inspection process to one of the first inspection mode and the second inspection mode.

In this regard, it is conceivable that an inspection target in the second inspection mode is either or both of the first and last medicine packs 451 packed based on the prescription data. Further, it is also conceivable that an inspection target in the second inspection mode is either or both of the first and last medicine packs 451 corresponding to the change of a dosing pattern in the prescription data. Further, the inspection target in the second inspection mode may be the medicine pack 451 which receives the tablets whose supply source is a pre-set supply source (for example, the manual distribution unit 42 or the specific tablet cassette 41). For example, the second inspection mode is applied to the medicine pack 451 which contains at least one type of tablet whose supply source is the manual distribution unit 42 or the specific tablet cassette 41. In this manner, only the medicine pack 451 having a particularly high necessity of inspection among the medicine packs 451 can be inspected without omission, thereby improving the efficiency of the inspection task conducted by a pharmacist. Alternatively, the switching between the first inspection mode and the second inspection mode is not limited to a unit of the medicine pack 451 but may be a unit of a series of the medicine packs 451 packed based on one prescription data.

The case where the tablets of some of the medicine packs 451 are the inspection target in the second inspection mode has been described as an example. On the other hand, as another example of the second inspection mode, it is conceivable that, when the tablets are sequentially dispensed from the same tablet cassette 41 in the packing process based on the prescription data, only the tablet first dispensed from the tablet cassette 41 is the inspection target. Further, as a further example of the second inspection mode, it is conceivable that, when various types of tablets are dispensed in the packing process based on the prescription data, only the tablet first dispensed with respect to each type of tablets is the inspection target.

Further, it is conceivable that the tablet packing device 4 has a third inspection mode where the presence or absence of the automatic inspection process is automatically switched by the control part 40. In the third inspection mode, the automatic inspection process is not executed with respect to the tablets dispensed from the tablet cassette 41 in the packing process, but the automatic inspection process is executed with respect to the tablets dispensed from the manual distribution unit 42. Thus, when the manual distribution unit 42 having a high likelihood of occurrence of man-made mistakes is used, the automatic inspection process is executed to suppress medicine preparation mistakes. When the manual distribution unit 42 is not used and instead the tablet cassette 41 is used, the automatic inspection process is not executed and the time required for the packing process is shortened. Further, in the case where the automatic inspection process is not executed, the image capturing of the tablets by the camera 462 or 463 of the image capturing part 46 may be executed or may be omitted.

Further, in the third inspection mode, it is also conceivable that the inspection method is changed according to whether the manual distribution unit 42 is used in the packing process. For example, in case where the supply source of at least one type of tablet among tablets subject to the packing process and included in the prescription data is the manual distribution unit 42, i.e., in case where the manual distribution unit 42 is used in the packing process, the control part 21 executes, as the automatic inspection process, an image inspection process of collating the identification information of the tablet acquired from the capture image with the prescription data. In this regard, the control part 40 used at the time of executing the image inspection process is an example of an image inspection processing part. On the other hand, in case where the tablet cassette 41 is used in the packing process without using the manual distribution unit 42, the control part 40 executes a count inspection process of collating the number of the dispensed tablets with the prescription data. In this regard, the control part 40 used at the time of executing the count inspection process is an example of a count inspection processing part.

Specifically, in the tablet packing device 4, the control part 40 detects the number of tablets to be received in the medicine pack 451 with respect to each of the medicine packs 451 by using the passage detection sensor 475. The control part 40 used at the time of detecting the number of tablets by using the passage detection sensor 475 is an example of a tablet number detection part. Further, the passage detection sensor 475 is disposed at a position where the tablets are detected at a timing when the tablets are dispensed from the rotary unit 44 and are received in the medicine pack 451. That is to say, the control part 40 detects the number of tablets by using the passage detection sensor 475 at a downstream side from the position where the tablets are captured by the cameras 462 and 463 in a dispensing direction of the tablets. Further, it is also conceivable that, for example, the medicine pack 451 is captured with a camera or the like after the medicine pack 451 receives the tablets, and that the number of the tablets received in the medicine pack 451 is detected from the capture image of the medicine pack by the control part 40. Then, the control part 40 collates the number of tablets to be received in the medicine pack 451 with the number of tablets detected by the passage detection sensor 475, based on the prescription data.

Thus, in the case where the manual distribution unit 42 having a high likelihood of occurrence of man-made mistakes is used, the image inspection process is executed as the automatic inspection process, thereby suppressing the medicine preparation mistakes with high accuracy. On the other hand, in the case where the tablet cassette 41 is used without using the manual distribution unit 42, the image inspection process is not executed, thereby shortening the time required for the packing process, and the count inspection process is executed, thereby suppressing the medicine preparation mistakes.

Further, in case where a failure occurs in the components such as the camera 462 or 463 used for the image inspection process, it is conceivable that the tablet packing device 4 does not execute the image inspection process as the automatic inspection process, but has, as one of the inspection modes, a failure mode where the count inspection process is executed. For example, it is conceivable that, when a failure is detected in the components used for the image inspection process, the control part 40 displays a manipulation screen for allowing a user to select whether to switch to the failure mode and executes whether to switch to the failure mode in response to a user's manipulation. Thus, even if a failure occurs in the components for executing the image inspection process of the tablet packing device 4, it is possible to execute a normal packing process by means of the tablet packing device 4. Further, the failure mode may be an inspection mode where neither the image inspection process nor the count inspection process is executed.

[Display Changing Function when Front and Back Surfaces are Identical] In the inspection detail screen D301, two capture images are displayed with respect to each tablet. In the inspection detail screen D302, one capture image is displayed with respect to each tablet. Therefore, when as many tablet images as the medicine packs 451 are displayed, the size of each of the capture images is smaller in the inspection detail screen D301 than in the inspection detail screen D302.

In this regard, in case of displaying the inspection detail screen D301 regarding the tablet having the same identification information attached to the front and back surfaces, it is conceivable that the control part 21 has a display changing function of enlarging the capture image of one of the front and back surfaces of the tablet within an area where the front surface capture image and the back surface capture image are displayed, and displaying the same in the inspection detail screen D301.

For example, the control part 21 compares the identification information of the tablet acquired from each of the front and back surfaces of the tablet, thereby determining whether the identification information on the front surface of the tablet and the identification information on the back surface of the tablet are the same. Further, it is conceivable that the information on whether the front surface and the back surface are the same is preregistered in the medicine master with respect to each type of tablet, and that the control part 21 determines whether the front surface and the back surface of the tablet are the same based on the medicine master and the medicine information of the tablet included as a dispensing target in the prescription data.

According to such a configuration, in the case where the identification information attached to the front surface of the tablet and the identification information attached to the back surface of the tablet are the same, the capture image corresponding to one of the front surface and the back surface of the tablet is enlargedly displayed. Thus, it is possible to easily check the identification information of the tablet. Further, regarding the tablet with different identification information attached to the front and back surfaces, the control part 21 displays the capture images corresponding to both the front and back surfaces of the tablet in the inspection detail screen D301.

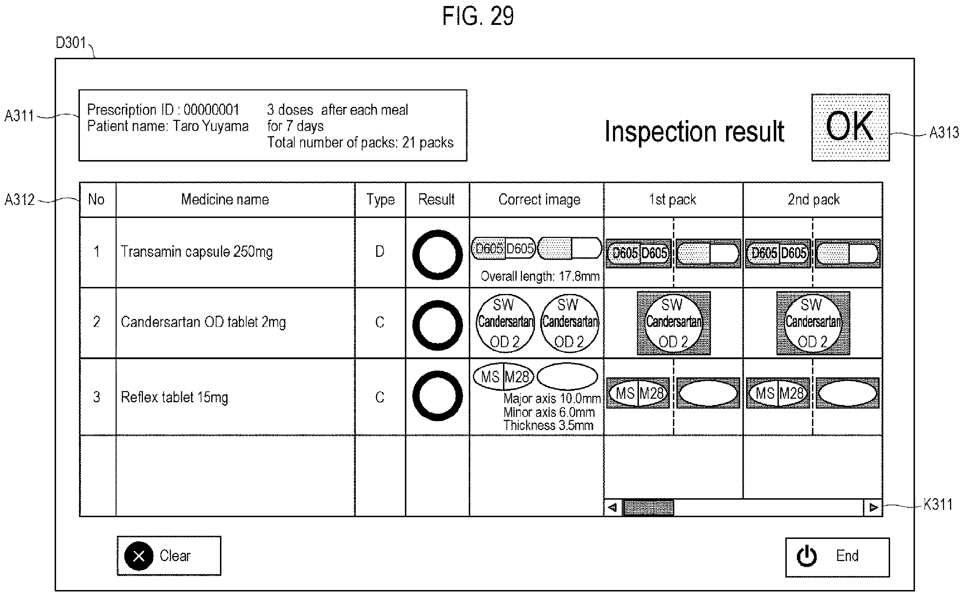

For example, among the tablets shown in FIGS. 16 and 19, "Cepharanthine tablet 1 mg" has different identification information attached to the front and back surfaces. However, for example, regarding the tablet such as "Candesartan OD tablet 2 mg", the identification information attached to the front surface and the identification information attached to the back surface are the same. Therefore, in case where a tablet subject to being packed is "Candesartan OD tablet 2 mg", as shown in FIG. 29, only one of the front surface and the back surface of the tablet is displayed in the inspection detail screen D301. Further, in the example shown in FIG. 29, the correct images of both the front surface and the back surface are displayed so that the identification information on the front surface and the identification information on the back surface can be checked to be the same with respect to the correct images of the tablets. On the other hand, it is also conceivable that only one of the front surface and the back surface of the tablet is displayed with respect to the correct images.

[On-error Image Display Function] As described above, in the inspection assistance system 1, the result of the automatic inspection process and the capture image of the tablet are displayed in the inspection detail screen D301 or the inspection detail screen D302. Further, in the enlarged screen D33 or the enlarged screen D34, an enlarged picture of the capture image of the tablets received in each medicine pack can be displayed.

On the other hand, it is conceivable that the inspection assistance system 1 has an on-error image display function of, when the result of the automatic inspection process is an error, displaying plural types of capture images obtained by capturing the error-causing tablet at different angles. Hereinafter, descriptions are first made as to a case where the server 2 has the on-error image display function. Then, descriptions are made as to a case where the tablet packing device 4 has the on-error image display function.

Specifically, in the server 2, when the result of the automatic inspection process is an error, the control part 21 can display a plurality of capture images, which correspond to different outer peripheral surfaces of the tablet and are captured with respect to each tablet in the tablet packing device 4, as the capture images of the error-causing tablet with respect to each tablet. Such a display process is executed by the inspection display processing part 211 of the control part 21.

Specifically, in the tablet packing device 4, as described above, the image capturing by the camera 462 and the camera 463 is performed at pre-set image capturing interval (for example, at an interval of several milliseconds). Therefore, a plurality of capture images acquired by capturing different regions on the outer peripheral surface of the tablet are obtained as the capture images of the tablet which is rotated in the tablet rotation part 441 of the rotary unit 44. Further, the image capturing interval is an interval set in advance so that a plurality of different regions on the outer peripheral surface of the tablet are captured by the camera 462 and the camera 463. Then, in the automatic inspection process using the identification information of the tablet read from the capture images, the identification information of the tablet is read from any one of a plurality of the capture images of the tablet captured with respect to each tablet, and determination is made as to whether the identification information matches the prescription data. For example, in the medicine master, the identification information formed by engraving or printing is registered with respect to each tablet. In the automatic inspection process, the medicine name of the tablet is specified based on the identification information of the tablet read from the medicine master and the capture images, and it is determined whether the medicine name matches the medicine name of the tablet included in the prescription data. Further, in the automatic inspection process performed by using the capture images and the correct images, one of a plurality of the capture images of the tablet captured with respect to each tablet is compared with the correct image to determine whether they match, or how much they match.

Further, when the result of the automatic inspection process is proper, the control part 40 transmits one or a plurality of the capture images, which include an original image whose tablet identification information is read from the tablet or an original image compared with the correct image among the capture images of the tablet captured by the camera 462 or the camera 463, to the server 2 together with the result of the automatic inspection process. On the other hand, when the result of the automatic inspection process is an error, the control part 40 transmits a plurality of the capture images, in which a plurality of different regions on the outer peripheral surface of the tablet are captured, among the capture images of the tablet captured by the camera 462 or the camera 463, to the server 2 together with the result of the automatic inspection process. Further, all the capture images of each of the tablets may be transmitted from the tablet packing device 4 to the server 2.

The error of the automatic inspection process includes: a first error where the tablet specified by the identification information included in the capture image does not match the tablet included in the prescription data; and a second error where the identification information included in the capture image does not match the identification information of all the tablets registered in the medicine master. Thus, similar to the case where the result of the automatic inspection process is proper, it is conceivable that, regarding the tablet whose result of the automatic inspection process is the first error, the control part 40 transmits the capture image of the tablet used when the identification information is read or the capture image compared with the correct image to the server 2. Further, it is conceivable that, regarding the tablet whose result of the automatic inspection process is the second error, the control part 40 transmits, to the server 2, a plurality of capture images, in which a plurality of different regions on the outer peripheral surface of the tablet are captured, among the capture images of the tablet.

As the capture image is transmitted from the tablet packing device 4 to the server 2 as described above, the control part 21 can display one or a plurality of the capture images of the tablet when the control part 21 causes the display part 24 of the server 2 or the display part 34 of the client terminal 3 to display the inspection detail screen D301 *(see* FIG. 17) or the inspection detail screen D302 *(see* FIG. 20). Further, the control part 21 causes the storage part 22 to store each of the capture images acquired from the tablet packing device 4 in association with the prescription data together with the result of the automatic inspection process.

Specifically, it is conceivable that the control part 21 causes the inspection detail screen D301 or the inspection detail screen D302 to display at least the capture image used when the identification information of the tablet is read or the capture image compared with the correct image with respect to the tablet whose result of the automatic inspection process is proper or the first error. Further, it is conceivable that the control part 21 causes the inspection detail screen D301 or the inspection detail screen D302 to display an arbitrary capture image among the capture images of the tablet with respect to the tablet whose result of the automatic inspection process is the second error. For example, it is conceivable that, with respect to tablet whose result of the automatic inspection process is the second error, the control part 21 displays one or both of the first capture image and the last capture image among the capture images of the tablet. Further, it is conceivable that, with respect to the tablet whose result of the automatic inspection process is the second error, the control part 21 displays the capture image including the identification information having a high degree of pattern matching with the identification information of the tablet among the capture images of the tablet or displays the capture image, in which a character is detected, among the capture images of the tablet.

Further, when a user's manipulation for selecting any one of the capture images of the tablet, whose result of the automatic inspection process is proper, is made on the inspection detail screen D301 or the inspection detail screen D302, the control part 21 causes the enlarged image of the tablet capture image to be displayed in the enlarged screen D33 *(see* FIG. 18) and the enlarged screen D34 *(see* FIG. 21). Further, the user's manipulation is, for example, a manipulation for selecting the capture image of the tablet, whose result of the automatic inspection process is proper, on the inspection detail screen D301 or the inspection detail screen D302.

On the other hand, when a user's manipulation for selecting any one of the capture images of the tablet, whose result of the automatic inspection process is an error, is made on the inspection detail screen D301 or the inspection detail screen D302, the control part 21 displays an error detail screen D35 in which a plurality of capture images of such a tablet is displayed. Further, the user's manipulation is, for example, a manipulation for selecting the capture image of the tablet, whose result of the automatic inspection process is an error, in the inspection detail screen D301 or the inspection detail screen D302. Further, similar to the case where the result of the automatic inspection process is proper, it is conceivable that, regarding the tablet whose result of the automatic inspection process is the first error, the control part 21 displays the enlarged screen D33 or the enlarged screen D34 instead of the error detail screen D35.

Further, in case where the result of the automatic inspection process is an error, it is conceivable that the control part 21 causes the error detail screen D35 to display which one of the first error and the second error the error is. For example, in case where the result of the automatic inspection process is the first error, it is conceivable that the control part 21 causes the error detail screen D35 to display the medicine name or the like corresponding to the identification information read from the tablet capture image, together with a message such as "medicine mistake" indicating that the error is the first error. Further, in case where the result of the automatic inspection process is the second error, it is conceivable that the control part 21 causes the error detail screen D35 to display a message such as "medicine unidentified" indicating that the error is the second error.

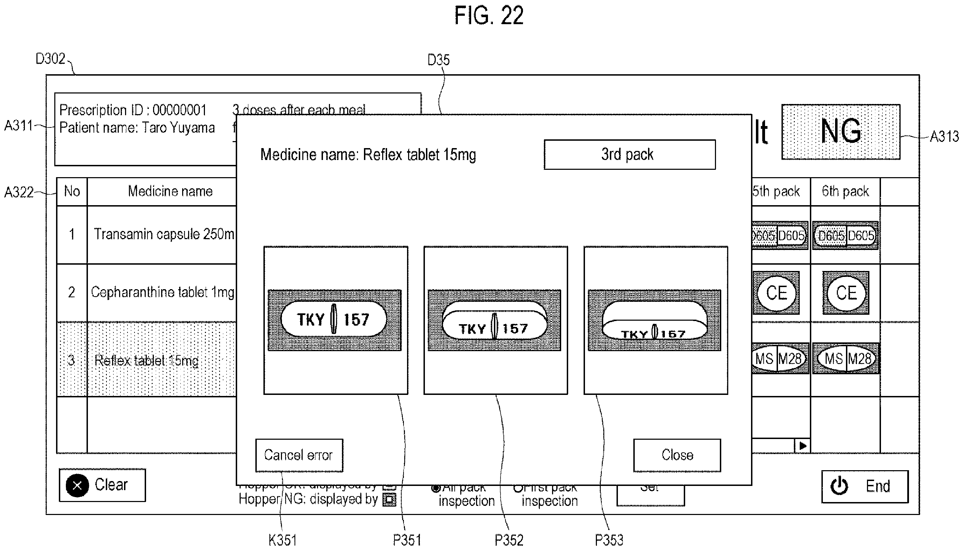

In this regard, FIG. 22 shows an example of the error detail screen D35. As shown in FIG. 22, in the error detail screen D35, together with the medicine name of the tablet selected on the inspection detail screen D301 or the inspection detail screen D302, a plurality of capture images P351 to P353, in which the different regions on the outer peripheral surface of the tablet are captured, are displayed as the capture images of the tablet whose result of the automatic inspection process is an error. At this time, the control part 21 causes the error detail screen D35 to display a predetermined number of capture images continuously captured at the image capturing interval or a plurality of capture images corresponding to a predetermined interval among a plurality of the capture images corresponding to the tablet, thereby displaying the capture images of the different regions on the outer peripheral surface of the tablet. Particularly, it is conceivable that, regarding the tablet whose result of the automatic inspection process is the first error, the control part 21 causes the error detail screen D35 to display one or a plurality of the capture images including the capture image used when the identification information is read or the capture image compared with the correct image. Thus, a pharmacist can determine the suitability by, for example, referring to a plurality of the capture images of the tablet displayed in the error detail screen D35.

For example, it is conceivable that a rotation correspondence information, which indicates a correspondence relationship between a rotation amount (rotation time) of the roller 100 and a rotation amount of the tablet in the tablet rotation part 441, is stored in the storage part 22, and that the control part 21 selects a plurality of capture images in which the different regions on the outer peripheral surface of the tablet are captured, based on the rotation correspondence information and the image capturing interval. Specifically, in case where the image capturing by the camera 462 or the camera 463 is performed every 1ms and it is known in the rotation correspondence information that the tablet is rotated by 10° every 10ms, it is conceivable that the control part 21 selects a plurality of capture images captured every 45ms or selects a plurality of capture images captured every 90ms. Thus, the control part 21 selects a plurality of capture images in which the tablet positions are different at each 45° or each 90° regarding the outer peripheral surface of the tablet. Further, in the tablet packing device 4, in case where the interval of the image capturing by the camera 462 or the camera 463 is 45ms, the control part 40 selects a plurality of consecutive capture images, thereby selecting a plurality of capture images in which rotation angles of the tablet are different at each 45°.

In this manner, in the inspection assistance system 1, when an error is included in the result of the automatic inspection process, the control part 21 can display the error detail screen D35 in which a plurality of the capture images with the different regions captured on the outer peripheral surface of the tablet are displayed. Accordingly, when the result of the automatic inspection process is an error, a pharmacist can grasp the reason of the error by referring to the plurality of capture images.

While three capture images P351 to P353 are displayed in the error detail screen D35 shown in FIG. 22, the number of the capture images displayed in the error detail screen D35 is not limited thereto. For example, it is conceivable that two capture images with the different regions captured on the outer peripheral surface of the tablet are displayed, or four or more of such capture images are displayed. Further, it is also conceivable that the control part 21 displays a plurality of the capture images one image at a time or by plural images at a time in response to a flick manipulation in the left-right direction or the up-down direction on the error detail screen D35. Further, it is also conceivable that a slide bar capable of being slidably manipulated in the left-right direction or the up-down direction is displayed in the error detail screen D35, and that the control part 21 sequentially displays a plurality of the capture images of the tablet one image at a time or by plural images at a time in response to the slide manipulation of the slide bar.

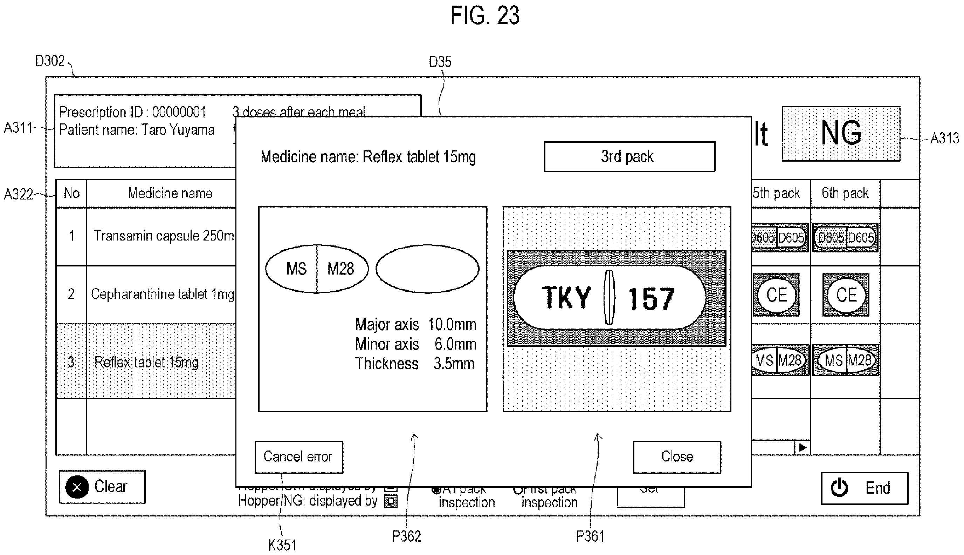

Further, it is conceivable that, in case where any one of the capture images displayed in the error detail screen D35 is selected, the control part 21 displays an enlarged screen D36 for enlarging and displaying the selected capture image. In this regard, FIG. 23 shows an example of the enlarged screen D36. Specifically, in the enlarged screen D36 shown in FIG. 23, similar to the enlarged screen D34 *(see* FIG. 18), a capture image P361 of a selected tablet and an enlarged image P362 of the correct image corresponding to the selected tablet are displayed side by side in the up-down direction or the left-right direction. Further, it is also conceivable that, in the error detail screen D35, the enlarged image P362 is displayed together with the capture images P351 to P353.

Further, as shown in FIG. 1, the control part 21 includes a cancellation processing part 215 capable of cancelling the error of the error-causing the tablet in response to a user's manipulation which is made after the capture image of the error-causing tablet in the automatic inspection process is displayed. Further, while cancelling the error, the cancellation processing part 215 can record, in association with the prescription data, an identification information of a user at the time of the user's manipulation. Further, the control part 21 functions as the cancellation processing part 215 by executing various processes according to the inspection assistance program.

Specifically, as shown in FIGS. 22 and 23, in the error detail screen D35 and the enlarged screen D36, an error cancellation key K351 for canceling the error, which is the result of the automatic inspection process, is displayed. Then, when the error cancellation key K351 is manipulated, the control part 21 cancels the error which occurs with respect to the tablet of the currently-displayed medicine pack among the results of the automatic inspection process of the prescription data. Thus, for example, when a pharmacist can check that there is no problem by referring to each of the capture images of the tablet, a pharmacist manipulates the error cancellation key K351. Thus, even when an error occurred in the automatic inspection process on the prescription data, it is possible to normally terminate the inspection of the prescription data.

Further, when the error cancellation key K351 is manipulated, the control part 21 causes the storage part 22 to record the purport of error cancellation and the identification information (name or ID) of a pharmacist who is a currently logged-in user, in association with the prescription data, together with the result of the automatic inspection process. Thus, the control part 21 can output (display) the information on the user who has canceled the error among the results of the automatic inspection process on the prescription data. Further, it is conceivable that the control part 21 causes the storage part 22 to record information such as a manipulation date of the error cancellation key K351 or an arbitrarily-inputted comment on the reason for cancellation of the error, in association with the prescription data, together with the result of the automatic inspection process.

Further, it is conceivable that, when the error cancellation key K351 is manipulated, the control part 21 does not cancel the error and causes the storage part 22 to record the purport that a check manipulation has been made with respect to the error (the decision result of the user) and an identification information of a pharmacist who is the user making the check manipulation, in association with the prescription data, together with the result of the automatic inspection process. Thus, the control part 21 can output (display) the information such as the following: the result of the automatic inspection process in the tablet packing device 4; the determination result of the user indicating whether the check manipulation is made by the pharmacist; and the identification information of the pharmacist who has made the check manipulation.

Further, in the inspection assistance system 1, the result of the automatic inspection process on the prescription data and the data of the capture images are stored in the storage part 22 of the server 2. In this regard, it is conceivable that, at the time of terminating the inspection assistance process *(see* FIG. 7) (S26: Yes), the control part 21 erases the capture image of the tablet, whose error is cancelled by the manipulation of the error cancellation key K351, among the data of the capture images stored in the storage part 22. Thus, it is possible to reduce a storage capacity that needs to be provided in the storage part 22. Further, it is conceivable that the control part 21 causes the storage part 22 to accumulate only one or a plurality of capture images arbitrarily selected by the user's manipulation on the error detail screen D35 among the capture images of the tablet whose error has been canceled by the manipulation of the error cancellation key K351, and to erase other capture images.

Next, descriptions are made as to a case where the tablet packing device 4 has the on-error image display function. Specifically, as shown in FIG. 1, the tablet packing device 4 includes a manipulation display part 48 such as a liquid crystal display, a touch panel or the like, and a storage part 49 such as a hard disk, an SSD or the like. Similar to the above-described control part 21, the control part 40 can cause the manipulation display part 48 to display the inspection detail screens D301 and D302, the enlarged screens D34 and D35, the error detail screen D35, the enlarged screen D36, etc. That is to say, when the result of the automatic inspection process is an error, the control part 40 can cause the manipulation display part 48 to display the error detail screen D35 in which, as capture images of the error-causing tablet, a plurality of capture images, which correspond to the different outer peripheral surfaces of the tablet and are captured with respect to each tablet in the tablet packing device 4, are displayed with respect to each tablet. In this regard, the control part 40 used at the time of executing such a process is an example of an inspection display processing part.

Further, it is conceivable that, regarding the tablet whose result of the automatic inspection process is the first error, similar to the case where the result of the automatic inspection process is proper, the control part 40 causes the manipulation display part 48 to display the capture image of the tablet used when the identification information is read or the capture image of the tablet compared with the correct image. Further, it is conceivable that, regarding the tablet whose result of the automatic inspection process is the second error, the control part 40 causes the manipulation display part 48 to display a plurality of capture images in which a plurality of different regions on the outer peripheral surface of the tablet are captured among the capture images of the tablet.

Further, it is conceivable that the control part 40 sequentially displays a plurality of tablet capture images one image at a time or by plural images at a time in response to a flick manipulation in the left-right direction or the up-down direction on the error detail screen D35. Further, it is also conceivable that a slide bar capable of being slidably manipulated in the left-right direction or the up-down direction is displayed in the error detail screen D35, and that the control part 40 sequentially displays a plurality of tablet capture images one image at a time or by plural images at a time in response to the slide manipulation of the slide bar.

Further, when the error cancellation key K351 is manipulated, the control part 40 causes the storage part 49 to record the purport of error cancellation and the identification information (name or ID) of the pharmacist who is the user currently logging in the tablet packing device 4, in association with the prescription data, together with the result of the automatic examination process. Further, when transmitting the result of the automatic inspection process and the capture images of the tablet to the server 2, the control part 40 also transmits the purport of error cancellation and the identification information of the pharmacist. Thus, in the server 2, when outputting (displaying) the result of the automatic inspection process on the prescription data, the control part 21 can output (display) the purport of error cancellation, the identification information of the pharmacist, etc..

Further, it is conceivable that, when the error cancellation key K351 is manipulated, the control part 40 does not cancel the error and causes the storage part 49 to record the purport that the check manipulation on the error has been made and the identification information of the pharmacist who is the user having made the check manipulation, in association with the prescription data, together with the result of the automatic examination process. Thus, the control part 40 can output (display) the information such as the following: the result of the automatic inspection process in the tablet packing device 4; the presence or absence of the check manipulation by the pharmacist; and the identification information of the pharmacist having made the check manipulation. For example, when transmitting the result of the automatic inspection process and the capture images of the tablet to the server 2, the control part 40 also transmits the purport that the check manipulation on the error has been made and the identification information of the pharmacist.

Further, in the tablet packing device 4, the result of the automatic inspection process on the prescription data and the data of the capture images are stored in the storage part 49 by the control part 40. Therefore, it is conceivable that, at the time of terminating the inspection assistance process (see FIG. 7) (S26: Yes), the control part 21 erases the capture image of the tablet, whose result of the automatic inspection process is normal, and the capture image, whose error is cancelled by the manipulation of the error cancellation key K351, among the data of the capture images stored in the storage part 22. Thus, it is possible to reduce the storage capacity that needs to be provided in the storage part 22. Further, it is conceivable that the result of the automatic inspection process and the data of the capture images are accumulated in one of the storage part 22 of the server 2 and the storage part 49 of the tablet packing device 4, and the result of the automatic inspection process and the data of the capture images are erased in the other of the storage part 22 of the server 2 and the storage part 49 of the tablet packing device 4 at the time of terminating the inspection assistance process.

[Printing Inspection Function] In the tablet packing device 4, a character information such as a patient name, a dosing time, a medicine name or a prescription quantity may be printed on the medicine pack 451 by the printing unit 453. Further, a medicine examination code for determining the suitability of the packing process when the medicine pack 451 is packed may be printed on the medicine pack 451 by a one-dimensional code or a two-dimensional code. In this regard, it is conceivable that the tablet packing device 4 has a printing inspection function of automatically inspecting whether the printing on the medicine pack 451 by the printing unit 453 is performed normally.

Specifically, as shown in FIG. 1, the tablet packing device 4 includes an image reading part 50 configured to read an image of the medicine pack 451. The image reading part 50 is disposed at a downstream side in a movement direction of the medicine pack 451 in the packing unit 45 from a position where the printing on the medicine pack 451 is performed by the printing unit 453. More specifically, the image reading part 50 is provided at a position which is at a downstream side in the movement direction of the medicine pack 451 from the position where the printing on the medicine pack 451 is performed by the printing unit 453, and at which the tablets are not yet received in the medicine pack 451. Further, the image reading part 50 may be provided at a position where the tablets are already received in the medicine pack 451.

The image reading part 50 includes an image sensor such as a CCD or a CIS that reads an image from the medicine pack 451. Based on the image read by the image reading part 50, the control part 40 acquires a character information printed on the medicine pack 451 by a well-known character recognition process (OCR) or the like. Further, in case where the medicine examination code is included in the image read by the image reading part 50, the control part 40 reads a medicine examination information from the medicine examination code.

Thereafter, the control part 40 collates the character information or the medicine examination information, which is the printing contents of the medicine pack 451 read by the image reading part 50, with a character information or a medicine examination information which are to be printed on the medicine pack 451. Then, if a collation result indicates non-coincidence, the control part 40 notifies a user of the purport of non-coincidence by displaying the purport of non-coincidence on the manipulation display part 48, and records the purport of non-coincidence as a result of a printing inspection process in the storage part 49 in association with the prescription data. Thus, it is possible to automatically find a printing omission or a printing mistake in the medicine pack 451 and to assist an inspection task of the printing contents of the medicine pack 451 conducted by a pharmacist.

The collation process of the printed contents may be executed not only by the tablet packing device 4 but also by the server 2. Specifically, the image of the medicine pack 451 read by the image reading part 50 is inputted from the tablet packing device 4 to the server 2. Then, the control part 21 of the server 2 determines the suitability of the information printed on the medicine pack 451, based on the prescription data and the image of the medicine pack 451 read by the image reading part 50 in the tablet packing device 4.

[Medicine Reuse Function] Regarding the medicine pack 451 including the tablet, the inspection result of the automatic inspection process executed in the tablet packing device 4 is an error, it is necessary to re-execute the packing process in the tablet packing device 4. At this time, in case where a plurality of tablets are contained in the medicine pack 451 and the inspection result of the automatic inspection process is an error with respect to only a portion of the tablets, the tablets whose inspection result is proper are discarded unnecessarily. Otherwise, it is necessary to perform a task of returning the tablets, whose inspection result is proper, to the tablet cassette 41 corresponding to such tablets. Thus, in the case where a plurality of tablets are contained in the medicine pack 451 and the inspection result of the automatic inspection process is an error with respect to only a portion of the tablets, it is conceivable that the tablet packing device 4 has a medicine reuse function capable of assisting the reuse of the tablets, whose inspection result is proper, in the re-execution of the packing process.

Specifically, when re-executing the packing process with respect to the medicine pack 451 including the tablet whose inspection result is an error, the control part 40 determines whether the tablets whose inspection result is proper are contained in the already-packed medicine pack 451. Then, in the case where the tablets whose inspection result is proper are contained in the medicine pack, the control part 40 causes the manipulation display part 48 to display a guide notice that urges a user to put such tablets into the manual distribution unit 42. For example, as for the guide notice, a guidance related to which of the tablets received in the medicine pack 451 is to be put into which position is displayed by an image, a character, or the like.

Then, when re-executing the packing process on the medicine pack 451, the control part 40 executes the packing process by using the tablets supplied in the manual distribution unit 42. Thus, the tablets whose inspection result is proper can be effectively used without being discarded, and the user's task of returning the tablets to the tablet cassette 41 is also unnecessary. Further, the tablet of the type whose inspection result is not proper is supplied from the tablet cassette 41, if the tablet cassette 41 corresponding to such a tablet exists. On the other hand, if the tablet cassette 41 corresponding to the tablet of the type whose inspection result is not proper does not exist, the purport that the tablet of the type whose inspection result is not proper is put into the manual distribution unit 42 together with the tablet the inspection result of is proper is displayed in the guide notice, and the packing process is executed by means of the manual distribution unit 42.

Further, even when the packing process is re-executed, the control part 40 executes the automatic inspection process and transmits the inspection result of the automatic inspection process and the capture images to the server 2. In this case, in the server 2, the control part 21 displays the inspection result of the automatic inspection process on the re-executed packing process in the inspection detail screen D31, D301 or D302. For example, it is conceivable that, in the inspection detail screen D31, D301 or D302, the control part 21 can sequentially switch and display, automatically or in response to a user's manipulation, the inspection results of the automatic inspection processes executed in the past on the same prescription data.

Further, it is also conceivable that the control part 21 rewrites and displays the latest inspection result of the automatic inspection process only with respect to the tablets which are subjected to re-execution of the packing process among the past inspection results of the automatic inspection process. Further, even in this case, it is conceivable that the control part 21 accumulates the inspection results of the automatic inspection process on the same prescription data and stores the accumulated inspection results in the storage part 22. Thus, in the server 2, it is possible to refer to the history of the inspection results of the automatic inspection process.

[Capsule Image Processing Function] In case where the tablet to be dispensed by the packing process is a capsule tablet, the automatic inspection process is executed based on the identification informations attached to two left and right capsule pieces constituting the capsule tablet. However, in case where engraved or printed identification informations exist on the two left and right capsule pieces constituting the capsule tablet, the position of the identification informations may be out of alignment in a circumferential direction.

Therefore, in the case where the tablet is a capsule tablet, it is conceivable that, in the automatic inspection process, the control part 40 has a capsule image processing function of acquiring capture images (divided images) of the respective capsule pieces of the capsule tablet.

Specifically, the control part 40 determines whether the tablet is a capsule tablet, according to the transition of the areas of tablet in a plurality of the capture images obtained by capturing the tablet. For example, in case where the ratio of a maximum value and a minimum value of the areas of tablet in the respective capture images is equal to or less than a preset threshold value, i.e. in case where the variation of the areas of tablet is extremely small, it is conceivable that it is determined that the tablet is a capsule tablet. Further, in case where a line segment dividing the tablet exists in the respective tablet capture images or in case where the left and right images of the tablet are different in color, it is also conceivable that it is determined that the tablet is a capsule tablet. Further, the control part 40 may determine whether the tablet is a capsule tablet, based on the medicine master or the medicine database.

Then, when it is determined that the tablet is a capsule tablet, the control part 40 divides the image of the capsule tablet in the respective capture images into the respective capsule pieces and acquires divided images. For example, the divided images are images obtained by dividing the capture image along the line segment indicating a boundary line between the capsule pieces or dividing the capture image along a center of the capsule tablet. Thereafter, in the automatic inspection process, the control part 40 collates the suitability of the capsule tablet based on the divided images of the respective capsule pieces. Specifically, it is conceivable that a correct division image bisected into two right and left pieces or a correct division image quadrisected into four upper, lower, left and right pieces are stored as a correct image of the capsule tablet in the medicine master, and that the control part 40 collates, with the correct division image, each of the divided images obtained by bisecting the capsule tablet capture image into two right and left pieces or each of the divided images obtained by quadrisecting the capsule tablet capture image four upper, lower, left and right pieces. Further, the suitability of the capsule tablet may be collated by individually reading the identification information of the tablets included in the divided images and comparing the read identification informations with the identification information of the tablet included in the correct division image.

Further, the control part 40 transmits at least the divided image compared with the correct division image or the divided image used when the identification information is read, among the divided images of the respective capsule pieces, to the server 2, together with the result of the automatic inspection process. Further, in the server 2, it is conceivable that the control part 21 composes the divided images, which includes the identification informations of the respective capsule pieces of the capsule tablet, as a capture image of the capsule tablet, and creates one composite image, and then causes the composite image to be displayed in the inspection detail screen D302. Thus, a user can check the identification informations of the respective capsule pieces of the capsule tablet on the inspection detail screen D302 through the composite image.

Further, in case where two images are displayed like the inspection detail screen D301, it is conceivable that the control part 21 causes the inspection detail screen D301 to display the image of the capsule tablet in which the identification information of the right side of the capsule tablet exists and the image of the capsule tablet in which the identification information of the left side of the capsule tablet exists.

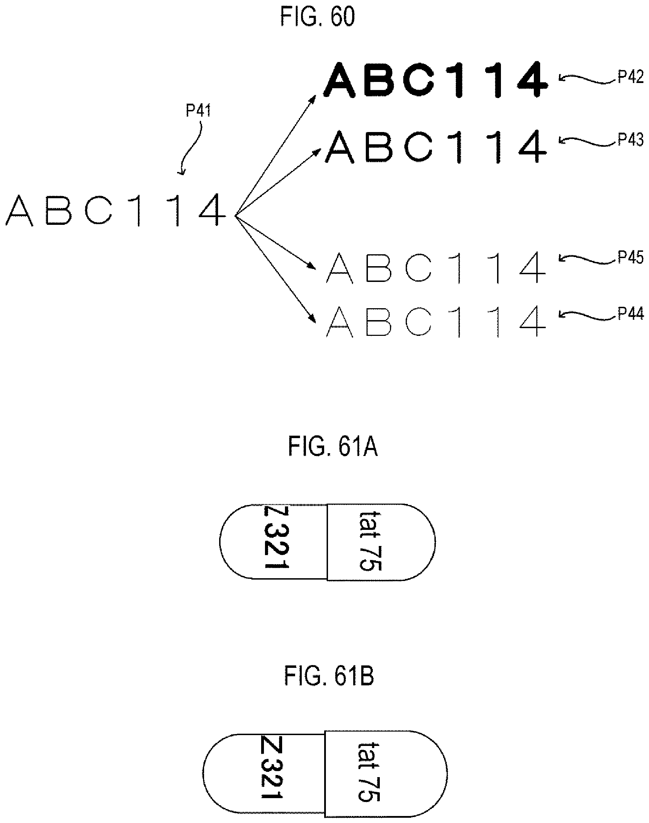

Further, when the tablet to be dispensed by the packing process is a capsule tablet, it is conceivable that the character of the identification information is formed along the circumferential direction of the capsule tablet. In this regard, FIG. 61A shows an example of the capsule tablet capture image in which "Altat75" and "TZ321" as the characters of the identification information are formed along the circumferential direction in the respective left and right capsule pieces. As shown in FIG. 61A, only a portion of the characters of the identification information of the capsule tablet appears in the respective capture images of the capsule tablet.

Therefore, it is conceivable that, regarding the capsule tablet in which the characters of the identification information are formed along the circumferential direction, the control part 40 composes a plurality of capture images corresponding to different positions in the circumferential direction of the capsule tablet to create a composite image, and decides the suitability of the capsule tablet by using the composite image. These features are described hereinafter. In this regard, it is assumed that, a correct division image including the characters of "Altat75" and a correct division image including the characters of "TZ321" are stored in the medicine master as the identification information corresponding to the respective capsule pieces of the capsule tablet.

Specifically, the control part 40 executes a distortion correction process, a combined characteristic point extraction process, an image composing process, a boundary removal process and the like with respect to a plurality of the capsule tablet capture images captured by the camera 462 or 463 in the packing process.

First, the capsule tablet has a cylindrical columnar shape. Thus, at a capsule tablet of the capture images, the characters of the identification information are distorted at the end portion in a lateral direction perpendicular to the longitudinal direction of the capsule tablet. Therefore, in the distortion correction process, the control part 40 corrects the character distortion of the identification information by expanding each of the capture images in the lateral direction of the capsule tablet. More specifically, a shrinkage degree of the characters of the identification information in the lateral direction becomes larger from a center the capsule tablet in the lateral direction toward the end portions in the lateral direction. Therefore, in the distortion correction process, the control part 40 executes the correction process of the capture image by adding a weighted value preset based on a diameter of the capsule tablet or the like such that an expansion degree of the characters of the identification information in the lateral direction becomes larger from the center of the capsule tablet in the lateral direction toward the end portions in the lateral direction. For example, it is conceivable that the control part 40 sets target points at such spacings that gradually narrow from the center toward the end portions in the lateral direction of the capsule tablet in the capture image, and then expands the capture image such that the target points are equispaced. Thus, for example, as shown in FIG. 61B, a capture image in which the character distortion of the identification information is corrected is obtained.

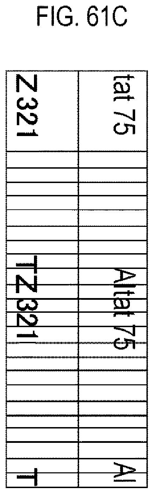

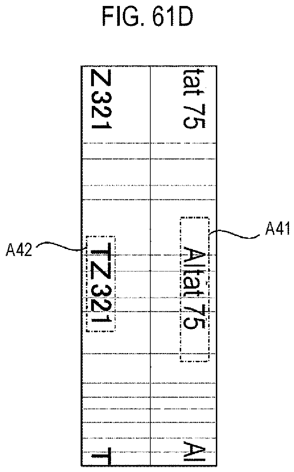

Next, in the combined characteristic point extraction process, the control part 40 trims an image, which has a preset necessary range including the identification information, from each of the capture images after the distortion correction process, and extracts characteristic points for composing the respective capture images from the respective trimmed capture images. Then, in the image composing process, the control part 40 creates a composite image by sequentially superimposing and composing the respective capture images based on the characteristic points. As a result, for example, as shown in FIG. 61C, a composite image in which the respective capture images are composed is obtained. Further, in the boundary removal process, the control part 40 executes a process of removing, from the composite image, boundary lines created due to the composition of the capture images. For example, in the boundary removal process, the boundary lines are removed by using a well-known gradient domain technique. As a result, for example, as shown in FIG. 61D, a capture image in which the character distortion of the identification information is corrected is obtained.

Then, the control part 40 decides the suitability of the capsule tablet by executing the image inspection process using the composite image as the capture image. Specifically, the control part 40 extracts character regions (for example, areas A41 and A42 in FIG. 61D) from the composite image, and decides the suitability of the characters of the identification information by a process of matching the respective images of the character regions and the correct division images. Thus, in the tablet packing device 4, even with respect to the capsule tablet in which the characters of the identification information are formed along the circumferential direction, it is possible to determine the suitability of the capsule tablet by the image inspection process.

[Omission display function] Further, it is conceivable that, in case where the identification information of the tablet is not included in at least one of the two capture images of the tablet in the inspection detail screen D301, the control part 21 has an omission display function of omitting the displaying of the capture image which does not include the identification information of the tablet, and enlarging and displaying the other capture image. In particular, it is conceivable to exclude a pre-set information of specific identification information from the respective tablet capture images in the inspection detail screen D301, and to determine the presence or absence of the identification information in the respective capture images.

Specifically, it is conceivable that a manufacturer name or mark of the tablet is set as the information of specific identification information in the medicine master and is stored in the storage part 22. On the other hand, the control part 21 obtains, as the identification information of the tablet, the information, which excludes the information of specific identification information, from the identification information of the tablet acquired from the tablet capture image. Thus, in case where only the specific identification information is included in the tablet capture image, the identification information of the tablet is not acquired from the tablet capture image.

Then, in case where the identification information is included in the capture images of the front and back surfaces of the tablet, the control part 21 displays the capture images of the front and back surfaces on the inspection detail screen D301. On the other hand, in case where the identification information is not included in one of the capture images of the front and back surfaces of the tablet, the control part 21 omits the displaying of the capture image not including the identification information, and displays only the capture image including the identification information on the inspection detail screen D301. At this time, the control part 21 enlarges and displays the capture image within the area where the capture images of the front and back surfaces are displayed. Further, in case where only the specific identification information exists on both the front and back surfaces of the tablet, only the capture image of one of the front and back surfaces of the tablet is enlarged and displayed.

Thus, in case where only the specific identification information exists in one of the front surface or the back surface of the tablet, the displaying of the capture image of the surface on which only the specific identification information exists is omitted, and the capture image of the other surface is enlarged. This enables a pharmacist to easily check the identification information of the tablet.

[Information Registration Function] In the inspection assistance system 1, various tablet images preregistered in the medicine master are used for collation and result display in the automatic inspection process. On the other hand, as for new medicines which are not registered in the medicine master, it is necessary to appropriately register information such as images of the new medicines in the medicine master. Therefore, in the inspection assistance system 1, the tablet packing device 4 has an information registration function of assisting a registration task for registering the identification information of the tablet, the tablet shape information and the like in the medicine master. Further, the information of the medicine master which is registered by the tablet packing device 4, is also transmitted to the server 2 and is also used in the server 2.

Specifically, the information registration function is realized by executing an information registration process by the control part 40. In the information registration process, the identification information of the tablet is acquired from capture images, which satisfy pre-set conditions, among a plurality of the capture images of the tablet, is registered in the medicine master or the like as a collation source data corresponding to the types of the tablets. Specifically, in the collation source data, the image of the tablet, which includes at least a region of the tablet identification information, among the capture images, is included as the correct image. In the image inspection process, the suitability of the tablet is decided by the collation of the correct image with the capture image. Further, the identification information of the tablet registered as the correct image is not limited to the image of a partial region including the identification information in the capture image, but may be the image of the entire region of the capture image. Further, the control part 40 used at the time of executing the information registration process is an example of a registration processing part.

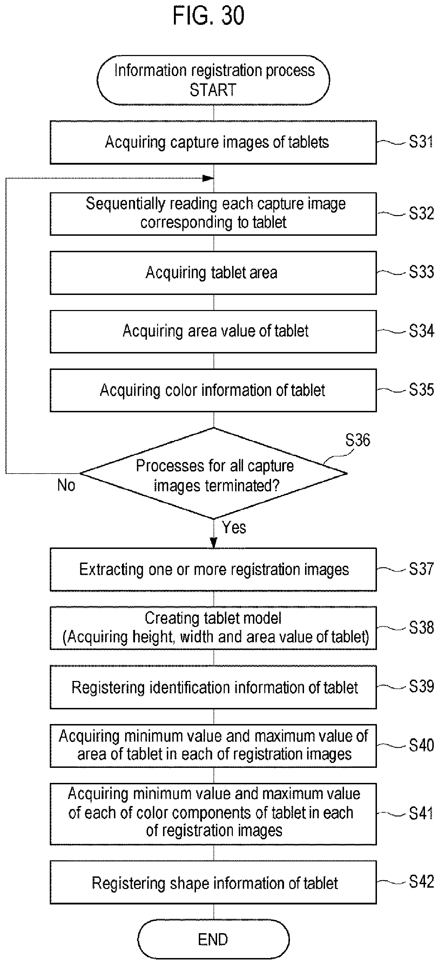

In this regard, FIG. 30 is a flowchart showing an example of the procedures of the information registration process executed by the control part 40. For example, the control part 40 can execute the information registration process as a portion of the initial setting process performed in the tablet packing device 4. Further, it is conceivable that, in case where the information of the tablet included in the prescription data to be subject to the packing process is not registered in the medicine master, or in case where the inspection result obtained in the automatic inspection process is an error, the control part 40 executes the information registration process automatically or in response to a user's execution request.
<Step S31> First, in step S31, the control part 40 executes a process of acquiring a plurality of capture images obtained by capturing the outer circumference of the tablet in different directions. Specifically, the control part 40 dispenses the tablets from the tablet cassette 41 or the manual distribution unit 42 in which the tablets to be registered are received. Then, similar to the execution of the packing process, the control part 40 captures images of the state where the tablet is rotated in the rotary unit 44, at a plurality of times by means of the camera 462 and the camera 463 of the image capturing part 46. In this regard, the control part 40 used at the time of executing such an image capturing process is an example of an image capturing processing part.
   More specifically, before capturing the image of the tablet, the control part 40 causes the manipulation display part 48 to display a selection screen for selecting whether the identification information of the tablet is formed by engraving or printing. Next, in response to a user's selection manipulation on the manipulation display part 48, the control part 40 specifies whether the identification information of the tablet is an engraving or print. In this regard, the control part 40 used at the time of executing the specifying process is an example of a specification processing part. Further, in case where the tablet to be registered is designated by a user's manipulation from the medicine database and a method of forming the identification information of the tablet is registered in the medicine database, the control part 40 can specify whether the identification information of the tablet is engraved or printed, based on the medicine database.
   Then, according to the method of forming the identification information, the control part 40 selects one of a first image capturing unit including the camera 462 and the illumination device 468 and a second image capturing unit including the camera 463 and the illumination device 469, and captures an image of the tablet. Specifically, when it is specified that the identification information of the tablet is an engraving, the image capturing of the tablet is executed using the first image capturing unit suitable for the engraving. When it is specified that the identification information of the tablet is a print, the image capturing of the tablet is executed using the second image capturing unit suitable for the print. As described above, when registering the correct image as the collation source data, the control part 40 selects the first image capturing unit or the second image capturing unit suitable for the method of forming the identification information of the tablet (engraving or printing), and captures an image of the tablet. Accordingly, a correct image to be registered in the medicine master as the collation source data of the tablet is acquired from the capture image in which the identification information of the tablet is clearly captured. Thus, in the image inspection process, the accuracy of the matching process performed by means of the correct image is enhanced.
   Further, it is conceivable that, in case where the packing process has already been executed and a plurality of the capture images are acquired with respect to the tablet in the automatic inspection process, the control part 40 reads a plurality of the capture images corresponding to the tablet from the storage part 49 as capture images of the tablet to be registered. That is to say, the control part 40 can not only execute the information registration process before the execution of the packing process, but also register the information of the tablet in the medicine master as a serial process at the time of executing the packing process. Further, in this case, it is conceivable that the control part 40 causes both the cameras 462 and 463 to capture images of the tablet in the packing process and reads, as the capture image of the tablet to be registered, the capture images captured by the camera 462 or 463 suitable for the engraving or the printing selected by a user's manipulation as described above.
   Further, when the tablet capture image is acquired at step S31, the tablet may be rotated in the tablet rotation part 441 of the rotary unit 44 in such a state that a side surface of the tablet faces toward the camera 462 or 463 (hereinafter referred to as "vertical rotation state"). In this case, the image the front or back surface of the tablet on which the identification information of the tablet exists is not captured by the camera 462 or 463. Therefore, the control part 40 determines whether the tablet is in the vertical rotation state. When the tablet is in the vertical rotation state, the control part 40 executes a process of changing an orientation of the tablet by vibrating the tablet. Further, not only in the information registration process but also during the execution of the packing process, the control part 40 executes the same process when capturing the image of the tablet by means of the camera 462 or 463.
   Specifically, when the image capturing of the tablet is executed at step S31, it is determined whether the tablet is being rotated in the vertical rotation state, based on the capture image acquired by the camera 462 or 463. In this regard, the control part 40 used at the time of executing such a process is an example of a tablet decision processing part. For example, the control part 40 determines whether aspect ratios of the tablet in a predetermined number of capture images or more among a plurality of the capture images are equal to or greater than a predetermined ratio. Specifically, in case where the predetermined number of the images is five and the predetermined ratio is 1.2, the control part 40 determines that the tablet is being rotated in the vertical rotation state, if values of length/width are equal to or greater than 1.2 in five or more capture images. Further, in case where the camera 462 or 463 is disposed in a position where the camera captures tablet rotation part 441 in a direction perpendicular to an axial direction of the rotating roller 100, the width direction of the tablet is parallel to the axial direction of the rotating roller 100, and the length direction of the tablet is a direction perpendicular to both an opposing direction of the rotating roller 100 and the camera 462 or 463 and the axial direction of the rotating roller s100.
   Then, when it is determined that the tablet is being rotated in the vertical rotation state, the control part 40 executes the process of changing the orientation of the tablet by vibrating the tablet placed on the tablet rotation part 441. In this regard, the control part 40 used at the time of executing such a process is an example of a vibration application processing part. Specifically, it is conceivable that the rotary unit 44 or the tablet rotation part 441 is provided with a vibration application part configured to apply vibration to the tablet by applying vibration to the rotary unit 44 or the tablet rotation part 441, and that the control part 40 drives the vibration application part such that the tablet falls down. Further, for example, the vibration application part is configured to apply vibration to the tablet rotation part 441 in the axial direction of the rotating roller 100. Further, it is also conceivable that the control part 40 executes a shaking process of reciprocating the rotary unit 44 by a predetermined distance by means of the unit rotation part 442 of the rotary unit 44. As a result, the tablet falls down in the tablet rotation part 441, whereby the orientation of the tablet is changed and the front and back surfaces of the tablet are captured by the camera 462 or 463.
   In this manner, in step S31, the operation of changing the orientation of the tablet using the vibration application part may be performed. The control part 40 does not cause the rotation part 442 to execute the rotation operation of the respective tablet rotation parts 441 for subsequent steps until the capture images required for acquiring the capture images of the front surface and the back surface of the tablet are obtained. Then, when it is not determined that the tablet is being rotated in the vertical rotation state based on the capture images captured by the camera 462 or 463, and when a pre-set number of capture images or more are acquired, the control part 40 terminates the image capturing, and causes the rotation part 442 to execute the rotation operation of the respective tablet rotation parts 441 to the subsequent steps. Further, it is conceivable that, when it is determined that the tablet is being rotated in the vertical rotation state based on the capture images captured by the camera 462 or 463, the control part 40 notifies an error and interrupts the process.
<Step S32> Each time when step S32 is executed, the control part 40 sequentially selects capture images subject to being processed in steps S33 to S35 among a plurality of the capture images corresponding to the tablet. Then, in steps S33 to S35, various processes are executed with respect to the capture images selected in step S32.
<Step S33> In step S33, the control part 40 specifies a tablet region, in which the tablet exists, from the capture image. For example, in case where a color of the rotating roller 100 provided in the tablet rotation part 441 is black, a background region of the tablet in the capture image is black. In this case, the control part 40 can acquire a region, from which a black region is excluded in the capture image, as the tablet region. Specifically, the control part 40 sets the black color of the rotating roller 100 in the capture image as a reference color (threshold value) and acquires a region having a color thinner than the reference color as the tablet region. For example, the reference color is represented by the respective component values of R (red), G (green) and B (blue).
<Step S34> In step S34, the control part 40 acquires an area value of the tablet region specified from the capture image. Further, when a plurality of the tablet regions are specified in step S33, the control part 40 selects, as the tablet region corresponding to the tablet, a circumscribed region of the tablet region having the largest area value among the tablet regions.
<Step S35> In step S35, the control part 40 acquires an index value such as an average value or a deviation value of a gray value in the tablet region. The gray value is a value available when the color of each pixel included in a gray scale image is expressed by multiple gradations such as 256 gradations or 65536 gradations from white to black. For example, the gray value "0" is white and the gray value "255" is black. Alternatively, the gray value "255" may be white and the gray value "0" may be black.
   Specifically, when the capture image is a color image, the control part 40 converts the capture image into a gray scale image and acquires an index value of the gray value of the tablet region of the converted capture image. Further, the index value is used for extracting an identification information such as printed characters or engravings in the tablet region.
   For example, it is conceivable that a proportion of a region, which is occupied by the identification information on the front surface or the back surface of the tablet, is small in the tablet region. Therefore, for example, when the gray value "0" is white and the gray value "255" is black, and when the color of the tablet is closer to black than the identification information, the average value of the gray values is greater than the gradation value of the identification information. Therefore, when the average value of the gray values is equal to or greater than a predetermined threshold value, the control part 40 can extract, as the identification information, a region whose gray value is smaller than the average value in the tablet region.
   Further, for example, when the gray value "0" is white and the gray value "255" is black, and when the color of the tablet is closer to white than the identification information, the average value of the gray values is smaller than the gradation value of the identification information. Therefore, when the average value of the gray values is smaller than a predetermined threshold value, the control part 40 can extract, as the identification information, a region whose gray value is larger than the average value in the tablet region.
<Step S36> In step S36, the control part 40 determines whether the processes in steps S33 to S35 have been completed with respect to all the capture images corresponding to the tablet. If it is determined that the processes have been completed (S36: Yes), the process proceeds to step S37. If it is determined that the processes have not been completed (S36: No), the process proceeds to step S32.
<Step S37> In step S37, the control part 40 specifies, as a registration image, one or a plurality of the capture images including the identification information of the tablet among a plurality of the capture images corresponding to the tablet. Further, when the tablet is a flat tablet, in step S37, the registration image is specified with respect to each of the front surface and the back surface of the tablet. Further, it is conceivable that, among a plurality of the capture images which are specified as the capture images f4 or f8 obtained by capturing the front surface and the back surface of the tablet from front side as shown in FIG. 24A and as described above, the control part 40 determines odd-numbered capture images to be an image of the front surface and determines even-numbered capture images to be an image of the back surface. Then, the processes of steps S38 to S39 to be described below are respectively executed with respect to one or a plurality of the registration images corresponding to the front surface of the tablet and one or a plurality of the registration images corresponding to the back surface of the tablet.
   For example, the control part 40 acquires an edge amount included in the tablet region of each of the capture images and specifies a reference capture image having the largest edge amount among the capture images as the registration image. Further, the edge amount is obtained by counting the number of edges in which a difference between the gradation values of adjacent pixels is equal to or greater than a pre-set threshold value. Thereafter, in case where a capture image, in which a ratio of the edge amount of the capture image to the edge amount of the reference capture image is equal to or greater than a pre-set threshold value (for example, 80% or 90%, etc.), exists in a plurality of the capture images, the control part 40 specifies such a capture image as the registration image.
   Further, the control part 40 may register the reference capture image as the correct image of the tablet in the medicine master. Further, the reference capture image registered as the correct image is transmitted to the server 2 by the control part 40 and is also registered in the medicine master stored in the storage part 22 of the server 2. Thus, in the server 2, the reference capture image can be displayed as the correct image in the inspection assistance process.
   Further, in other embodiment, it is conceivable that the control part 40 registers the capture image selected by a user's manipulation among the plurality of capture images corresponding to the tablet as the correct image in the medicine master. For example, in step S37, the control part 40 causes the manipulation display part 48 to display a plurality of the capture images corresponding to the tablets, receives the selection of the capture image performed by a user's manipulation using the manipulation display part 48, and registers the capture image in the medicine master.
   Further, it is conceivable that, when the correct image is registered in the medicine master in step S37, the control part 40 registers a correct orientation of the correct image designated by a user's manipulation in the medicine master. Specifically, in step S37, the control part 40 causes the manipulation display part 48 to display the capture image to be registered as the correct image, and receives the designation of an upward position (the correct orientation of the identification information) in the capture image by a user's manipulation using the manipulation display part 48. Then, the control part 40 rotates the capture image such that the upward position designated by the user's manipulation is a top side, and registers the rotated capture image as the correct image in the medicine master. That is to say, a correct position is indicated, as the upward position in the correct image, by the orientation of the correct image which is the collation source data. Thus, the correct image, which is rotated such that the upward position in the capture image is a top side, is registered as the collation source data in the medicine master. Therefore, in the image inspection process, the control part 40 can decide the suitability of the identification information by collating the correct image and the capture image in the state where the orientation of the identification information included in the correct image is correct. Further, it is also conceivable that the control part 40 registers the capture image as the correct image without rotating the capture image, and registers information such as coordinates indicating the upward position as a portion of the collation source data in association with the correct image. Even when the information indicative of the upward position in the correct image is registered in the medicine master as a portion of the collation source data as described above, in the image inspection process, the control part 40 can decide the suitability of the identification information by collating the correct image with the capture image in the state where the orientation of the identification information included in the correct image is correct.
   Further, it is conceivable that an initial correct image of the tablet, in which the identification information of the tablet is captured in the correct orientation, is stored in advance in the medicine database. In this case, it is also conceivable that the control part 40 determines the upward position of the correct image (the correct orientation of the identification information) based on the initial correct image stored in the medicine database instead of the user's manipulation. For example, the control part 40 executes a matching process of collating the correct image in each rotation state with the initial correct image while gradually rotating the correct image. Then, according to the collation result of the correct image and the initial correct image, the control part 40 determines that the state where the difference between the correct image and the initial correct image is smallest is the correct state of the correct image, and registers the correct image of such a state in the medicine master. Further, it is also conceivable that, according to the collation result of the correct image and the initial correct image, the control part 40 registers the capture image as the correct image without rotating the capture image, and registers the information such as coordinates indicating the upward position as a portion of the collation source data in association with the correct image. Further, the control part 40 can register the information registered in the medicine database as initial information of the medicine master. Regarding tablets which are not subject to registration in the information registration process, it is also conceivable that the initial correct image of the medicine database is registered as the correct image in the medicine master.
<Step S38> In step S38, the control part 40 specifies a shape of the tablet based on the registration image and creates data of a tablet model corresponding to the shape. The data of the tablet model includes information used for specifying the shape of the tablet, such as a height, a width and an area value of the tablet. Further, the data of the tablet model further includes information such as the position and range of the identification information included in the tablet.
   In particular, the control part 40 individually registers the information on the front and back surfaces of the tablet in the tablet model. Specifically, the control part 40 creates a tablet model corresponding to the front surface of the tablet based on one or a plurality of registration images corresponding to the front surface extracted in step S37, and creates a tablet model corresponding to the back surface of the tablet based on one or a plurality of the registration images corresponding to the back surface extracted in step S37. Further, the data of the tablet model is used for, for example, collating the suitability of the shape or size of the tablet in the automatic inspection process or specifying an existence region of the tablet identification information in the capture image in the automatic inspection process.
   Further, it is conceivable that, when a plurality of the registration images are specified in step S37, the control part 40 acquires information such as the position and range of the identification information in the tablet based on the registration images. For example, a value, which is obtained by averaging the values indicating the position and range of the identification information specified based on each of the registration images, is created as the tablet model. Further, in view of the pre-set weighted value corresponding to the ratio of the edge amount of the registration image to the edge amount of the reference capture image, the value indicating the position and range of the identification information specified based on each of the registration images may be created as the tablet model. Thus, the control part 40 can create the tablet model with high accuracy even when a missing or the like occurs in the identification information of a portion of the registration images.
<Step S39> In step S39, the control part 40 acquires the identification information of the tablet from the reference capture image or the registration image, and registers the identification information of the tablet in the medicine master in association the medicine identification information such as a medicine name or a medicine code indicating the type of the tablet. Thus, the identification information of the tablet acquired in the information registration process is registered in the medicine master as the collation source data of the identification information of the tablet in the automatic inspection process. For example, the control part 40 acquires the identification information on the front surface of the tablet based on one or a plurality of the registration images corresponding to the front surface, and acquires the identification information on the back surface of the tablet based on one or a plurality of the registration images corresponding to the back surface.
<Step S40> In step S40, the control part 40 acquires a minimum value and a maximum value of the area value of the tablet from one or a plurality of the registration images. For example, the minimum value and the maximum value of the area value of the tablet are used for obtaining the tablet region from the capture image in the automatic inspection process, or are used for calculating a circumferential length of the tablet. Further, the minimum value and the maximum value of the area value of the tablet may be acquired from a plurality of the capture images rather than the registration images.
<Step S41> In step S41, the control part 40 acquires a minimum value and a maximum value of each color component of the tablet from one or a plurality of registration images. For example, the control part 40 acquires a minimum value and a maximum value with respect to each of the color components of RGB. Further, the minimum value and the maximum value of each of the color components of the tablet may be acquired from a plurality of the capture images rather than the registration images.
<Step S42> In step S42, the control part 40 registers the shape information of the tablet, which is subject to being processed in the information registration process, in the medicine master in association with the medicine identification information such as a medicine name or a medicine code indicating the type of the tablet. Specifically, the shape information of the tablet includes the following: a height, a width and an area value of the tablet specified in step S38; the minimum value and the maximum value of the area value of the tablet acquired in step S39; and the minimum value and the maximum value of each color component of the tablet acquired in step S40.

In the tablet packing device 4, when the shape information of the tablet is registered in the medicine master as described above, it is conceivable that, the control part 40 determines, in the automatic inspection process, the suitability of the tablet based on the capture image of the tablet and the shape information of the tablet. More specifically, in the automatic inspection process, the control part 40 collates the shape of the tablet, which is included in the capture image, with the shape of the tablet, which is included in the tablet model corresponding to the tablet included in the prescription data. Then, if the collation result of the shape of the tablet based on the tablet model is coincidence, the control part 40 subsequently collates the identification information of the tablet included in the capture image with the identification information of the tablet registered in the information registration process.

Further, when the collation result of the shape of the tablet based on the tablet model is non-coincidence, the control part 40 does not determine the suitability of the tablet based on the identification information included in the capture image, and determines that the inspection result of the automatic inspection process is an error. That is to say, in the automatic inspection process, only when the collation result of the shape of the tablet is proper, the decision of the suitability of the tablet is executed based on the identification information of the tablet. Accordingly, in the tablet packing device 4, the process requiring a heavy load, such as the process of acquiring the identification information from the capture image, the process of collating the tablet based on the identification information, or the like, can be omitted as far as possible.

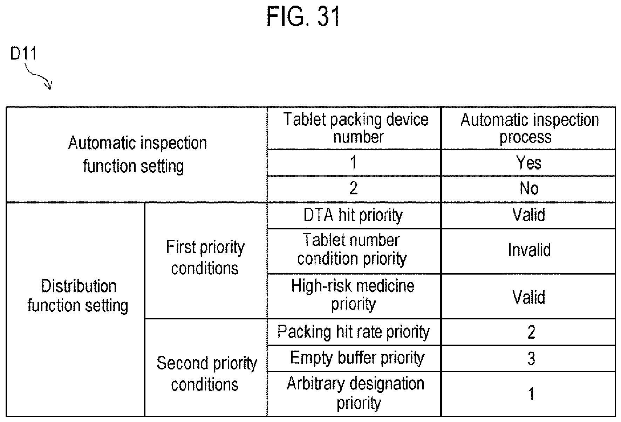

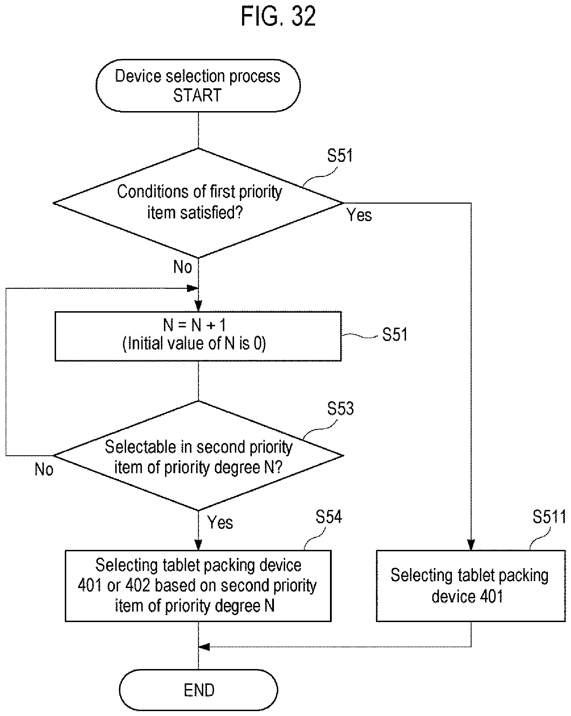

[Device Selection Function] As described above, a plurality of the tablet packing devices 4 may be connected to the inspection assistance system 1. Therefore, the control part 21 has a device selection function of selecting the tablet packing device 4, which executes the packing process based on the prescription data, based on preset conditions. Hereinafter, descriptions are made as to the device selection function with reference to FIGS. 31 and 32.

In this regard, a plurality of the tablet packing devices 4, which is connectable to the inspection assistance system 1, may include a tablet packing device 4 which is capable of executing the packing process and the image inspection process (hereinafter referred to as a first tablet packing device 401), and a tablet packing device 4 which is capable of executing the packing process but incapable of executing the image inspection process (hereinafter referred to as a second tablet packing device 402). For example, it is conceivable that the second tablet packing device 402 does not include the image capturing part 46. Further, in case where the first tablet packing device 401 is capable of executing the image inspection process and capable of switching validity and invalidity of the image inspection process, the tablet packing device 4 in which the execution of the image inspection process is set invalid is recognized as the second tablet packing device 402 by the server 2. Further, in the following description, where collectively referring to the first tablet packing device 401 and the second tablet packing device 402, they are simply referred to as "tablet packing device 4".

Selection setting information D11 used for selecting the tablet packing device 4 is stored in the storage part 22 of the server 2. In this regard, FIG. 31 shows an example of the selection setting information D11. As shown in FIG. 31, the selection setting information D11 includes an automatic inspection function setting item and a distribution function setting item. Further, the selection setting information D11 is inputted from the first tablet packing device 401 to the control part 21 of the server 2, or is read out from the first tablet packing device 401 by the control part 21 of the server 2.

In the automatic inspection function setting item, the availability of the automatic inspection process is stored in association with each tablet packing device number which is identification information for identifying the tablet packing device 4. Further, when the validity and invalidity of the automatic inspection process can be switched as described above, the set state of the validity or invalidity of the automatic inspection process is stored.

The distribution function setting item includes first priority conditions and second priority conditions, both of which are set in advance. The first priority conditions are conditions for determining whether to execute the automatic inspection process. The second priority conditions are conditions for selecting the tablet packing device 4 regardless of the necessity of the automatic inspection process. In the inspection assistance system 1, in the initial setting process or the like, the control part 21 can set each of the first priority conditions to either validity or invalidity in response to a user's manipulation. Further, the control part 21 can set priority degrees in the second priority conditions with respect to each of the second priority conditions in response to a user's manipulation.

The first priority conditions include respective items of "DTA hit priority", "tablet number condition priority" and "high risk medicine priority". The "DTA hit priority" is a condition of selecting the first tablet packing device 401 when the manual distribution unit 42 is used in the packing process. The "tablet number condition priority" is a condition of selecting the first tablet packing device 401 when the number of tablets per one pack is equal to or greater than a predetermined threshold value in the packing process. The "high risk medicine priority" is a condition of selecting the first tablet packing device 401 when a medicine subject to being packed in the packing process is a predetermined high-risk medicine. In the example shown in FIG. 31, the "DTA hit priority" and the "high risk medicine priority" are set valid and the "tablet number condition priority" is set invalid.

The second priority conditions include respective items of "packing hit rate priority", "empty buffer priority" and "arbitrary designation priority". The "packing hit rate priority" is a condition of selecting the tablet packing device 4 which has the largest number of types of medicines received in the tablet cassettes 41 among medicines subject to being packed in the packing process. The "empty buffer priority" is a condition of selecting the tablet packing device 4 which has the smallest amount of prescription data standing by as a target of the packing process. The "arbitrary designation priority" is a condition of selecting the tablet packing device 4 which is arbitrarily designated by a user. In the example shown in FIG. 31, the priority degree of the "arbitrary designation priority" is set to "1", the priority degree of the "packing hit rate priority" is set to "2", and the priority degree of the "empty buffer priority" is set to "3". Further, the second priority conditions are not limited to the ones to which the aforementioned priority degree is set, but may be selected alternatively.

Then, the control part 21 executes a device selection process for selecting the tablet packing device 4, which executes the packing process corresponding to each of the prescription data, based on the selection setting information D11 and at least one of the contents of the prescription data and the contents of the packing process. Further, the control part 21 used at the time of executing the device selection process is an example of a selection processing part. In this regard, FIG. 32 is a flowchart showing an example of the device selection process. The control part 21 executes the device selection process with respect to the prescription data, which needs the packing process using the tablet packing device 4, among the aforementioned prescription data.
<Step S51> First, in step S51, the control part 21 determines whether at least one of the contents of the prescription data and the contents of the packing process satisfies any one of the first priority conditions. Then, when it is determined that any one of the first priority conditions is satisfied, the control part 21 causes the process to proceed to step S511. When it is determined that all of the first priority conditions are not satisfied, the control part 21 causes the process to proceed to step S52.
   Herein, the "DTA hit priority" and the "high risk medicine priority" are set to be valid among the first priority conditions. Thus, in case where the manual distribution unit 42 is used in the packing process on the prescription data or in case when a medicine subject to be being packed is a high-risk medicine, it is determined that such a case corresponds to the first priority conditions.
   <Step S511> In step S511, the control part 21 selects the first tablet packing device 401 capable of executing the image inspection process as the tablet packing device 4 which executes the packing process based on the prescription data, and transmits the prescription data to the selected tablet packing device 4. That is to say, when at least one of the first priority conditions is satisfied, the first tablet packing device 401 is selected. Accordingly, when it is preferable to execute the image inspection process, the packing process can be executed using the first tablet packing device 401.
<Step S52> If it is determined in step S51 that the first priority conditions are not satisfied, the tablet packing device 4 is selected according to the second priority conditions in subsequent steps S52 to S53. Specifically, in step S52, the control part 21 adds 1 to a value of a counter N used for specifying a value of the priority degree of the second priority conditions. An initial value of the counter N is 0, and is reset to 0 at the start or end of the device selection process.
<Step S53> In step S53, the control part 21 determines whether a pre-set value of the priority degree among the second priority conditions can select any one of the tablet packing devices 4 based on the second priority conditions of the counter N. For example, in case where there is only one tablet packing device 4 that meets the second priority conditions, it is determined that the tablet packing device 4 can be selected. If it is determined that the tablet packing device 4 can be selected (S53: Yes), the control part 21 causes the process to proceed to step S54. If it is determined that the tablet packing device 4 cannot be selected (S53: No), the control part 21 returns the process to step S52.
   Further, if it is determined that the tablet packing device 4 cannot be selected even when the value of the counter N is equal to the number of the second priority conditions ("3" in this example), it is conceivable that a pre-set specific tablet packing device 4 is selected among the tablet packing devices 4. Further, in case where a plurality of tablet packing devices 4 is narrowed down as a selection target from the plurality of tablet packing devices 4 based on the second priority conditions, and in case where it is determined that the tablet packing device 4 cannot be selected even when the value of the counter N is equal to the number of the second priority conditions, it is conceivable that the tablet packing device 4 having the maximum or minimum tablet packing device number is selected among the tablet packing devices 4.
<Step S54> In step S54, the control part 21 selects the tablet packing device 4 for executing the packing process, based on the second priority conditions in which the priority degree is the value of the counter N, and transmits the prescription data to the selected tablet packing device 4. As a result, the packing process is executed by the tablet packing device 4 selected according to the priority degree of the second priority conditions.

Further, in step S54, it is conceivable that the first tablet packing device 401 is not selected. That is to say, in step S54, it is conceivable that the control part 21 selects one of the second tablet packing devices 402 based on the second priority conditions in which the priority degree is the value of the counter N. As a result, when all the first priority conditions are not satisfied, the second tablet packing device 402 which does not execute the image inspection process is used. Thus, the packing process based on the prescription data is rapidly performed.

Further, in the image inspection process, based on the capture image captured by the camera 462 or the camera 463 before the tablets are packed into the medicine pack 451, it is determined whether the collation result of the respective types of the tablets and the types of the tablets included in the prescription data is coincidence, whereby it is inspected whether the result of the packing process is proper. That is to say, in the image inspection process, by determining whether the tablets not yet packed into the medicine pack 451 are proper, the suitability of the medicine pack 451 obtained by the packing process is determined. On the other hand, the image inspection process is not limited thereto. For example, there may be provided an image capturing part which captures an image of the medicine pack 451 available after the tablets are packed into the medicine pack 45. Based on the capture image captured by such an image capturing part, it may be determined whether the collation result of the respective types of the tablets and the types of the tablets included in the prescription data is coincidence. Further, in the image inspection process, it may be determined, based on the capture images of the tablet, whether the collation result of the number of the respective tablets and the number of the tablets included in the prescription data is coincidence. That is to say, the image inspection process may be any process for determining whether a portion or all of the steps of the packing process have been properly performed, based on the capture images of the tablets or the medicine pack 451.

[Inspection Recording Function] Further, when the packing process is executed by the first tablet packing device 401, the automatic inspection process is executed by the first tablet packing device 401. Therefore, the burden of the inspection task on the packing process conducted by a pharmacist is reduced. However, even when the packing process is executed by selecting one of the first tablet packing device 401 and the second tablet packing device 402 by the device selection function, a pharmacist cannot recognize, by seeing the medicine pack 451, whether the packing process has been executed by the first tablet packing device 401.

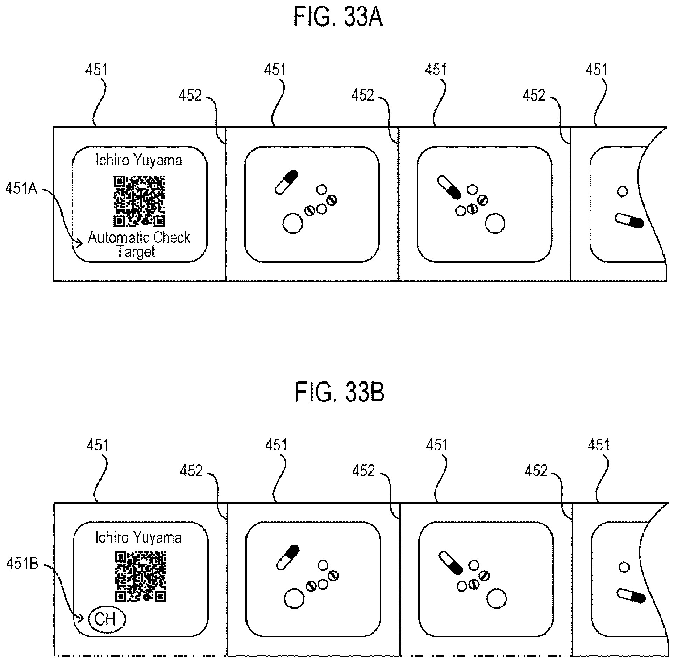

Thus, it is conceivable that the tablet packing device 4 has an inspection recording function of recording where the image inspection process has been executed as the automatic inspection process, on the first or last medicine pack 451 among a series of the medicine packs 451 dispensed by the packing process. For example, as shown in FIG. 2, it is conceivable that the first tablet packing device 401 includes, in addition to the printing unit 453, a stamp unit 454 capable of recording the execution or non-execution of the image inspection process on the medicine pack 451 after the packing process. The stamp unit 454 can record an inspection execution information such as a character or an image indicating that the image inspection process has been executed, on the medicine pack 451 by means of a stamp. For example, as shown in FIGS. 33A and 33B, a character string "automatic check target" indicating that the medicine pack is a target to be checked by the image inspection process, or a character "CH", which is an acronym of CHECK and indicates that the medicine pack has been checked by the image inspection process, is used as the inspection execution information.

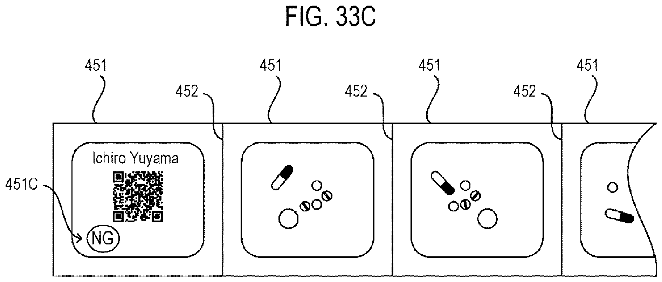

Further, it is conceivable that the stamp unit 454 includes a stamp for recording "automatic check target" or "CH" as described above and a stamp for recording "NG" as the inspection execution information on the medicine pack 45 as shown in FIG. 33C. Then, when the result of the image inspection process is OK, "automatic check target" or "CH" is recorded on the medicine pack 451 as described above. When the result of the image inspection process is an error, "NG" is recorded as the inspection execution information on the medicine pack 451. Further, the purport that the image inspection process has not been executed is indicated by not recording the inspection execution information such as "automatic check target", "CH" or "NG" on the medicine pack 451.

Further, it is conceivable that the control part 40 uses the printing unit 453 to print "automatic check target" or "CH" on the medicine pack 451 as information indicating whether the medicine pack is a target of the image inspection process. Further, it is also conceivable to employ a configuration in which the image inspection process on the tablets already packed into the medicine pack 451 is terminated at a timing when the inspection execution information can be recorded on the medicine pack 451 by the printing unit 453. In this case, it is conceivable that, when the result of the image inspection process is OK, the control part 40 uses the printing unit 453 to print "automatic check target" or "CH" as the inspection execution information on the medicine pack 451, and that, when the result of the image inspection process is an error, the control part 40 uses the printing unit 453 to print "NG" as the inspection execution information on the medicine pack 451.

[Second Embodiment] Subsequently, descriptions are made as to an inspection assistance system 1 according to a second embodiment of the present invention. The configurations not described herein are the same as those of the first embodiment. Hereinafter, descriptions are made as to an example of the procedure of the automatic inspection process executed by the tablet packing device 4 in the inspection assistance system 1 according to the present embodiment. Then, descriptions are made as to other display examples in the inspection assistance process executed by the server 2.

[Automatic Inspection Function] As described above, the tablet packing device 4 has an automatic inspection function of executing the automatic inspection process for deciding the suitability of the tablets contained in the medicine pack 451 packed by the packing process. Herein, a specific example of the automatic inspection process is described.

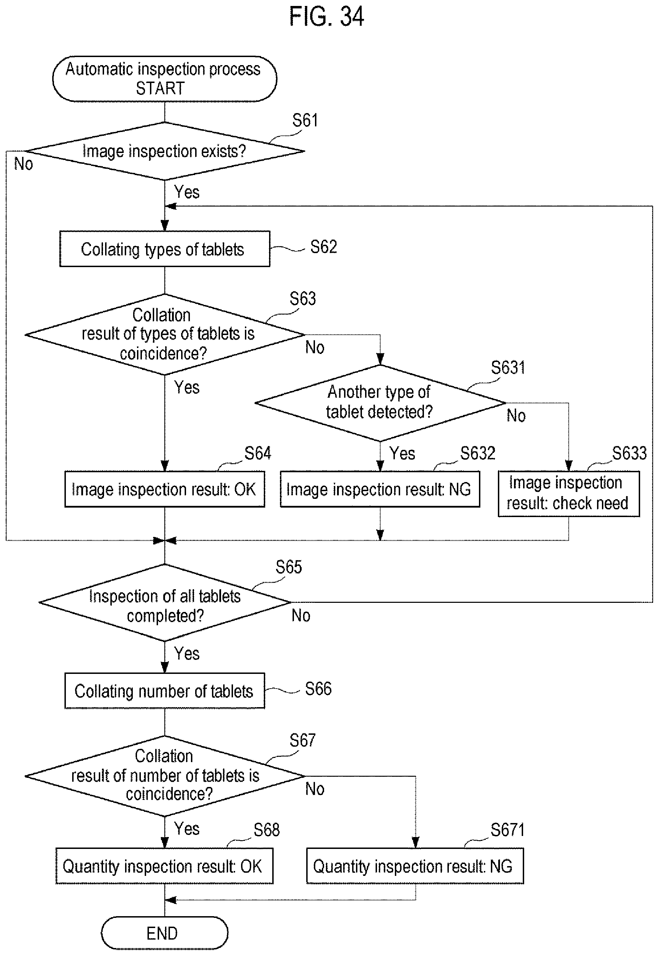

First, when the control part 40 executes the packing process based on the prescription data, images of the tablets are captured by the cameras 461 to 467 of the image capturing part 46. Further, when the packing process is executed, the control part 40 counts the number of the tablets detected by the passage detection sensor 475 of the passage detection part 47, with respect to each of the medicine packs 451. Further, the control part 40 used at the time of counting the number of tablets is an example of a tablet number detection part. Then, when the packing process is terminated, the control part 40 executes the automatic inspection process according to, for example, the processing procedures shown in FIG. 34, thereby deciding the suitability of the tablets received in each of the medicine packs 451 packed by the packing process. The automatic inspection process is executed with respect to each of the medicine packs 451. Further, the control part 40 may execute the automatic inspection process and the packing process in parallel.
<Step S61> First, in step S61, the control part 40 determines whether it is necessary to execute the image inspection process. If it is determined that it is necessary to execute the image inspection process (S61: Yes), the control part 21 causes the process to proceed to step S62. For example, the control part 40 decides whether to execute the image inspection process, according to the current inspection mode (one of the first inspection mode to the third inspection mode) switched by the inspection mode switching function. Further, in case where the image inspection process is always executed by the tablet packing device 4, step S61 is omitted.
<Step S62> Then, in steps S62 to S65, the control part 40 executes an image inspection process of collating the identification information of the tablet acquired from the capture image with respect to each tablet received in the medicine pack 451 with the identification information of the tablet included in the prescription data.
   More specifically, the control part 40 determines whether the shape of the tablet matches the shape of the tablet which is registered in the medicine master in association with the tablet included in the prescription data. Then, when the shapes of the tablets do not match, the control part 40 determines that the collation result is non-coincidence. On the other hand, when the shapes of the tablets match, the control part 40 acquires the identification information of the tablet from the capture image and executes an image inspection process of collating the acquired identification information with the identification information of the tablet which is registered in the medicine master in association with the tablet included in the prescription data. In this regard, the control part 40 used at the time of executing such a process is an example of an image inspection processing part. For example, in the image inspection process, the control part 40 extracts the image of the tablet including the identification information of the tablet from the capture image, and executes a process of matching the image of the tablet in the capture image and the image of the identification information of the tablet in the correct image. Further, the characters of identification information may be recognized from the capture image by a well-known character recognition process (OCR) and the collation may be performed using the characters of identification information. Further, as described above, when the tablet is a flat tablet having front and back surfaces, the control part 40 specifies capture images f4 and f8 corresponding to the front and back surfaces of the tablet as shown in FIG. 24 and acquires the identification information from the capture images f4 and f8. Further, when the tablet is a capsule tablet, the control part 40 acquires the identification information from the left and right capsule pieces of the capsule tablet.
<Step S63> In step S63, if the collation result in step S62 is coincidence (S63: Yes), the control part 40 causes the process to process to step S64. If the collation result is non-coincidence (S63: No), the control part 40 causes the process to proceed to step S631.
<Step S64> In step S64, the control part 40 causes the storage part 49 to record the purport that the inspection result of the tablet in the prescription data is proper. That is to say, when the shape and identification information of the tablet matches the shape and identification information of the tablet registered in the medicine master, the control part 40 determines that the inspection result of the image inspection process is proper.
   <Step S631> In step S631, the control part 40 determines whether another type of tablet, which is not included in the prescription data as types of the tablets, has been detected. For example, when the type of the tablet is not detected, that the identification information attached to the tablet may have a defect. Further, the collation by the image inspection process cannot be performed with respect to the tablet to which the identification information is not attached. Therefore, even when the identification information is not attached to the tablet, another type of tablet is not detected. On the other hand, if another type of tablet is detected, such a tablet is clearly erroneous. Thus, in step S631, if another type of tablet is detected (S631: Yes), the process proceeds to step S632. If another type of tablet is not detected (S631: No), the process proceeds to step S633. Further, regarding the tablet to which the identification information is not attached, it is determined whether the appearance such as the shape, size and color of the tablet registered in the medicine master is matched. It is conceivable that, if the appearance is matched, the process proceeds to step S632, and that, if the appearance is not matched, the process proceeds to step S633. Further, when the matching process is executed in step S62, it is conceivable that it is determined in step S631 whether a value of a coincidence degree (matching rate) obtained as a result of the matching process is less than a preset threshold value, and that, if the value of the coincidence degree is less than the threshold value, the process proceeds to step S632.
   <Step S632> Then, in step S632, the control part 40 causes the storage part 49 to record the purport that the inspection result of the image inspection process on the tablet is an error. That is to say, when the identification information of the tablet is clearly different from the identification information of the tablet registered in the medicine master, the control part 40 determines that the inspection result of the image inspection process is an error.
   <Step S633> On the other hand, in step S633, the control part 40 causes the storage part 49 to record, as the inspection result of the tablet, the purport that the inspection result of the image inspection process on the tablet is probably not proper and a visual check is needed. That is to say, if the identification information of the tablet is not clearly different from the identification information of the tablet registered in the medicine master, the control part 40 determines that the inspection result of the image inspection process is a check need. Further, in the image inspection process, it is conceivable that, even when the coincidence degree in the collation result of the identification information of the tablet is equal to or less than a predetermined value set in advance, the inspection result of the image inspection process is determined to be a check need.
<Step S65> Next, in step S65, if it is determined that the image inspection process has been terminated with respect to all the tablets of the medicine pack 451 (S65: Yes), the control part 40 causes the process to proceed to step S66. If the image inspection process has not been terminated (S65: No), the control part 21 returns the process to step S62.
<Step S66> In step S66, the control part 40 executes a count inspection process of determining whether the number of tablets detected by the passage detection sensor 475 in the packing process matches the number of tablets to be packed into the medicine pack 451 in the prescription data. Specifically, the passage detection sensor 475 *(see* FIG. 2) is disposed in a position which is at a downstream side of the image capturing position of the tablet by the cameras 462 and 463, and where the tablet can be detected before the tablet is received in the medicine pack 451. Accordingly, in the count inspection process, it is decided whether the number of the tablets, which are counted at least at the downstream side of the image capturing position of the tablet before the medicine pack 451 is sealed, is proper. In this regard, the control part 40 used at the time of executing such a count inspection process is an example of a count inspection processing part.
<Step S67> In step S67, if the collation result in step S66 is coincidence (S67: Yes), the control part 40 causes the process to proceed to step S68. If the collation result is non-coincidence (S67: No), the control part 40 causes the process to proceed to step S671.
<Step S68> In step S68, the control part 40 causes the storage part 49 to record the purport that the inspection result of the count inspection process on the tablet is proper, in association with the tablet which was a collation target in step S66.
   <Step S671> In step S671, the control part 40 causes the storage part 49 to record the purport that the inspection result of the count inspection process on the tablet is an error, in association with the tablet which was a collation target in step S66.

In this manner, when the execution of the image inspection process is set valid, both the image inspection process and the count inspection process are executed in the automatic inspection process. When the inspection results of the image inspection process and the count inspection process are proper, it is determined that the inspection result of the automatic inspection process is proper. If one of the inspection results of the image inspection process and the count inspection process is an error, it is determined that the inspection result of the automatic inspection process is an error.

Then, if the automatic inspection process is executed with respect to the tablets of all the medicine packs 451 packed by the packing process, the control part 40 transmits the results of the image inspection process to the server 2 together with the capture images. Further, the number of the tablets used in the count inspection process is also transmitted to the server 2 by the control part 40. As a result, in the server 2, it is possible to, when necessary, display the result of the image inspection process, the result of the count inspection process, the capture image and the like in the inspection assistance process executed by the control part 21.

[Display Example in Inspection Assistance Process] Specifically, in the inspection assistance process *(see* FIG. 7) executed by the control part 21, various display screens are displayed as described above. Herein, other examples of the various display screens displayed in the inspection assistance process are described.

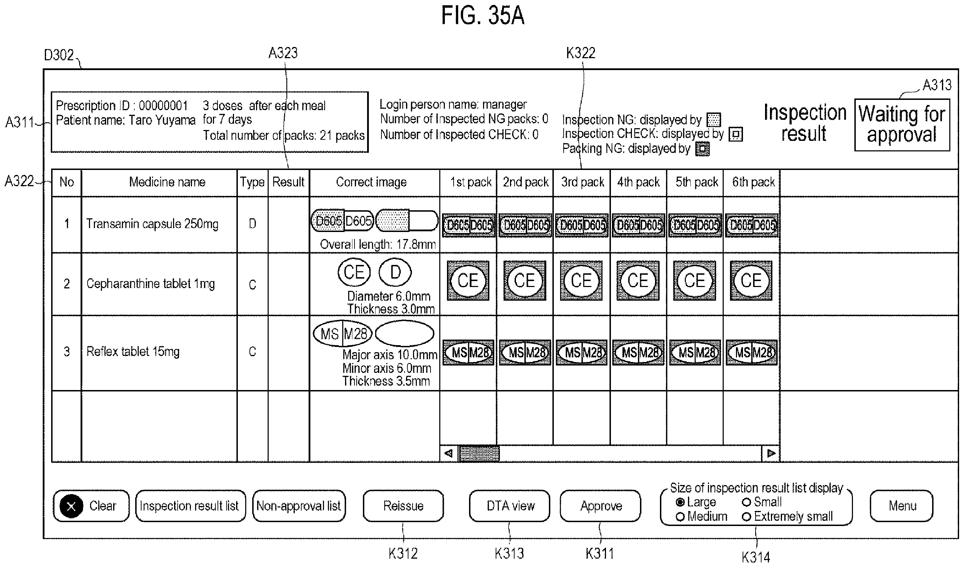

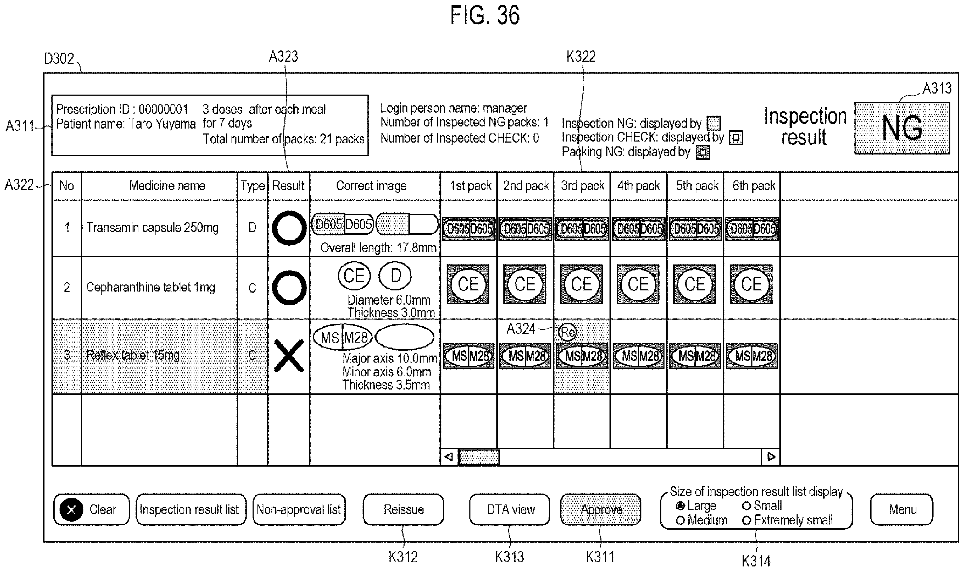

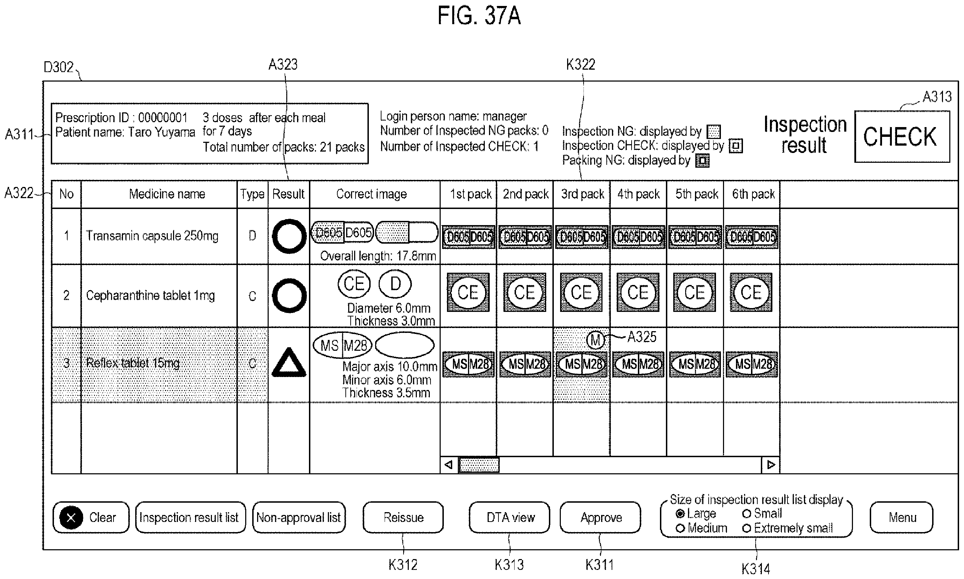

In this regard, FIGS. 35A, 36 and 37A shows an inspection detail screen D303 which is another example of the inspection detail screen D302 *(see* FIGS. 19 and 20). Specifically, FIG. 35A shows a display example wherein the inspection result obtained by the automatic inspection process is proper, FIG. 36 shows a display example wherein the inspection result obtained by the automatic inspection process is an error, and FIG. 37A shows a display example wherein the inspection result obtained by the automatic inspection process is a check need.

As shown in FIG. 35A, when the inspection result obtained by the automatic inspection process is proper, "Waiting for approval" is displayed as the inspection result of the automatic inspection process at the inspection result area A313 in the inspection detail screen D303. Further, in the inspection detail screen D303, the result display area A323, which indicates the result of the automatic inspection process on each tablet in the medicine preparation detail area A322, is blank. Further, an approval key K311, on which a manipulation is made to approve the displayed inspection result, is displayed in the inspection detail screen D303.

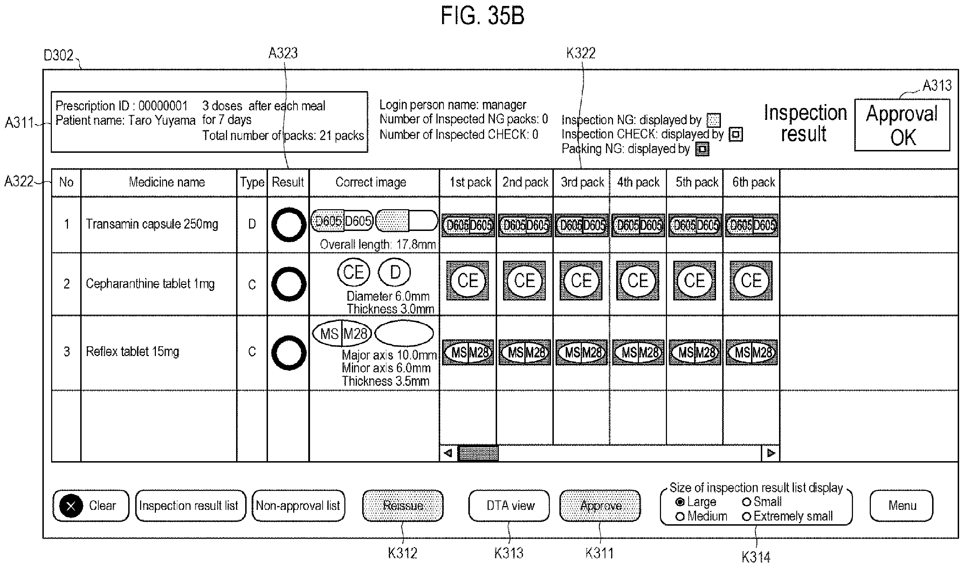

Then, when the approval key K311 is manipulated on the inspection detail screen D303, the control part 21 receives the approval of the inspection result of the automatic inspection process and stores the approval in association with the prescription data. Further, as shown in FIG. 35B, the control part 21 displays "Approval OK" as an inspection result of the automatic inspection process at the inspection result area A313 in the inspection detail screen D303. Further, as shown in FIG. 35B, the control part 21 displays "O", which indicates the suitability as an inspection result of the automatic inspection process, at the result display area A321 in the inspection detail screen D303. Further, as shown in FIG. 35B, when the inspection result obtained by the automatic inspection process is "Approval OK", it is displayed by gray-out or the like that the approval key K311 become non-manipulable.

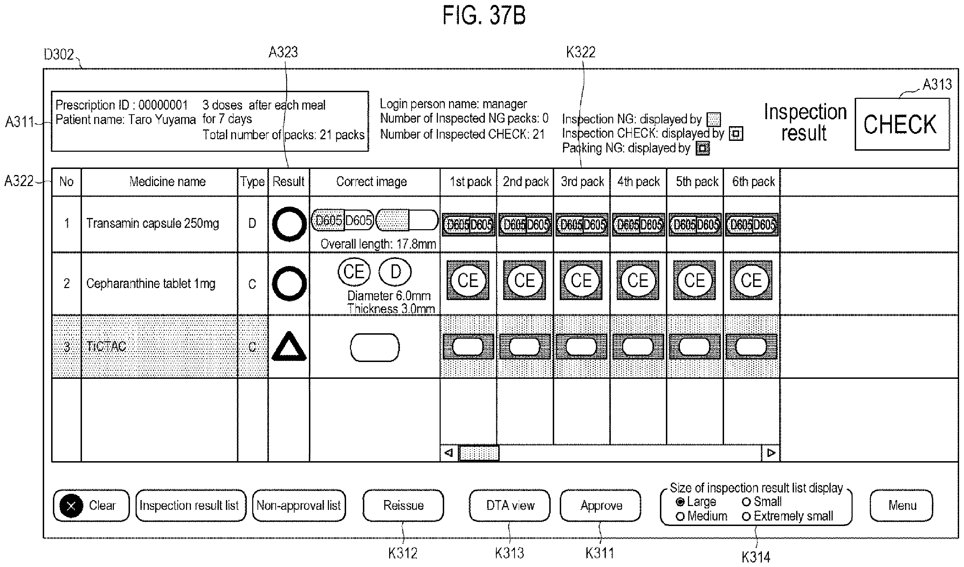

On the other hand, as shown in FIG. 36, when the inspection result obtained by the automatic inspection process is an error, "NG" indicating an error as an inspection result of the automatic inspection process is displayed at the inspection result area A313 in the inspection detail screen D303. Further, as shown in FIG. 37A, when the inspection result obtained by the automatic inspection process is a check need, "CHECK" indicating a check need as an inspection result of the automatic inspection process is displayed at the inspection result area A313 in the inspection detail screen D303. In particular, when the identification information of the tablet cannot be acquired in the image inspection process, i.e. when the character printing or engraving does not exist in the tablet like a tablet "TiCTAC" shown in FIG. 37B, the control part 21 displays the purport that all the medicine packs 451 receiving such tablets need to be checked. Further, when the inspection result of the automatic inspection process is a check need, it is possible to manipulate the approval key K311 as shown in FIGS. 37A and 37B. However, when the inspection result of the automatic inspection process is an error, the approval key K311 cannot be manipulated by a gray-out or the like as shown in FIG. 36,.

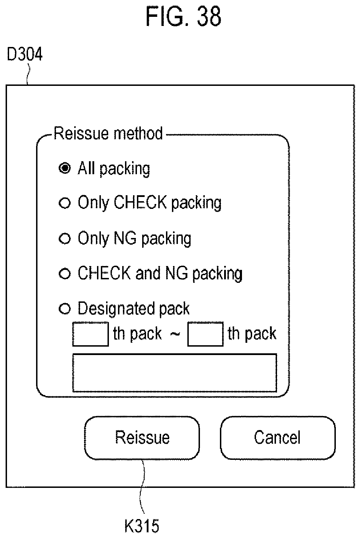

Further, manipulation keys K312 to K314 are displayed in the inspection detail screen D303. The manipulation key K312 is a manipulation key for re-executing the packing process with respect to one or a plurality of medicine packs 451. When the reissue key K312 is manipulated, the control part 21 displays a reissue manipulation screen D304 for setting a method of re-executing the packing process. In this regard, FIG. 38 shows an example of the reissue manipulation screen D304.

As shown in FIG. 38, on the reissue manipulation screen D304, it is possible to select "All packing", "Only CHECK packing", "Only NG packing", "CHECK and NG packing" and "Designated pack" as a target for re-execution of the packing process. If "All packing" is selected, all the medicine packs 451 become the target. If "Only CHECK packing" is selected, only the medicine pack 451 whose inspection result is a check need becomes the target. Further, if "Only NG packing" is selected, the medicine pack 451 whose inspection result is an error becomes the target. If "CHECK and NG packing" is selected, the medicine pack 451 whose inspection result is an error and the medicine pack 451 whose inspection result is a check need become the target. Further, in the case of "Designated pack", it is possible to arbitrarily designate the medicine pack 451 to be re-executed and it is possible to perform, for example, consecutive designation or individual designation. Further, as described below, when the medicine pack 451, which is the target for the re-execution of the packing process, has already been designated on another screen, the identification information such as a medicine pack number of the designated medicine pack 451 or the like is displayed at an input field of the designated package.

A reissue key K315 for starting the execution of reissue is displayed in the reissue manipulation screen D304. In response to the manipulation of the reissue key K315, the control part 21 transmits a control instruction for re-executing the packing process on the designated medicine pack 451 to the tablet packing device 4. Thus, the tablet packing device 4 re-executes the packing process with respect to the medicine pack 451 designated on the reissue manipulation screen D304 in response to the control instruction.

Further, the manipulation key K313 is a manipulation key for displaying the manual distribution check screen D5. Further, the manipulation key K313 can be manipulated when the manual distribution unit 42 is used in the packing process. When the manual distribution unit 42 is not used in the packing process, the manipulation key K313 becomes non-manipulable by a gray-out or the like. Then, if the manipulation key K313 is manipulated, the control part 21 displays the manual distribution check screen D5 *(see* FIG. 14) in which the manual distribution image is displayed.

Further, the manipulation key K314 is a manipulation key for changing a display size of the list display of the inspection result on the inspection detail screen D303. Then, when the display size is changed by the manipulation of the manipulation key K314, the control part 21 changes the number of the medicine packs 451 simultaneously displayed in the inspection detail screen D303. Four types of display sizes of "Large", "Medium", "Small" and "Extremely small" can be arbitrarily selected in the manipulation key K314. Thus, when referring to the result of the automatic inspection process, a user can select a display mode for the purpose of enlarging and checking the respective capture images or for the purpose of grasping all the results of the automatic inspection process.

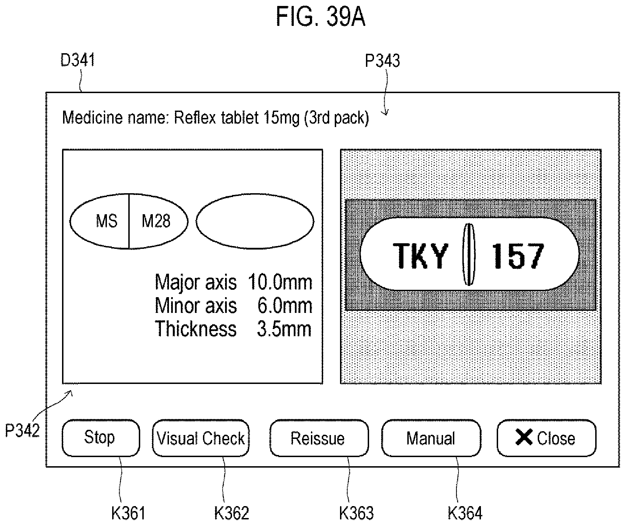

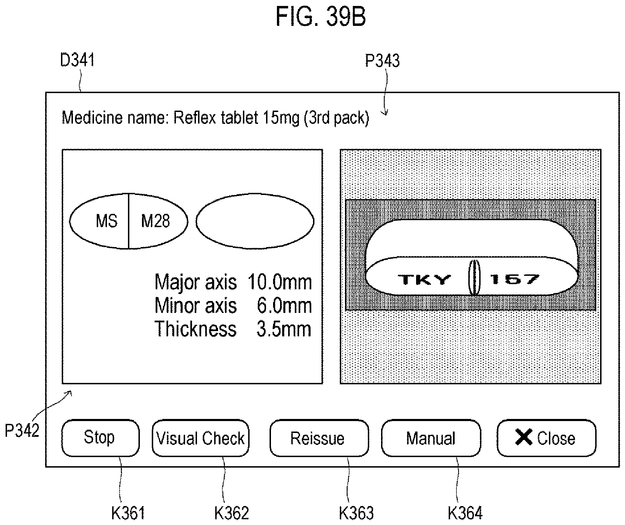

Further, as described above, when the capture image of a specific tablet in a specific medicine pack 451 is selected on the inspection detail screen D303, the control part 21 displays the enlarged screen D34 corresponding to the tablet. In this regard, FIGS. 39A and 39B show the enlarged screen D341 as another example of the enlarged screen D34. As shown in FIGS. 39A and 39B, the enlarged image P342 corresponding to the correct image and the enlarged image P343 of the capture image corresponding to the selected tablet are displayed side by side in the enlarged screen D 341. Further, the control part 21 may display the enlarged screen D34 with respect to only the capture image of the tablet, whose inspection result of the automatic inspection process is an error or a check need, among the capture images. However, the control part 21 may display the enlarged screen D34 with respect to the capture images of all the tablets.

Further, for example, as shown in FIGS. 39A and 39B, the control part 21 can display the enlarged image P343 displayed in the enlarged screen D341, by sequentially switching a plurality of the capture images corresponding to the tablets at predetermined intervals. More specifically, if the enlarged screen D341 is displayed, the control part 21 starts switching the enlarged image P343. Thus, a user can determine the suitability of the tablet by referring to a plurality of the capture images obtained by capturing the outer circumference of the tablet at different angles.

Manipulation keys K361 to K364 are displayed in the enlarged screen D341. When the manipulation key K361 is manipulated, the control part 21 temporarily stops the automatic switching of the enlarged image P343. When the manipulation key K361 is manipulated again, the control part 21 resumes the automatic switching of the enlarged image P343. Further, it is also conceivable that the control part 21 starts the automatic switching of the enlarged image P343 as the manipulation key K361 is manipulated after the enlarged screen D341 is displayed.

Further, when the manipulation key K362 is manipulated in the case where the inspection result of the automatic inspection process of the displayed tablet is a check need or an error, the control part 21 records, as the inspection result of the tablet, the purport that the tablet was determined to be proper by a visual check. Further, when the manipulation key K363 is manipulated, the control part 21 selects and records the medicine pack 451 receiving the displayed tablet as the reissue target. In this regard, it is conceivable that, as shown in FIG. 36, the tablet or the medicine pack 451 selected as the reissue target is displayed in the inspection detail screen D303 in association with the additional information A324 such as a character or a symbol indicating that the tablet or the medicine pack 451 is the reissue target.

Further, when the manipulation key K364 is manipulated, the control part 21 records the purport that a treatment such as manual replacement of the tablets received in the medicine pack 451 containing the displayed tablet or the like is executed. Even in this case, it is conceivable that, as shown in FIG. 37A, the tablet selected as a target to be subject to the manual treatment is displayed in the inspection detail screen D303 in association with the additional information A325 such as a character or a symbol indicating that the tablet is a target of the manual treatment.

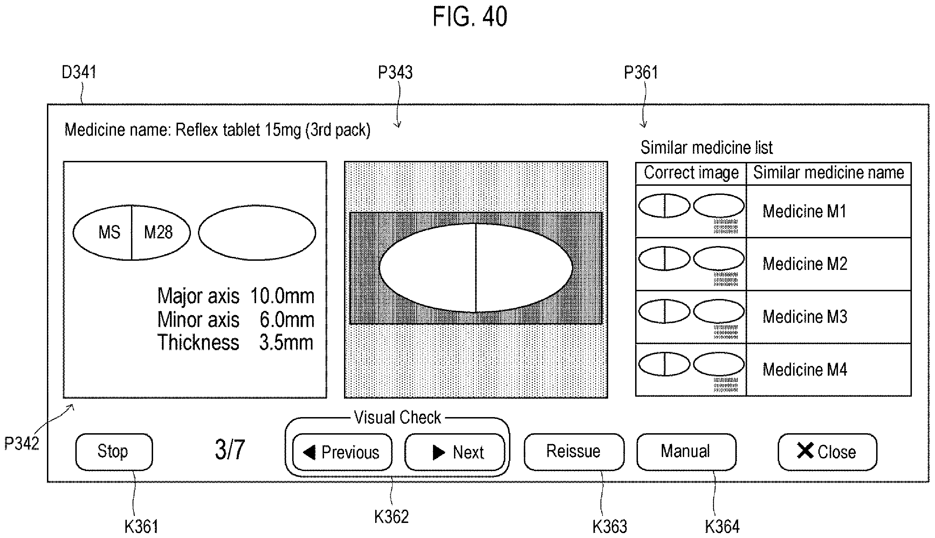

Further, when the inspection result of the automatic inspection process on the tablet displayed in the enlarged screen D341 is a check need, it is conceivable that, as shown in FIG. 40, the control part 21 causes the enlarged screen D341 to display a similar medicine list P361 indicating information on one or more types of tablets which are similar in identification information to the displayed tablet. Further, when the identification information is not attached to the tablet displayed in the enlarged screen D341, the control part 21 causes the similar medicine list P361 to display information on one or more types of tablets which are similar in shape to the displayed tablet. As the similar medicine list P361 is displayed in this manner, a user can easily determine the suitability of the tablet by referring to the similar medicine list P361. Further, as shown in FIG. 40, the manipulation key K362 may include a manipulation key which simultaneously receives a manipulation of sequentially switching the tablet to be displayed in the enlarged screen D341 forwardly and backwardly and a manipulation of inputting the purport that the tablet is determined to be proper by a visual check.

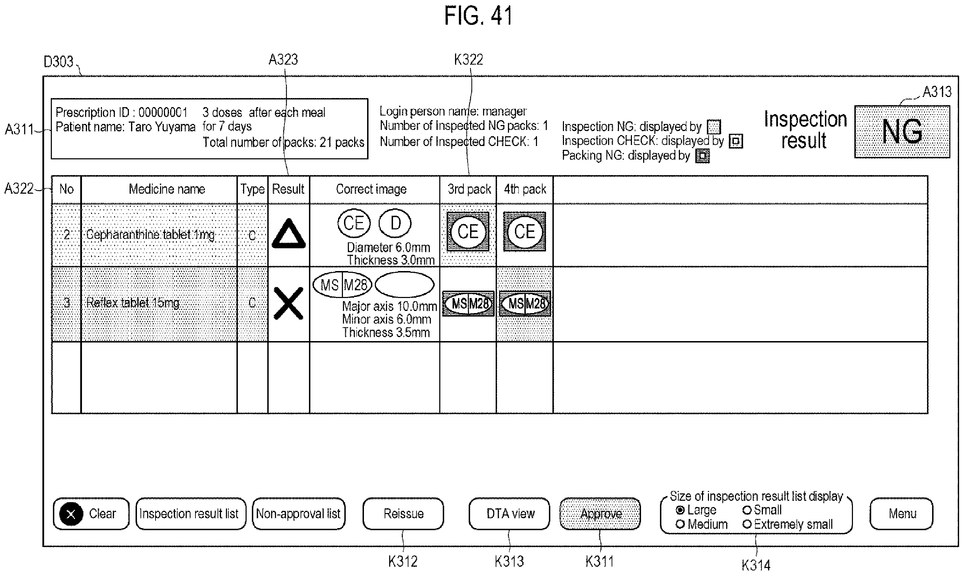

Further, as shown in FIGS. 36 and 37A, when an error or a check need is included in the inspection result of the automatic inspection process, the control part 21 causes the inspection detail screen D303 to display both the tablet whose inspection result of the automatic inspection process is OK and the tablet whose inspection result of the automatic inspection process is an error or a check need. On the other hand, as shown in FIG. 41, when an error or a check need is included in the inspection result of the automatic inspection process, it is conceivable that the control part 21 causes the inspection detail screen D303 to display only the tablet whose inspection result of the automatic inspection process is an error or a check need. That is to say, the display of the tablet whose inspection result of the automatic inspection process is proper is omitted. As a result, it is possible for a user to easily grasp only the inspection result which needs to be checked. Thus, the efficiency of a task of checking the inspection result of the automatic inspection process is improved. Further, when the tablet whose inspection result of the automatic inspection process is an error is included, "NG" is displayed at the inspection result area A313. When the tablet whose inspection result of the automatic inspection process is an error is not included and when the tablet whose inspection result of the automatic inspection process is a check need is included, "CHECK" is displayed at the inspection result area A313. Further, as shown in FIG. 37A, at the result display area A323 corresponding to the tablet whose inspection result is a check need, a mark "Δ", which indicates the purport that the inspection result is a check need, is displayed. At the result display area A323 corresponding to the tablet whose inspection result is an error, a mark "X", which indicates the purport that the inspection result is an error, is displayed. At the result display area A323 corresponding to the tablet whose inspection result is proper, a mark "O", which indicates the purport that the inspection result is proper, is displayed.

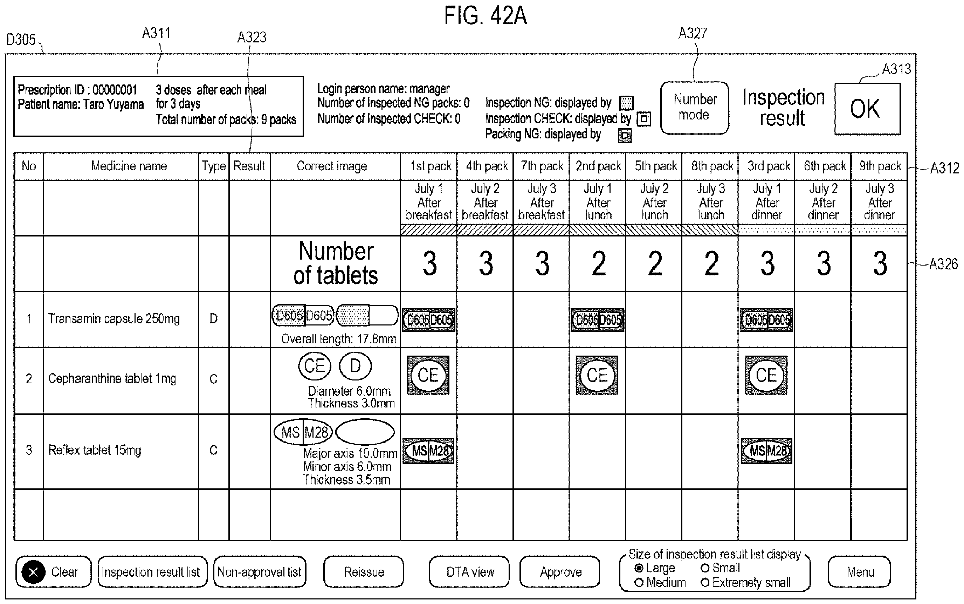

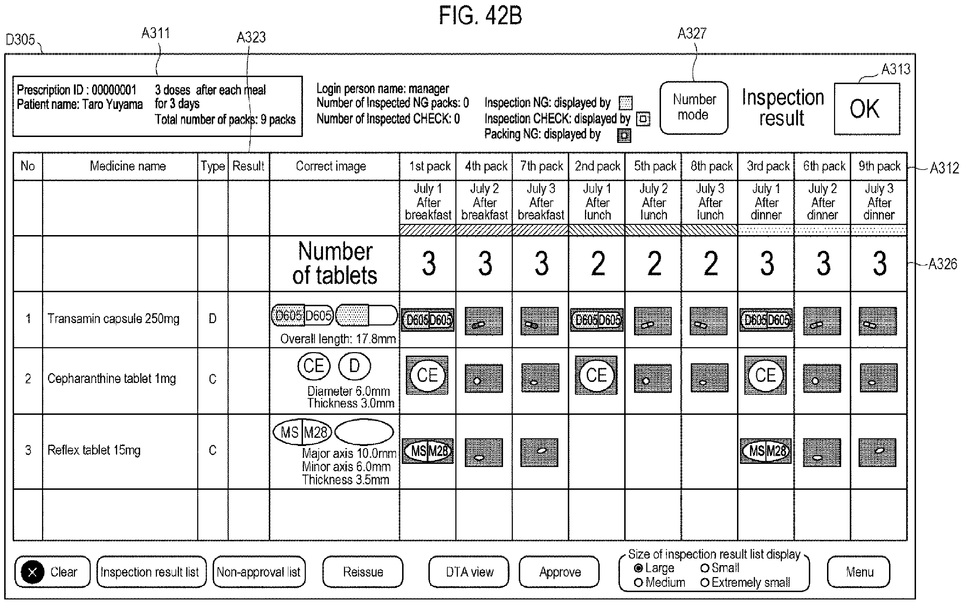

Further, the image inspection process is displayed in the inspection detail screen D303 shown in FIGS. 35A, 36 and 37. On the other hand, it is conceivable that the control part 21 displays, in place of the inspection detail screen D303, an inspection detail screen D305 in which both the inspection result of the image inspection process and the inspection result of the count inspection process are displayed. In this regard, FIGS. 42A and 42B show an example of the inspection detail screen D305.

Specifically, in the inspection detail screen D305 shown in FIGS. 42A and 42B, the count inspection process is executed with respect to all the medicine packs 451 by the automatic inspection process, and the image inspection process is executed only with respect to the first medicine pack 451 for each dosing time, and thus the inspection results is displayed with the first arrangement *(see* FIG. 27). In the inspection detail screen D305, a "Number mode", which indicates the purport that the image inspection process is omitted partially or in its entirety and the count inspection process is executed, is displayed at the display area A327.

In particular, in the inspection detail screen D305 shown in FIG. 42A, the control part 21 displays the capture image used in the image inspection process only with respect to the first medicine pack 451 at each dosing time. That is to say, the image capturing by the cameras 462 and 463 is performed with respect to the tablets in the medicine pack 451 for which the image inspection process has not been executed, but the capture images of such tablets is not displayed. Thus, a user can easily grasp whether the image inspection process has been executed. Further, the capture image used in the image inspection process is an image which is trimmed to extract the tablet region from the capture image actually captured by the cameras 462 and 463.

Further, in the inspection detail screen D305 shown in FIG. 42B, the control part 21 displays the capture image used in the image inspection process with respect to the first medicine pack 451 at each dosing time. On the other hand, regarding the tablet for which the image inspection process has not been executed, the control part 21 displays the capture image which is actually captured by the cameras 462 and 463 and is not trimmed at the time of executing the image inspection process. Thus, a user can easily grasp whether the image inspection process has been executed.

On the other hand, the count inspection process is executed with respect to all the medicine packs 451. Therefore, in the inspection detail screen D305, the control part 21 causes the number of the tablets used in the count inspection process of the respective medicine packs 451 to be displayed at a tablet number display area A326. Thus, a user can easily grasp that there is no problem in the number of tablets detected in the count inspection process.

Further, the control part 21 can display the inspection results obtained by the image inspection process and the count inspection process in the inspection detail screen D305. Specifically, when an error occurs in the image inspection process, regarding the tablet at which the error has occurred, a background of the capture image is displayed in a sixth specific color such as a red color indicating that the result of the automatic inspection process is an error. Further, when an error occurs in the count inspection process, regarding the medicine pack 451 at which the error has occurred, for example, a background of the tablet number display area is displayed in the sixth specific color such as a red color indicating an error.

Further, in case where the tablet packing device 4 includes a camera for capturing an image of the medicine pack 451 available after the tablets are packed in the packing unit 45, it is conceivable that the control part 21 causes the detail screen D305 to display the capture images which are captured by the camera after packing. For example, the capture images after packing are displayed side by side under the tablet number display area A326.

[Shift Detection Function] In the tablet packing device 4, for example, when a plurality of tablets is packed into the medicine pack 451 in the packing process executed by the packing unit 45, there is a concern that a shift occurs in which some tablets are shifted to and packed into the next medicine pack 451. Thus, it is conceivable that the tablet packing device 4 has a shift detection function of detecting the shift.

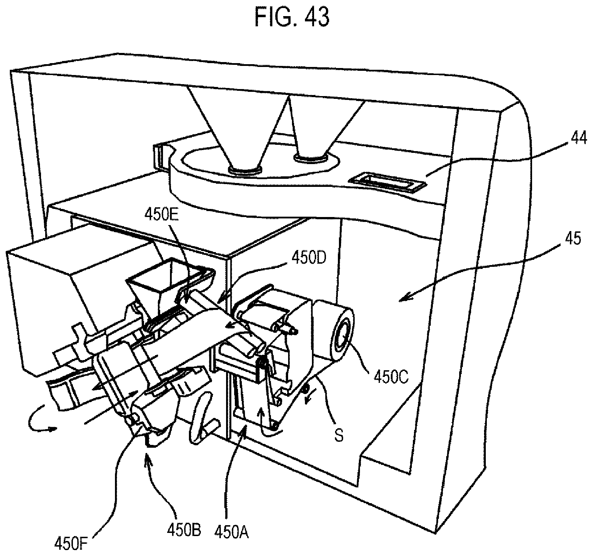

Specifically, the tablet packing device 4 includes a detection part 476 for detecting whether the tablet to be received in the medicine pack 451 when closing the medicine pack 451 exists at a downstream side of the medicine pack 451 to be closed. Then, based on the detection result of the detection part 476, the control part 40 detects the shift of the tablet to be received in the medicine pack 451. Hereinafter, the shift detection function is described with reference to FIGS. 43 to 52.

As shown in FIG. 43, the packing unit 45 is provided below the rotary unit 44 and is capable of packing the tablets M which are dispensed one pack dosage at a time from the rotary unit 44. The packing unit 45 includes a packing paper supply part 450A and a packing mechanism 450B. The packing paper supply part 450A is a mechanism that unwinds a packing paper S wound around a roll shaft 450C and sends the packing paper toward the packing mechanism 450B. The packing paper S is a heat-fusible sheet having a sheet shape and is wound around the roll shaft 450C in a state where the packing paper S is half-folded in a lateral direction. The packing mechanism 450B includes a sheet support portion 450D, a guide member 450E and a sealing device 450F. The packing mechanism 450B can pack the medicine M supplied from the rotary unit 44 by pressing the packing paper S sent from the packing paper supply part 450A into a bag shape.

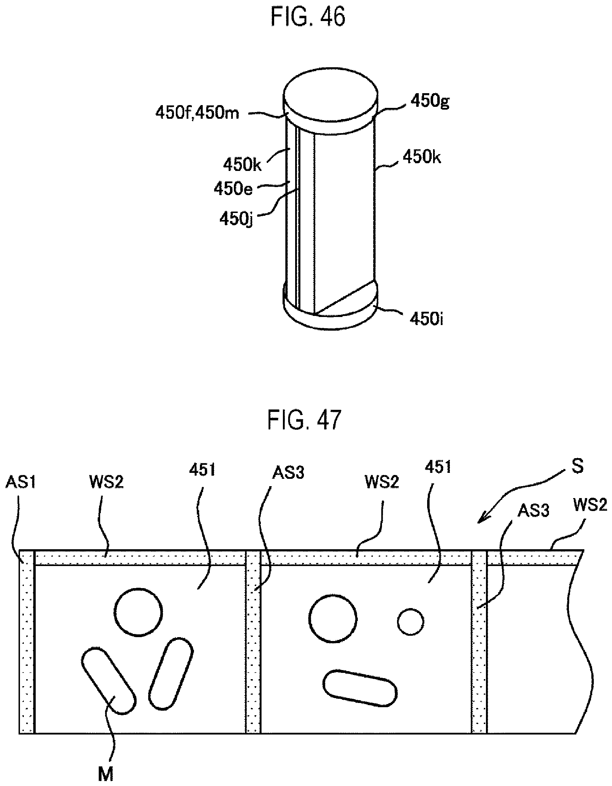

More specifically, the guide member 450E functions as a guide for guiding the packing paper S sent from the packing paper supply part 42. The sealing device 50 can press a longitudinal (downstream-side) end portion of the packing paper S, which is supplied while being guided by the guide member 44b, into a half-bag shape and can press and close an open portion of the packing paper S, which is formed in the half-bag shape, into a bag shape. More specifically, by pressing the packing paper S by the sealing device 50, it is possible to form the medicine pack 451 containing the medicine M as shown in FIG. 47. The sealing device 450F forms a longitudinal seal WS2, and forms a transversal seal (a first transversal seal AS1 or a second transversal seal AS3) closing a portion of the medicine pack 451 to be manufactured, which is located at a downstream side in a travel direction of the packing paper S, in the lateral direction of the packing paper S. As a result, a half-bag-shaped packing paper S (medicine pack 451) having an open portion at an upstream side in the travel direction of the packing paper S is formed. In this state, the medicine M is put into the half-bag-shaped packing paper S (medicine pack 451) and thereafter the open portion is closed by the sealing device 450F. That is to say, when a portion of the longitudinal seal WS2 remains unsealed, the unsealed portion of the longitudinal seal is closed by the sealing device 450F and is sealed by forming a transversal seal (second transversal seal AS3) which closes the unsealed portion at an upstream side of the travel direction of the packing paper S in the lateral direction of the packing paper S.

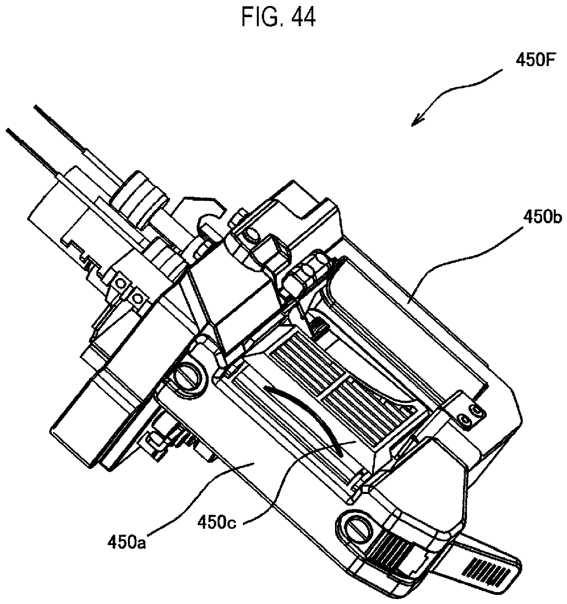

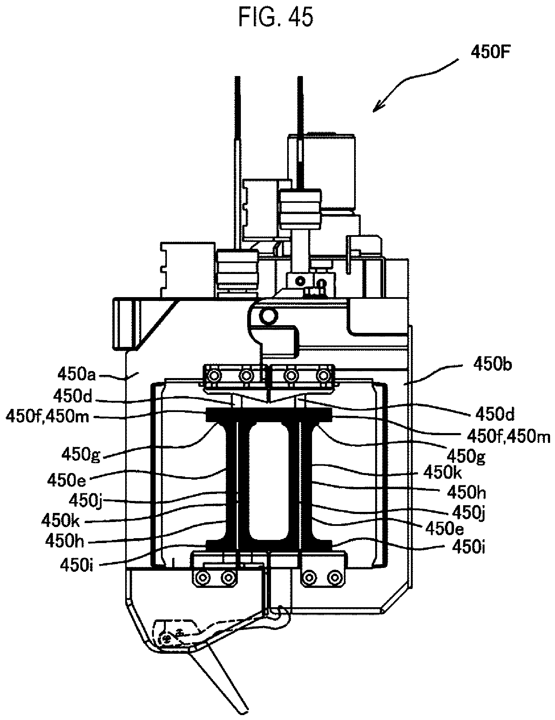

As shown in FIG. 44, the main portion of the sealing device 450F is configured by a pair of roller frames 450a and 450b. In the sealing device 450F, a protective cover 450c is provided at the roller frame 50a. In a state where the protective cover 450c is removed, as shown in FIG. 45, the roller frames 50a and 50b are substantially symmetrical in a left-right direction in a state where the roller frames abut against each other.

As shown in FIG. 45, the roller frames 450a and 450b are configured by metal frames having a substantially U-like shape (gate shape) which is viewed from a front. The roller frames 450a and 450b are provided with a vertically-extending support shaft 450d to which a transversal seal member 450e and a longitudinal seal member 450f are attached. Each of the transversal seal member 450e and the longitudinal seal member 450f are attached so as to be rotatable with respect to the support shaft 450d. Further, the transversal seal member 450e and the longitudinal seal member 450f are respectively connected to separate power sources (not shown) via separate power transmission mechanisms (not shown). Thus, the transversal seal member 450e and the longitudinal seal member 450f can rotate independently from each other. A length of the medicine pack 451 can be changed by changing rotational speeds of the transversal seal member 450e and the rotational speed of the longitudinal seal member 450f.

The transversal seal member 450e is made of metal. As shown in FIG. 45, the transversal seal member 450e has a substantially linear shape when viewed from the front. As shown in FIG. 46, the transversal seal member 450e includes a disk-shaped lower end portion 450i and a plate-shaped heating portion 450k. The heating portion 450k is located between an upper end portion 450g and a lower end portion 450i of the longitudinal seal member 450f, which are described below, and is substantially perpendicular to the upper end portion 450g and the lower end portion 450i. On both side surfaces of the heating portion 450k, a heater 450h and a tear-off line forming portion 450j are linearly arranged from the upper end portion 450g toward the lower end portion 450i. The heaters 450h and 450h are capable of thermally fusion-bonding the packing paper S. Therefore, by rotating the transversal seal members 450e and 450e arranged in parallel and allowing the half-folded packing paper S to pass between the transversal seal members 450e and 450e, it is possible to form a seal (transversal seal) extending in the lateral direction of the packing paper S.

Further, the tear-offline forming portion 450j is capable of forming perforations in the packing paper S. In the present embodiment, the tear-offline forming portion 450j of the roller frame 450b is configured by a cutter for forming perforations. The tear-off line forming portion 450j of the roller frame 450a is configured by a receiving blade provided in association with the cutter.

As shown in FIG. 45, the longitudinal seal member 450f includes the above-described upper end portion 450g and a heater 450m. The upper end portion 450g is a disk-shaped member provided at an upper portion of the heating portion 450k of the transversal seal member 450e. The heater 450m is provided around the entire circumferential periphery of the upper end portion 450g. Therefore, by rotating the longitudinal seal members 450f and 450f arranged in parallel and allowing the half-folded packing paper S to pass between the upper end portions 450g and 450g, it is possible to form a seal (longitudinal seal) extending in the longitudinal direction of the packing paper S.

As shown in FIG. 45, in the sealing device 450F, the transversal seal members 450e and 450e and the longitudinal seal members 450f and 450f are disposed in the substantially rectangular area surrounded by the roller frames 450a and 450b, in substantially parallel with each other with a predetermined clearance therebetween. The sealing device 450F can form the medicine pack 451 by rotating the transversal seal members 450e and 450e and the longitudinal seal members 450f and 450f and allowing the packing paper S to pass through the clearance to form longitudinal seals and transversal seals.

As shown in FIG. 48, the rotary unit 44 is provided with a medicine introduction part 80 for supplying the tablets M individually dispensed from the rotary unit 44 to the packing unit 45. The medicine introduction part 80 may be of any type as long as it can supply the tablets M into the packing paper S. In the present embodiment, the medicine introduction part 80 is configured by a hopper. As shown in FIGS. 48 and 49, the medicine introduction part 80 is inserted into the open portion of the unsealed medicine pack 451 formed from the packing paper S by the sealing device 450F and is capable of introducing the tablets M into the medicine pack 451. Specifically, the medicine introduction part 80 is disposed such that a proximal end portion thereof faces toward the rotary unit 44 and a distal end portion thereof is inserted into the unsealed medicine pack 451 which is being formed by the sealing device 450F. That is to say, the medicine introduction part 80 is inserted into the half-folded packing paper S at an upstream side of the travel direction of the packing paper S with respect to the sealing device 450F.

In the tablet packing device 4, the tablets received in the medicine pack 451 may be affected by the heat generated from the heaters 450h and 450m. Therefore, as shown in FIG. 49, the tablet packing device 4 is provided with a blower fan 51 capable of blowing an air toward the packing paper S in a position where the tablets are supplied from the medicine introduction part 80 to the packing paper S. Thus, the packing paper S is cooled by the air blown from the blower fan 51, and the influence of the heat generated from the heaters 450h and 450m is suppressed. Specifically, as shown in FIG. 49, the blower fan 51 is disposed above the passage path of the packing paper S and is capable of blowing the air to an inside of the packing paper S through the open portion of the packing paper S. Further, the blower fan 51 may be disposed in a position where the blower fan 51 can blow the air in another direction, such as a position below the passage path of the packing paper S.

Further, as shown in FIGS. 48 and 49, the packing unit 45 is provided with a detection part 476 for detecting the presence of the tablets M in an introduction path of the tablets M introduced by the medicine introduction part 80. As shown in FIG. 48, the detection part 476 includes a camera 476a capable of capturing an image of the introduction path of the tablets M introduced by the medicine introduction part 80, and an illumination device 476b. The camera 476a is disposed so as to capture (detect) the inside of the packing paper S at an upstream side of the sealing device 450F in a conveyance direction of the packing paper S. In the present embodiment, the camera 476a is disposed from the proximal end of the medicine introduction part 80 toward the distal end of the medicine introduction part 80. Further, as described above, the medicine introduction part 80 is located at an upstream side of the sealing device 450F. Therefore, the camera 476a is disposed so as to capture (detect) an area located at the upstream side of the sealing device 450F in the conveyance direction of the packing paper S. Further, the illumination device 476b includes a light source such as a light emitting diode or an electric bulb. Similar to the camera 476a, the illumination device 476b is arranged so as to illuminate an internal region of the medicine introduction part 80 from the proximal end of medicine introduction part 80 to the distal end of the medicine introduction part 80. Further, the control part 40 stores the capture image captured by the camera 476a in the storage part 22 and transmits the capture image to the server 2.

In the tablet packing device 4, the control part 40 can decide occurrence of an abnormal packing caused by the overflow of the tablets M from the medicine pack 451 into which the tablets M have to be packed originally, based on the detection data inputted from the detection part 476.

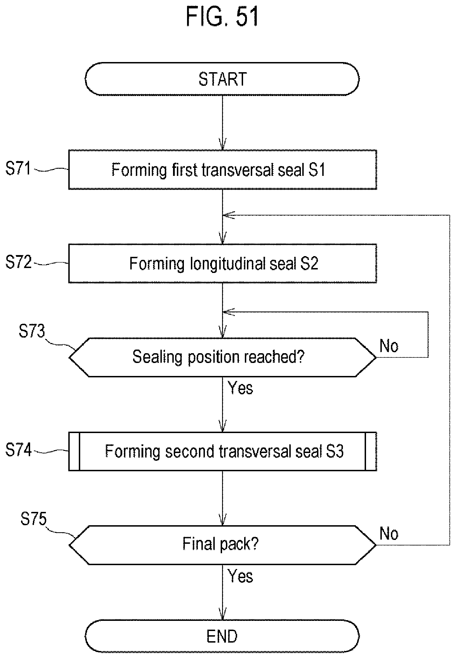

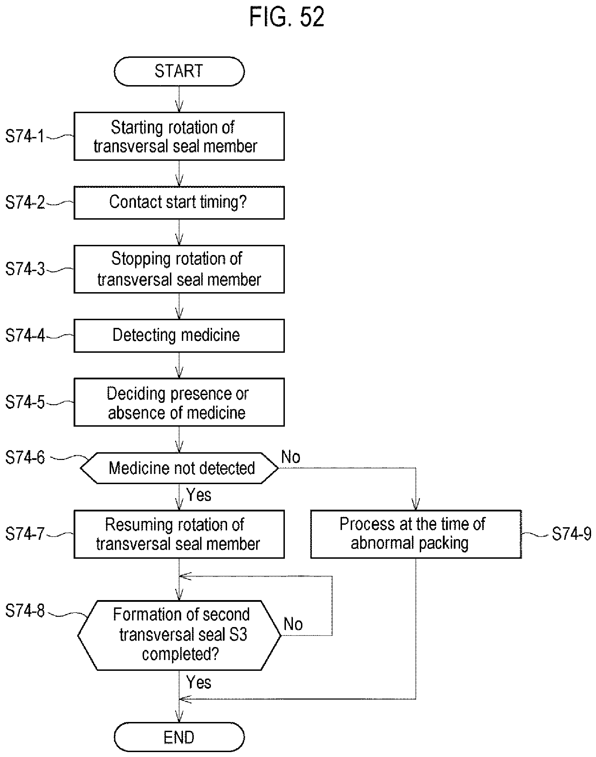

Subsequently, descriptions are made as to a method of forming the medicine pack 451 by the sealing device 450F, which is performed in the tablet packing device 4, and a method of deciding an abnormal packing, which is performed in the process of forming the medicine pack 451. In the following descriptions, the method of forming the medicine pack 451 is first described with reference to FIG. 51. Then, a subroutine related to a step of forming a second transversal seal is described with reference to FIG. 52.

[Regarding Method of Forming Medicine Pack 451] The control part 40 forms the medicine pack 451 in accordance with a control flow shown in FIG. 51. Hereinafter, a specific operation and control are described with reference to FIG. 51.
<Step S71> When forming the medicine pack 451, in step S71, a transversal seal (hereinafter also referred to as "first transversal seal AS1") for closing an upstream end of the medicine pack 451 located in a leading position in the traveling direction of the packing paper S is formed by the transversal seal members 450e and 450e (*see* FIG. 47). Thereafter, the control flow proceeds to step S72.
<Step S72> In step S72, a longitudinal seal WS2 (*see* FIG. 47) is formed for closing an end portion located opposite to a folding line of the packing paper S supplied in a half-folded state. Specifically, the longitudinal seal is formed by rotating the longitudinal seal members 450f and 450f and allowing the packing paper S to pass between the longitudinal seal members 450f and 450f.
<Step S73> In step S73, it is checked whether the longitudinal seal WS2 has been formed until the packing paper S reaches a position (sealing position) where the medicine pack 451 is to be sealed. If it is determined that the longitudinal seal WS2 has reached the sealing position (step S73: YES), the control flow proceeds to step S74. If it is determined that the longitudinal seal WS2 has not reached the sealing position (step S73: NO), the control flow returns step S72.
<Step S74> In step S74, a transversal seal (hereinafter also referred to as "second transversal seal AS3") for closing a downstream end of the medicine pack 451 in the traveling direction of the packing paper S is formed in accordance with a subroutine of FIG. 52 which is described below. In this regard, the second transversal seal AS3 functions as the first transversal seal AS1 of the medicine pack 451 to be formed next. Therefore, the second transversal seal AS3 functions as seals that form boundaries of the medicine packs 451 continuously formed in the longitudinal direction of the packing paper S. If the second transversal seal AS3 is formed, the control flow proceeds to step S75.
<Step S75> In step S75, it is checked whether the medicine pack 451 sealed by the second transversal seal AS3 in step S74 is the final one. If the medicine pack 451 sealed in step S74 is not the final one (step S75: NO), the control flow returns to step S72. If the medicine pack 451 sealed in step S74 is the final one (step S75: YES), a series of the control flow is completed.

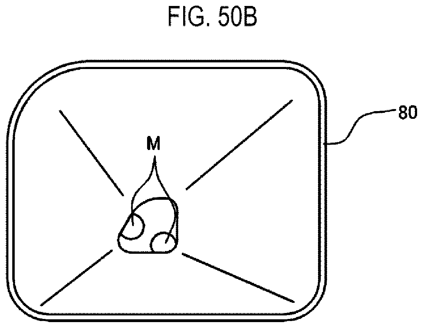

[Regarding Steps of Forming Second Transversal Seal AS3] Subsequently, a subroutine of the step of forming the second transversal seal AS3 related to the above-described step S74 is described in detail with reference to FIG. 52.
<Step S74-1> In step S74-1, in order to form the second transversal seal AS3, the rotation of the transversal seal members 450e and 450e is started such that the heating portions 450k and 450k are positioned so as to face toward each other. Thereafter, the control flow proceeds to step S74-2.
<Step S74-2> In step S74-2, it is checked whether the heating portions 450k and 450k of the transversal seal members 450e and 450e have reached the point of time (contact start timing) at which the heating portions 450k and 450k start making contact with the packing paper S as shown in FIG. 6. In this regard, whether the heating portions have reached the contact start timing can be checked by various methods. Specifically, for example, the following methods are conceivable: a method of providing a timer that starts time counting from a timing at which the rotation of the transversal seal members 450e and 450e is started in step S74-1 and checking a lapse of predetermined time by the timer; a method of providing a rotation detection device capable of detecting the rotation amount of the transversal seal members 450e and 450e and checking whether the rotation detection amount has reached a predetermined amount; or a method of providing a detection device capable of detecting the angle or position of the transversal seal members 450e and 450e and checking whether the heating portions 450k and 450k of the transversal seal members 450e and 450e have reached an angle or position at which the contact is started. If it is checked by these methods that the heating portions have reached the contact start timing (step S74-2: YES), the control flow proceeds to step S74-3. If not checked (step S74-2: NO), the control flow continues as it stays in step S74-2.
<Step S74-3> In step S74-3, control is performed to temporarily stop the rotation of the transversal seal members 450e and 450e. As a result, the transversal seal members 450e and 450e are temporarily stopped in the position in which the heating portions 450k and 450k start making contact. Thereafter, the control flow proceeds to step S74-4.
<Step S74-4> In step S74-4, the detection part 476 detects whether the tablets M are present at the internal region of the medicine introduction part 80 and at an area which is located inside the packing paper S and is located in the conveyance direction at an upstream side of the transversal seal (the first transversal seal AS1 or the second transversal seal AS3) which is already formed by the sealing device 450F (sealing detection). At this time, the illumination device 450b is turned on and the internal region of the medicine introduction part 80 is illuminated. Detection data obtained by the detection part 476 is inputted to the control part 40. The detection data may be any data as long as it is valid data for deciding the presence or absence of the tablets M. In the present embodiment, image data captured by the camera 467a is inputted to the control part 40 as the detection data. Specifically, if the tablets M are not present, an image of only the medicine introduction part 80 is acquired as shown in FIG. 50A. If the tablets M are present, an image containing the tablets M is acquired as shown in FIG. 50B. Such image data are inputted to the control part 40 as the detection data. Thereafter, the control flow proceeds to step S74-5.
<Step S74-5> In step S74-5, a decision part 102 of the control part 40 decides the presence or absence of the tablets M based on the detection data (image data) acquired by the sealing detection performed in step S74-4. In the present embodiment, since the image data is acquired as the detection data, the presence or absence of the tablets M is decided by a method utilizing the image data, such as image analysis or the like. The decision on the presence or absence of the tablets M may be made by any method. For example, an image, which is obtained by the camera 92 when the tablets M do not exist, may be prepared as a master image, and the presence or absence of the tablets M may be decided using the image actually obtained by the camera 476a and the master image. Further, when the decision is made using the master image as described above and when the packing paper S appears in the image obtained by the camera 476a, it is preferable that different master images are prepared according to the type of the packing paper S. Specifically, the packing paper S is supplied in a state where the packing paper S is half-folded such that two surfaces overlap with each other. In the packing paper S, the two surfaces may be transparent. Alternatively, one surface may be transparent and an opaque portion (for example, a band having a color such as white color or the like) may be provided on the other surface. It is obvious that a difference exists in the images obtained by the camera 476a between the case where the former packing paper S is used and the case where the latter packing paper S having the opaque portion is used. Therefore, it is preferable that master images are prepared according to the type of the packing paper S so as to cope with the difference of the packing paper S. After the decision on the tablets M is made as described above, the control flow proceeds to step S74-6.
<Step S74-6> In step S74-6, it is checked whether the tablets M are detected as a result of the decision in step S74-5. If the tablets M are not detected (step S74-6: YES), it is assumed that the tablets M to be packed are packed without leakage from the medicine pack 451. In this case, it is determined that the packing of the tablets M is normally performed, and the control flow proceeds to step S74-7. On the other hand, if the tablets M are detected (step S74-6: NO), it is highly likely that, as shown in FIG. 48, the tablets M to be packed into the preceding medicine pack 451 (a medicine pack 451a shown at a lower side in the figure) are leaked out of the medicine pack 451 and are moved to the medicine pack 451 to be formed later (a medicine pack 451b being formed at an side in the figure). In this case, it is determined that an abnormal packing occurs, and the control flow proceeds to step S74-9.
<Step S74-7> In step S74-7, control is performed for restarting the rotation of the transversal seal members 450e and 450e which were temporarily stopped in step S74-3. As a result, in the transversal seal members 450e and 450e, the heating portions 450k and 450k start making surface-to-surface contact and the second transversal seal AS3 is formed.
<Step S74-8> In step S74-8, it is checked whether the formation of the second transversal seal AS3 has been completed. If it is determined that the formation of the second transversal seal AS3 has been completed (step S74-8: YES), a series of control flow is completed. On the other hand, if it is determined that the formation of the second transversal seal AS3 has not been completed (step S74-8: NO), the control of step S74-8 is continuously performed.
<Step S74-9> In step S74-9, a process for coping with that the leakage of the tablets M is checked in the above-mentioned step S74-6 (a process of an abnormal packing) is performed. Specifically, a process of informing the occurrence of an abnormal packing by a method such as voice, image display, lamp lighting or the like is executed as the process of an abnormal packing. As a result, a series of control flow shown in FIG. 52 is completed.

As described above, in the tablet packing device 4 of the present embodiment, it is decided that the abnormal packing occurs under the condition that the presence of the tablets M is detected by the detection part 476 within a period after the timing of starting the sealing of the medicine pack 451 into which the tablets M are introduced and before the timing at which the tablets M to be packed are introduced into the next medicine pack 451. By doing so, it is possible to accurately detect the abnormal packing caused by the overflow of the tablets M from the medicine pack 451 to be originally packed and to minimize the labor needed for inspection.

Further, in the above-described tablet packing device 4, the timing at which the transversal seal member 450e for forming the second transversal seal AS3 makes contact with the packing paper S is set as a timing at which the sealing of the medicine pack 451 into which the tablets M have been introduced is started. At this timing, the bonding of the packing paper S by the sealing device 450F is interrupted and the tablets M are detected by the detection part 476. As a result, it is possible to more accurately detect the abnormal packing caused by the leakage of the tablets M at the time of sealing the medicine pack 451. Further, it is possible to suppress such a trouble that the tablets M leaking out of the medicine pack 451 are pinched between the transversal seal members 450e and 450e.

Further, the present embodiment has illustrated the example where the tablets M are detected by the detection part 476 by regarding the timing at which the transversal seal member 450e makes contact with the packing paper S to form the second transversal seal AS3 as the timing at which the sealing of the medicine pack 451 is started. However, the present invention is not limited thereto. The same process may be performed by regarding other timing as the timing at which the sealing the medicine pack 451 is started. Further, the present embodiment has illustrated the example where the tablets M are detected by the detection part 476 at the timing at which the sealing of the medicine pack 451 is started. However, the present invention is not limited thereto. The presence of the tablets M may be detected at an arbitrary timing within a period from the timing at which the sealing of the medicine pack 451 is started to the timing at which the sealing is completed (for example, after the sealing). Further, the present embodiment has illustrated the example where the sealing by the transversal seal member 450e is temporarily stopped at the timing at which the tablets M are detected by the detection part 476. However, the present invention is not limited thereto. Specifically, when the presence or absence of the tablets M is detected by the detection part 476, the sealing by the transversal seal member 450e may not be stopped, or the speed of formation of the seal formed by the transversal seal member 450e may be reduced.

The present embodiment has illustrated the example where, in order to be compatible with the medicine packs 451 having different lengths, the sealing device 450F is configured to individually drive and control the roller-shaped transversal seal members 450e and 450e and the roller-shaped longitudinal seal members 450f and 450f. However, the present invention is not limited thereto. That is to say, in case where it is preferable that the length of the medicine pack 451 is constant, the sealing device 450F may be configured such that the transversal seal members 450e and 450e and the longitudinal seal members 450f and 450f are driven as a single unit.

The present embodiment has illustrated the example where the packing paper S is pinched and sealed by the roller-shaped transversal seal members 450e and 450e and the roller-shaped longitudinal seal members 450f and 450f to form the medicine pack 45. However, the present invention is limited thereto. The medicine pack 451 may be formed by sealing the packing paper S in another form and manner.

Further, the present embodiment has illustrated the example where the medicine pack 451 is formed by applying a seal to the overlapped portion of the half-folded packing paper S. However, the present invention is not limited thereto. Specifically, two packing papers S may be supplied and the medicine pack 451 may be formed by superimposing and bonding the packing papers S.

The present embodiment has illustrated the example where the detection part 476 is provided with the camera 476a as a detection device for detecting the tablets M. However, the present invention is not limited thereto. The detection part 476 may be any device as long as it can detect the presence of the tablets M. Specifically, as the detection part 476, it may be possible to provide an optical sensor, an infrared sensor or the like capable of detecting the presence or absence of the tablets M at the inside of the packing paper S and at an upstream side of the sealing device 450F in the conveyance direction of the packing paper S. Further, when an optical sensor or the like is used as the detection part 476, it is preferable to take measures in view of the characteristics of such a sensor so that sufficient detection accuracy can be obtained. Specifically, if the detection distance of an optical sensor or the like is set to become short, there is a concern that the detection accuracy for the presence of a small tablet M is lowered. That is to say, when the size of the tablet M is small, the distance from the optical sensor or the like to the surface of the tablet M is larger than when the size of the tablet M is large. Therefore, if the detection distance of the optical sensor or the like is set to become short, there is a concern that the detection accuracy for a small tablet M decreases. On the other hand, if the detection distance of the optical sensor or the like is set to become long, there is a concern that the transversal seal member 450e or the like is detected as the tablet M. Accordingly, when an optical sensor or the like is employed in the detection part 476, it is preferable to take measures such as a measure of setting the detection distance in view of the size of the tablet M to be handled.

The detection part 476 may be disposed in any position as long as it can detect the presence of the tablet M at the inside of the packing paper S and at the upstream side of the sealing device 450F in the conveyance direction of the packing paper S. More specifically, as shown by a two-dot chain line in FIG. 49, a detection part 476X similar to the detection part 476 may be disposed at the distal end of the medicine introduction part 80, or a detection part 476Y may be disposed at an upstream side of the medicine introduction part 80.

Further, in addition to the detection part 476, another sensor may be provided in order to improve the detection accuracy of the correct packing of the tablets M. Specifically, a fall sensor for detecting the falling of the tablets M may be provided in the medicine introduction part 80 or the like. If decision conditions are that the tablets M are detected by the fall sensor and that the leakage of the tablets M is not detected based on the detection result of the detection part 476, it is possible to more accurately detect whether the tablets M dispensed for packing purposes have been correctly packed.

The present embodiment has illustrated the example where the detection part 476 is utilized to detect that the tablets M are correctly packed at the time of sealing the medicine pack 451. However, the detection part 476 may be utilized for other purposes. Specifically, the detection part 476 may detect the presence of the tablets M at the time point at which the tablets M for packing purposes are to be introduced into the packing paper S from the medicine introduction part 80. That is to say, the detection part 476 may be used not only for the above-described sealing detection, but also for the introduction detection for detecting the introduction of the tablets M into the packing paper S by the medicine introduction part 80. In this case, the detection part 476 can be effectively used for checking whether the tablets M have been supplied into the packing paper S. Accordingly, it is possible to check the non-leakage of the tablets M at the time of sealing the packing paper S (forming the medicine pack 451) through the sealing detection and the reliable supply of the tablets M through the introduction detection. Only when both the non-leakage of the tablets M and the reliable supply of the tablets M can be checked, it is decided that the tablets M have been normally packed. Therefore, it is possible to further improve the decision accuracy of the correct packing of the tablets M.

The present embodiment has illustrated the example where the presence or absence of the tablets M is detected by the detection part 476 at the timing of starting the formation of the second transversal seal AS3. However, the present invention is not limited thereto. That is to say, the tablets M may be detected by the detection part 476 at any timing as long as the timing falls within a period (hereinafter also referred to as "detectable period") after the timing of starting the sealing of the medicine pack 451 into which the tablets M are introduced and before the timing at which the tablets M to be packed are introduced into the medicine pack 451 to be formed next through the medicine introduction part 80. Specifically, the detection of the tablets M by the detection part 476 may be performed within a period after completion of the sealing of the medicine pack 451 into which the tablets M has been introduced (after formation of the second transversal seal AS3) and before the timing at which the tablets M to be packed are introduced into the medicine pack 451 to be formed next. Further, the detection of the detection part 476 may be performed at a predetermined timing (time point) within the above detection period. Further, the detection by the detection part 476 may be continuously performed over a predetermined period included within the aforementioned detectable period, or may be intermittently performed within the detectable period.

The present embodiment has illustrated the example where the packing paper S is pinched and bonded between the roller-shaped members which are comprised of the transversal sealing members 450e and 450e and the longitudinal sealing members 450f and 450f. However, the present invention is not limited thereto. A device capable of bonding the packing paper S by another method can be employed in place of the sealing device 450F. Specifically, a pair of plate-shaped heating bodies, which have a planar shape such as a T shape or the like and are capable of pinching and bonding the packing paper S therebetween, may be used instead of the sealing device 450F of the present embodiment.

[Packing View Display Function] In the process (*see* FIG. 52) executed to realize the shift detection function, the image capturing by the camera 476a is performed at the time point (contact start timing) at which the heating portions 450k and 450k of the transversal seal members 450e and 450e start to make contact with the packing paper S. On the other hand, it is conceivable that, independently of the contact start timing, the control part 21 uses the camera 476a to capture an image when the tablets are fed from the rotary unit 44 through the medicine introduction part 80 into the medicine pack 451 formed by the packing unit 45.

In this regard, it is conceivable that the feed timing at which the tablets are fed is a detection timing at which the tablets are detected by the passage detection sensor 475. Further, the feed timing is a timing set in advance as a timing at which the tablet dispensed from the paper supply cassette 41 or the manual distribution unit 42 of the tablet packing device 4 is introduced from the rotary unit 44 into the medicine introduction part 80. Thus, the image capturing range of the camera 476a includes the medicine introduction part 80, the tablets and the medicine pack 451. Further, when the shift does not occur, the tablets fed into the medicine introduction part 80 or the medicine pack 451 are captured in the capture image captured at the feed timing. However, the tablets are not included in the capture image obtained at the contact start timing because the tablets is moved to a downstream side of the transversal seal member 450e.

Then, the control part 40 stores the capture images captured by the camera 476a in the storage part 22 and transmits the capture image to the server 2. Thus, in the tablet packing device 4 or the server 2, the control part 40 or the control part 21 can realize a packing view display function of displaying the capture images.

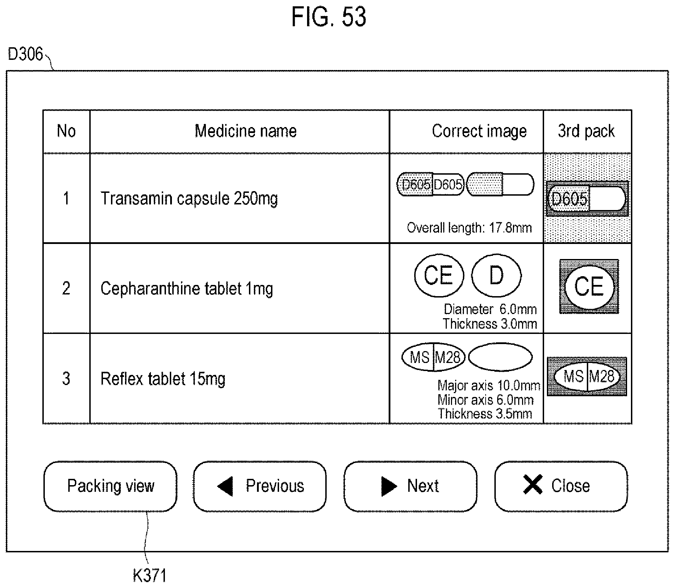

For example, in the server 2, when the medicine pack identification information such as a medicine pack number or the like for identifying the medicine pack 451 is selected on the inspection detail screens D301 to D303 or the like, the control part 21 displays a medicine pack individual information screen D306 which shows a list of tablets received in the pack 451. In this regard, FIG. 53 shows an example of the medicine pack individual information screen D306.

As shown in FIG. 53, a medicinal name, a correct image, a capture image, etc. of each of the tablets received in the third medicine pack 451 in the packing process are displayed in the medicine pack individual information screen D306. Further, in the medicine pack individual information screen D306, regarding the tablet whose inspection result of the automatic inspection process is an error among the tablets received in the displayed medicine pack 451, a background of the display area of the capture image is displayed in the sixth specific color such as a red color indicating the purport that the result of the automatic inspection process is an error.

Further, a manipulation key K371 is displayed in the medicine pack individual information screen D306. In response to a manipulation of the manipulation key K371, the control part 21 causes the medicine pack individual information screen D306 to display a packing screen D307 in which the capture image obtained by capturing the tablets received in the medicine pack 451 with the detection part 476.

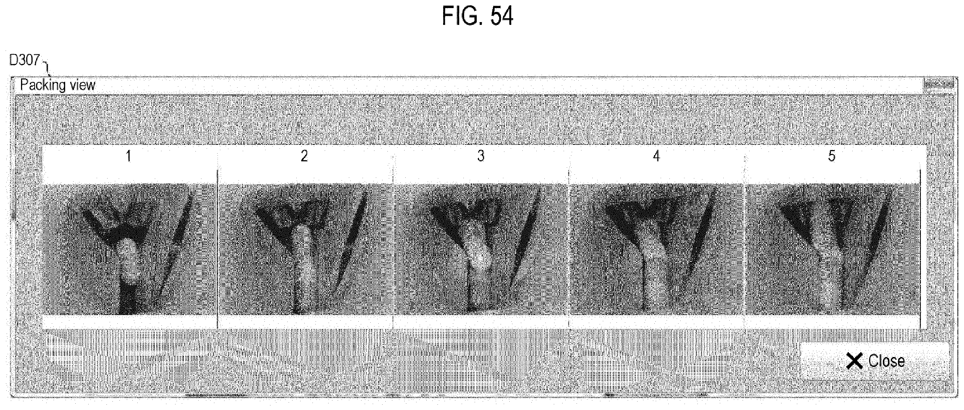

In this regard, FIG. 54 shows an example of the packing screen D307. As shown in FIG. 54, in the packing screen D307, the capture images of the tablet, which are captured by the detection part 476 whenever the tablets fall down from the rotary unit 44 through the medicine introduction part 80 toward the medicine pack 451, are displayed in the image capturing order of the capture images. As described above, the image capturing range of the capture images includes the tablet introduction part 80, the tablets and the medicine pack 451. Thus, a user can easily grasp the falling state of each tablet dropped into the medicine pack 451 by referring to the packing screen D307.

Further, the control part 21 may cause the packing view D307 to display the capture image captured at the contact start timing, in place of or together with the capture image captured at the feed timing. Further, when the occurrence of the shift is detected by the shift detection function, it is conceivable that the control part 21 causes the packing view D307 to display the capture image captured at the contact start timing and a message indicating the purport that the shift has occurred.

Further, the configurations and functions described in the first embodiment and the second embodiment can be arbitrarily combined. Further, the subject having the configurations and processing functions described in the first embodiment and the second embodiment is not limited to those described herein. At least one of the server 2, the client terminal 3, the tablet packing device 4 and the like may have the configurations and processing functions described in the first embodiment and the second embodiment. For example, it is conceivable that a process equivalent to the automatic inspection process is executed by the control part 21 in the server 2, and that a process equivalent to the inspection assistance process is executed by the control part 40 in the tablet packing device 4.

[Third Embodiment] Subsequently, descriptions are made as to an inspection assistance system 1 according to a third embodiment of the present invention. The configurations not described here are the same as those described in the first embodiment or the second embodiment.

As described in the first embodiment or the second embodiment, in the inspection assistance system 1, the control part 40 executes an image inspection process that collates, by means of images, the identification information of the tablet acquired from the capture image with the identification information of the tablet corresponding to the type of the tablet included in the prescription data. Specifically, in the image inspection process, a matching process, which collates the image corresponding to the identification information of the tablet included in the capture image with the correct image preregistered in association with the identification information of the tablet included in the prescription data, is executed by image comparison. Further, the correct image corresponding to the identification information of the tablet included in the prescription data is a registration image which is preregistered in the medicine master or the like as a correct image of a tablet corresponding to the identification information.

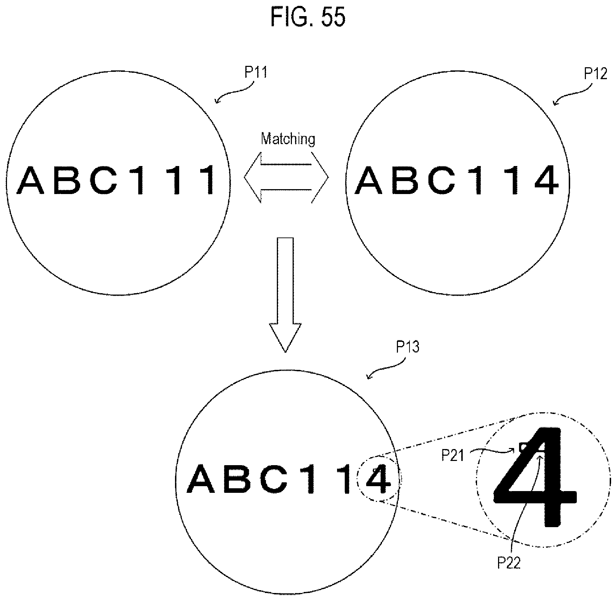

First, with reference to FIG. 55, descriptions are made as to a case where the image inspection process is executed using a method of a first matching process for detecting, as a coincidence degree, the amount of overlapped portion in two images. In this regard, descriptions are made by way of example where: tablet identification information subject to the image inspection process is "ABC111"; a correct image P11 corresponding to a tablet is registered in the medicine master; and a capture image P12 of the tablet whose identification information is "ABC114" is captured as the capture image as shown in FIG. 55. In this case, in the first matching process, the coincidence degree (coincidence ratio) is detected by comparing the correct image P11 and the capture image P12. When the coincidence degree is equal to or greater than a pre-set threshold value, it is determined that the collation result is coincidence. For example, on the basis of the correct image P11, the number of black pixels, which are included in the capture image P12 among black pixels included in the correct image P11, is counted, and a ratio of the number of the black pixels included in the capture image P12 to the number of all the black pixels of the correct image is calculated as the coincidence degree. Therefore, when the most of the correct image P11 is included in the capture image P12, even if extra black pixels are included in the capture image P12, the coincidence degree may be high and the collation result may be coincidence.

For example, a composite image P13 shown in FIG. 55 is an image obtained by composing the correct image P11 and the capture image P12. As shown in FIG. 55, in the composite image P13, the identification information "ABC111" of the correct image P11 and the identification information "ABC114" of the capture image P12 differ from each other by one character. However, only regions P21 and P22 of the capture image P11 are not included in the capture image P12. Therefore, there is a concern that the coincidence degree increases and the collation result is coincidence thereby.

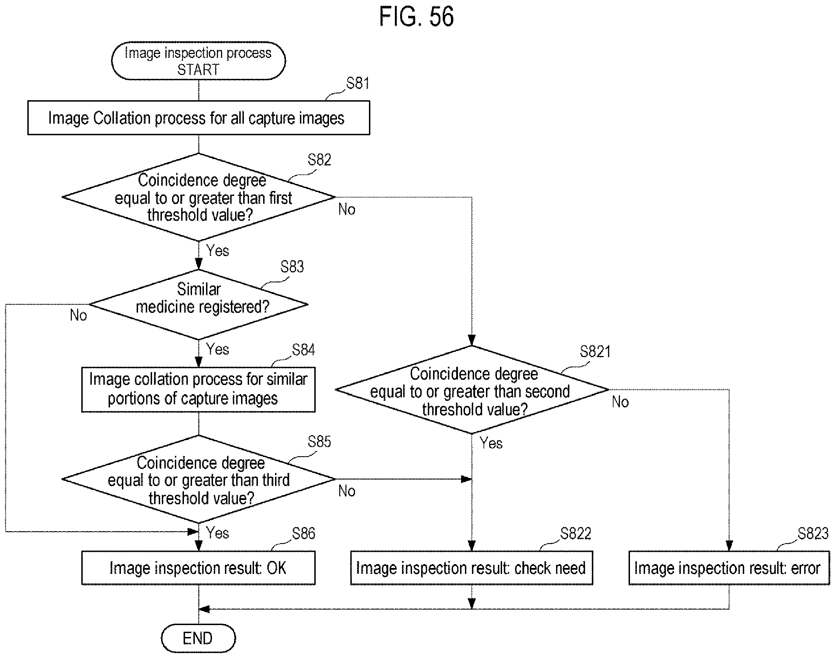

In this regard, in the inspection assistance system 1 according to the present embodiment, an image inspection process (*see* FIG. 56) described below is executed by the control part 40 of the tablet packing device 4, whereby a highly accurate image inspection process is realized. For example, in the second embodiment, the image inspection process is executed instead of steps S62 to S64 of the automatic inspection process (*see* FIG. 34). Further, as described above, the image inspection process may be executed by the control part 21 of the server 2.

Further, in the inspection assistance system 1 according to the present embodiment, the medicine master is stored in the storage part 49. In the medicine master, a similar tablet is associated and stored in advance as a similar medicine. For example, a tablet whose tablet identification information is different by one character is associated as the similar medicine. Specifically, in the medicinal master, the same similar group code is set to similar tablets. Further, when the similar group code is set, a portion of the tablet similar to other tablets is set in advance as a similar portion. In the present embodiment, it is assumed that the same similar group code is set to the tablet corresponding to the identification information "ABC111" and the tablet corresponding to the identification information "ABC114", and that a first place area corresponding to "1" and "4", which are a different character between the tablets, is set as the similar portion. Further, the similar group code and the similar portion are registered in the medicine master by the control part 40 in response to, for example, a user's manipulation using the manipulation display part 48.

[Image Inspection Process] Hereinafter, an example of the image inspection process executed by the inspection assistance system 1 according to the present embodiment is described with reference to FIG. 56. As described above, in the inspection assistance system 1, before executing the image inspection process, a capture image of a tablet subject to being packed is acquired in the packing process executed by the tablet packing device 4. Further, even when acquiring the capture image in the packing process, the control part 40 determines whether the tablet is in the vertical rotation state. If the tablet is in the vertical rotation state, the control part 40 executes a process for changing the orientation of the tablet. Then, the control part 40 specifies, as a capture image to be used in the image inspection process, the capture image of either or both of the front surface and the back surface of the tablet, in which the identification information of the tablet exists, among the capture images. Further, the capture image to be used in the image inspection process is an image including at least the identification information of the tablet among the images captured by the camera 462 or 463. For example, the capture image to be used in the image inspection process is an image obtained by trimming a portion of the capture image, or the capture image itself.
<Step S81> First, in step S81, the control part 40 executes a first matching process of collating the entire capture images with the correct image. Specifically, the control part 40 acquires the coincidence degree by executing the first matching process for the correct image registered in the medicine master in association with the tablet subject to the image inspection process and the capture image of the tablet captured in the packing process. Further, the collation target in the first matching processing may be a pre-set identification information region in which the identification information exists among the correct image and the capture image.
<Step S82> In step S82, the control part 40 determines whether the coincidence degree acquired in step S81 is equal to or greater than a preset first threshold value. The first threshold value is a value which is set in advance for determining that the collation result of the first matching process is coincidence. If it is determined that the coincidence degree is equal to or greater than the first threshold value (S82: Yes), the process proceeds to step S83. If it is determined that the coincidence degree is less than the first threshold value (S82: No), the process proceeds to step S821.
   <Step S821> In step S821, the control part 40 determines whether the coincidence degree acquired in step S81 is equal to or greater than a preset second threshold value. The second threshold value is a value smaller than the first threshold value and is a value which is set in advance for determining that the collation result of the first matching process is non-coincidence. If it is determined that the coincidence degree is equal to or greater than the second threshold value (S821: Yes), the process proceeds to step S822. If it is determined that the coincidence degree is less than the second threshold value (S821: No), the process proceeds to step S 823.
   <Step S822> When the coincidence degree acquired in step S81 is less than the first threshold value and equal to or greater than the second threshold value, it is not possible to clearly decide whether the collation result of the correct image and the capture image is coincidence or non-coincidence. Therefore, in step S822, the control part 40 records the purport that a visual check is needed (a check need) in the storage part 49 as a result of the image inspection process on the tablet.
   <Step S823> On the other hand, when the coincidence degree acquired in step S81 is less than the second threshold value, it is possible to clearly decide that the collation result of the correct image and the capture image is non-coincidence. Therefore, in step S823, the control part 40 records the purport that the collation result is non-coincidence (an error) in the storage part 49 as a result of the image inspection process on the tablet.
<Step S83> In step S83, the control part 40 determines whether another tablet is registered in the medicine master as a similar medicine in association with the tablet subject to the image inspection process. Specifically, the control part 40 determines whether a tablet having the same similar group code as the tablet subject to the image inspection process exists in the medicine master. If it is determined that the similar medicine exists (S83: Yes), the process proceeds to step S84. If it is determined that the similar medicine does not exist (S83: No), the process proceeds to step S86.
   Specifically, as shown in FIG. 55, in the identification information "ABC111" of the correct image P11 and the identification information "ABC 114" of the capture image P12, the regions P21 and P22 exist as a difference between a region of the character "1" and a region of the character "4". However, the difference is extremely small in the entire characters of the identification information, and has a small influence on the coincidence degree. Thus, the coincidence degree may be equal to or greater than the first threshold value. Further, as the first threshold value is set at a larger value, it is highly likely that the collation result of the first matching process is heavily influenced by an extremely small noise or the like and thus the collation result is non-coincidence. In this regard, in the inspection assistance system 1 according to the present embodiment, the control part 40 determines in step S83 whether a similar medicine exists with respect to the tablets subject to the image inspection process. If the similar medicine exists, a more detailed image inspection process is executed in subsequent step S84. Therefore, it is possible to set the first threshold value to low extent such that the collation result is not heavily affected by an extremely small noise or the like.
<Step S84> In step S84, the control part 40 executes a second collation process of collating only the similar portion of the capture image with the correct image. Specifically, the control part 40 sets a region corresponding to the similar portion in each of the correct image and the capture image as a target of the collation range, and executes a pre-set second matching process. In the second matching process, after the similar portion of the capture image is binarized, a difference between the similar portion in the capture image and the similar portion in the correct image is detected, and a proportion of a coincident region, which excludes a portion in which the difference is generated, from the similar portions of the collation range, is acquired as the coincidence degree. For example, a value obtained by subtracting the number of pixels, which are detected as the difference in the similar portions, from the number of pixels of the similar portion in the correct image is calculated, and the ratio of the calculated value to the number of the pixels of the similar portion in the correct image is calculated as the coincidence degree. That is to say, in the second matching process, dissimilar to the first matching process, in case where extra black pixels are included in the capture image even when the most of the similar portion of the correct image is included in the similar portion of the capture image, the coincidence degree is calculated in view of the difference. Therefore, the coincidence degree may decrease and the collation result may be non-coincidence thereby. Further, in case where there is a plurality of similar medicines associated with the tablet subject to the image inspection process, a plurality of similar portions may be set with respect to the tablet. In this case, the coincidence degree between the capture image and the correct image is acquired with respect to each of the similar portions by the second matching process.
   Specifically, in the above-described example, only the region (the enlarged range in FIG. 55), which corresponds to the first place character "1" set in advance as the similar portion of the identification information "ABC111" of the tablet to be subject to the image inspection process, become a target of the second matching process. Since the target region of the second matching process is limited in the above-described manner, the coincidence degree which largely reflects the influence of the difference in the similar portions is detected. Further, in case where a tablet having such identification information "ABO111" is registered as a similar medicine in the medicine master in addition to the tablet having an identification information "ABC114", the second matching process of collating the capture image with the correct image is executed with respect to a region corresponding to "C" in the capture image.
<Step S85> In step S85, the control part 40 determines whether the coincidence degree obtained by the first matching process executed in step S84 is equal to or greater than a preset third threshold value. The third threshold value is a value set in advance for determining that the collation result of the similar portions is coincidence. For example, the third threshold value may be a value equal to the first threshold value, or may be a value equal to or greater than the first threshold value or less than the first threshold value.
   If it is determined that the coincidence degree is equal to or greater than the third threshold value (S85: Yes), the process proceeds to step S86. If it is determined that the coincidence degree is less than the third threshold value (S85: No), the process proceeds to step S822. Further, when it is determined that the coincidence degree is less than the third threshold value (S85: No), the process may proceed to step S823. Further, in case where the second matching process has been executed with respect to a plurality of the similar portions in the step S84, it is determined whether the coincidence degrees in all the similar portions are equal to or greater than the third threshold value. If all the coincidence degrees are equal to or greater than the third threshold value, the process proceeds to step S86.
<Step S86> In step S86, the control part 40 determines that the collation result in the image inspection process is coincidence. The control part 40 records "OK" as the image inspection result in association with a tablet of the prescription data to be subject to the image inspection process.

As described above, in the image inspection process according to the present embodiment, regarding the tablet for which a similar medicine exists, in the image inspection process, the second matching process is executed with respect to the similar portion after the first matching process is executed with respect to the entire capture image and the entire normal image. Thus, even if the difference between the capture image and the correct image is small as a whole, it is possible to determine the suitability of the tablet according to the magnitude of the difference in the similar portions. Accordingly, it is possible to enhance the inspection accuracy in the image inspection process on the tablet for which a similar medicine exists. Further, in the image inspection process, regarding the tablet for which a similar medicine does not exist, only the first matching process is executed with respect to the entire capture image and the entire correct image. Thus, regarding the tablet for which a similar medicine does not exist, it is possible to reduce a process load of the image inspection process or to shorten the time required for the image inspection process.

There has been described the case where, in step S81, the first matching process is executed with respect to the entire capture image. However, like step S84, the second matching process may be executed with respect to the entire capture image. That is to say, in step S81, it is conceivable that the coincidence degree is detected in view of the difference between the entire capture image and the entire correct image. Likewise, there has been described the case where, in step S84, the second matching process is executed with respect to the similar portion in the capture image. However, like step S81, the first matching process may be executed with respect to the similar portion in the capture image. That is to say, in step S84, it is conceivable that the amount of overlapped portion in the similar portion of each of the capture image and the correct image is detected as the coincidence degree.

Further, it is also conceivable that, instead of the image inspection process (FIG. 56), the second matching process is executed only for the entire capture image and the entire correct image. Thus, a difference between the entire capture image and the entire correct image is extracted. Therefore, for example, when compared with the first matching process for detecting the number of black pixels overlapping with the correct image, the acquired coincidence degree is low. This is because, when extra black pixels are included in the capture image, the black pixels are also detected as the difference. Accordingly, by executing the second matching process with respect to the entirety of the capture image and the correct image, the inspection accuracy in the image inspection process is higher than when the first pattern matching process is executed. Further, in this case, for example, when the coincidence degree detected in the second matching process is equal to or greater than the first threshold value, the control part 40 determines that the coincidence degree is normal. When the coincidence degree is less than the second threshold value, the control part 40 determines that the coincidence degree is abnormal. When the coincidence degree is less than the first threshold value and equal to or greater than the second threshold value, the control part 40 determines that a check is needed.

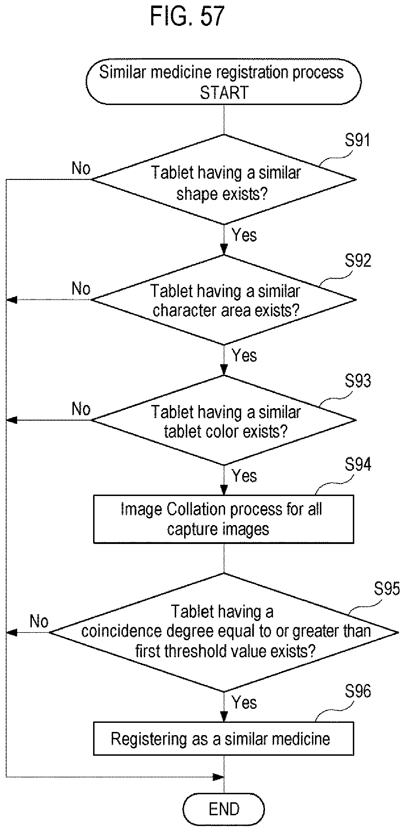

[Similar Medicine Registration Process] As described above, the information such as the presence or absence of a similar medicine and a similar portion is preregistered with respect to each of the tablets in the medicine master. In this regard, it is conceivable that the control part 40 of the tablet packing device 4 has a similarity registration function of automatically registering information on the similar medicine. Specifically, the control part 40 implements the similarity registration function by executing a similar medicine registration process (*see* FIG. 57) which is described below. Hereinafter, an example of the similar medicine registration process executed by the control part 40 is described with reference to FIG. 57. The similar medicine registration process is executed, for example, after step S42 of the information registration process (*see* FIG. 30). Further, it is also conceivable that the information registration process, the similar medicine registration process, and the like are executed by the control part 21 of the server 2.
<Step S91> In step S91, the control part 40 determines whether a tablet (hereinafter referred to as "first similar target tablet"), which is similar in shape such as a height, a width or an area to a tablet subject to registration in the information registration process (hereinafter referred to as "registration target tablet"), exists in the medicine master. If it is determined that the first similar target tablet exists (S91: Yes), the process proceeds to step S92. If it is determined that the first similar target tablet does not exist (S91: No), the similar medicine registration process is terminated. Further, in step S91, it may be determined that a plurality of first similar target tablets exists.
<Step S92> In step S92, the control part 40 determines whether a tablet (hereinafter referred to as "second similar tablet") similar in a character area to the registration target tablet exists among the first similar target tablets. Specifically, the control part 40 acquires the sum of gray values with respect to the capture image and the correct image. When a difference between the sums of gray values falls within a pre-set threshold value, the control part 21 determines that character areas are similar to each other. In this regard, if it is determined that the second similar tablet exists (S92: Yes), the process proceeds to step S93. If it is determined that the second similar tablet does not exist (S92: No), the similar medicine registration process is terminated. Further, in step S92, it may be determined that a plurality of second similar target tablets exists.
<Step S93> In step S93, the control part 40 determines whether a tablet (hereinafter referred to as "third similar tablet") similar in a tablet color to the registration target tablet exists among the second similar target tablets. Specifically, the control part 40 acquires the sums of a R value, a G value and a B value with respect to the capture image and the correct image. When a difference between the sums of the R value, the G value and the B value falls within a pre-set threshold value, the control part 40 determines that tablet colors are similar to each other. In this regard, if it is determined that the tablet colors are similar (S93: Yes), the process proceeds to step S94. If it is determined that the tablet colors are not similar (S93: No), the similar medicine registration process is terminated. Further, in step S93, it may be determined that a plurality of third similar target tablets exists.
<Step S94> In step S94, the control part 40 executes a collation process with respect to the entirety of the correct image of the third similar target tablet and the entirety of the capture image of the registration target tablet. Specifically, the control part 40 executes the first matching process or the second matching process as the collation process with respect to the correct image of the third similar target tablet and the capture image of the registration target tablet, thereby acquiring the coincidence degree. In this regard, the collation process has the same contents as the process executed in step S81 of the image inspection process among the first matching process and the second matching process.
<Step S95> Then, in step S95, the control part 40 determines whether a tablet (hereinafter referred to as "fourth similar target tablet"), whose result of the collation process in step S94 is coincidence, exists among the third similar target tablets. Specifically, it is determined whether the coincidence degree corresponding to each of the third similar target tablets is equal to or greater than the first threshold value. If it is determined that the fourth similar target tablet exists (S95: Yes), the process proceeds to step S96. If it is determined that the fourth similar target tablet does not exist (S95: No), the similar medicine registration process is terminated. That is to say, in steps S94 to S95, it is decided whether the collation result in steps S81 to S82 of the image inspection process (*see* FIG. 56) is decided to be coincidence. Further, in step S95, it may be determined that a plurality of fourth similar target tablets exists.
<Step S96> In step S96, the control part 40 registers the registration target tablet and one or a plurality of the fourth similar target tablets as similar medicines in the medicine master. Specifically, the control part 40 registers one or a plurality of the similar group codes attached to one or a plurality of fourth similar target tablets as the similar group code of the registration target tablet. That is to say, a tablet, whose collation result in steps S81 to S82 of the image inspection process (*see* FIG. 56) is decided to be coincidence, is registered as the similar medicine of the registration target tablet. Further, the control part 40 registers a similar portion of the registration target tablet similar to each of the similar medicines, for example, in response to a user's manipulation in the step S96. Alternatively, the similar portion is automatically registered by the control part 40, based on the result of the collation process of step S94.

As described above, by executing the similar medicine registration process with the control part 40, the similar medicine of each tablet is automatically registered in the medicine master without requiring a user's manipulation. Thus, the burden of a registration task conducted by a user is reduced.

[Fourth Embodiment] In the present embodiment, descriptions are made as to another example of the enlarged screen D341 (*see* FIG. 39Aor 40) that can be displayed when the result of the automatic inspection process is displayed as described above. As described in the third embodiment, there is a case where the second matching process for detecting the difference between the capture image and the correct image may be executed in the tablet packing device 4. In this case, the control part 40 transmits information on the difference between the capture image and the correct image to the server 2 together with the result of the image inspection process and the capture image. Further, the control part 21 can execute the second matching process with respect to the capture image and the correct image in the image inspection process to acquire a difference between the capture image and the correct image. Further, in the server 2, the control part 21 displays the difference between the capture image and the correct image when displaying the result of the image inspection process. Specifically, it is conceivable that the control part 21 superimposes the enlarged image P343, which is displayed as the enlarged image of the capture image, on the enlarged screen D341, and displays the difference between the capture image and the correct image in a visually recognizable manner. That is to say, in the enlarged screen D341, the control part 40 displays the difference at the capture image side rather than the correct image side.

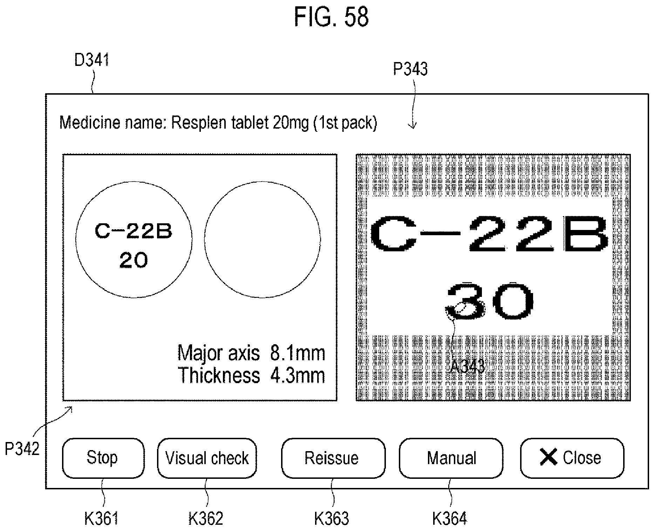

More specifically, in the second matching process, an extra region, which is a region of the capture image where the black pixels not included in the correct image exist, and a deficient region which is a region of the capture image where the black pixels included in the correct image do not exist, are individually detected. Then, the control part 40 outputs the extra region and the deficient region as a difference between the capture image and the correct image. Thus, in the server 2, as the difference between the capture image and the correct image, the extra region and the deficient region are displayed at the enlarged image P343 of the enlarged screen D341 by the control part 21. In this regard, FIG. 58 shows another example of the enlarged screen D341.

As shown in FIG. 58, in the enlarged image P343, the control part 21 highlights and displays a difference area A343, which includes the extra region and the deficient region, in such a manner that it can be distinguished from other areas. In particular, in the enlarged screen D341 shown in FIG. 58, a region, which includes the identification information of the tablet among the capture image of the tablet, is enlarged and displayed. For example, it is conceivable that, in the enlarged image P343, an outer edge of the difference area A343 is colored in a specific color such as a red color. Further, it is also conceivable that the difference area A343 is displayed by being filled with in a specific color. Further, it is conceivable that, at the difference area A343, the extra region and the deficient region are displayed in an individually-distinguishable manner. For example, it is conceivable that the extra region and the deficient region are displayed with edges of different colors or are filled with different colors. Further, the display of the difference area A343 may be highlighted by flickering or the like.

In particular, it is conceivable that, only when the result of the image inspection process (*see* FIG. 56) is an error (step S823) or a check need (step S822), the control part 21 displays the deficient region and the extra region in the enlarged screen D341. Thus, when the result of the image inspection process is an error or a check need, a user can easily grasp the reasons thereof by seeing the enlarged image P341 in the enlarged screen D341.

Further, when the result of the image inspection process is an error, the correct image and the capture image are obviously different from each other. However, when the result of the image inspection process is a check need, the difference between the correct image and the capture image may be small. Therefore, it is conceivable that the control part 21 does not display the deficient region and the extra region when the result of the image inspection process as an error, but displays the deficient region and the extra region in the enlarged screen D341 only when the result of the image inspection process is a check need. Thus, when the result of the image inspection process is a check need, a user can easily grasp the reason thereof from the enlarged screen D341. In other cases, unnecessary information is not displayed in the enlarged screen D341. Further, the present embodiment has been described with respect to the case where, in the server 2, the difference between the capture image and the correct image is displayed by the control part 21. However, it is conceivable that, in the tablet packing device 4, the control part 40 displays the difference between the capture image and the correct image in the manipulation display part 48.

[Fifth Embodiment] In the present embodiment, descriptions are made as to variation examples of the information registration process (*see* FIG. 30) and the image inspection process (*see* FIGS. 34 and 56).

As described above, the capture image obtained by capturing a tablet with the camera 462 or 463 is registered as a correct image of the tablet in the medicine master. Further, when the identification information of the tablet is an engraving, for example, unevenness of light and shade, which is caused by the position of a shadow in the tablet on the tablet rotation part 441 to be captured by the camera 462 or 463 or the unevenness of the engraving, may vary depending on the orientation of the tablet which is viewed from the camera 462 or 463. Therefore, there is a concern that the difference in the shadow position or the unevenness of light and shade included in the correct image affects the accuracy of the image inspection process. Further, the shadow in the tablet may be generated, for example, when there is a height difference between a pair of the rotating rollers 100 of the tablet rotation part 441. For example, the shadow in the tablet may be generated on the rotating roller 100 having a short distance from the camera 462 or 463 among a pair of the rotating rollers 100. Particularly, when the identification information of the tablet is an engraving, there is a concern that that a shadow is generated in the tablet by the rotating roller 100, since light is irradiated from the lateral side or the oblique upper side toward the tablet on the tablet rotation part 441 by the illumination device 468.

In this regard, in the tablet packing device 4, it is conceivable that a plurality of correct images can be registered in the medicine master with respect to each tablet in the information registration process (*see* FIG. 30). Specifically, each of the correct images is an image which is captured in a plurality of states where the orientation of the tablet viewed from the camera 462 or 463 is different, namely in a plurality of states where the shadow position or the unevenness of light and shade is different.

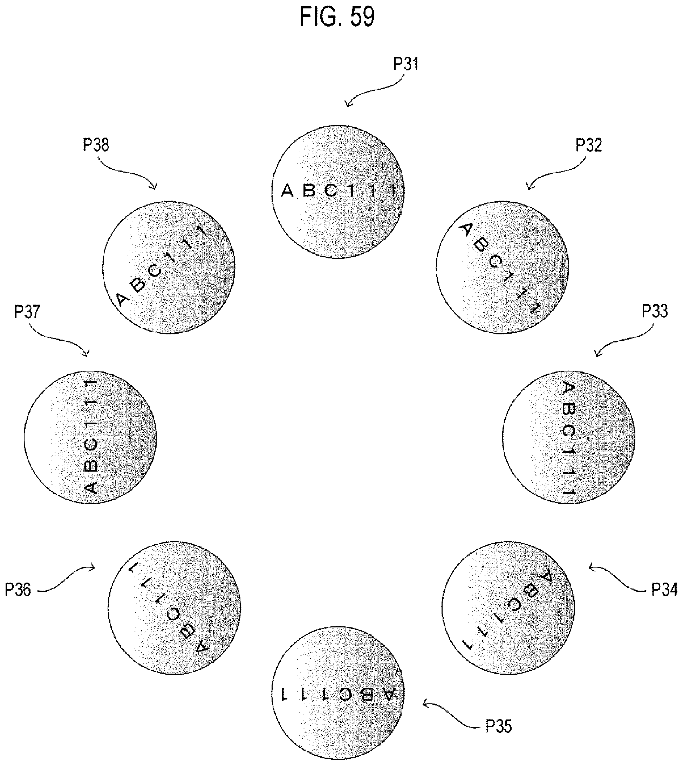

In this regard, FIG. 59 shows correct images P31 to P38 which are examples of the plurality of correct images. More specifically, the respective correct images P31 to P38 are registered in the medicine master as the correct images which correspond to the cases where angles of the identification information of the tablet are 0°, 45°, 90°, 135°, 180°, 225°, 270° and 315°. In the correct images P31 to P38, a shadow is formed in the right direction in FIG. 59. The positional relationship between the identification information "ABC 111" and the shadow in each of the correct images P31 to P38 is different. Although not shown in FIG. 59, when the identification information of the tablet is an engraving, the brightness and darkness of the engraving may differ for each of the correct images P31 to P38.

In the tablet packing device 4 configured as described above, the collation between the capture image and the correct image in the image inspection process is performed by using a correct image which has an angle close to the capture image among the correct images. Specifically, when the image inspection process is executed, the control part 40 detects the coincidence degree of the capture image with one pre-set specific correct image (for example, the correct image P31) among the correct images while gradually rotating the capture image by 360°, and detects a rotation angle of the capture image available when the coincidence degree is highest, as a specific angle indicating an inclination angle of the capture image. Then, the control part 40 specifies a correct image corresponding to the angle closest to the specific angle among the correct images, and executes the image inspection process by using such a correct image and the capture image. Thus, the influence of the shadow or the unevenness of light and shade included in the correct image is reduced and, therefore, the inspection accuracy in the image inspection process is enhanced.

Further, the control part 40 performs the collation by using the correct image corresponding to the angle closest to the specific angle among a plurality of the correct images. Therefore, when compared with a case where the collation between each of the correct images and the capture image is individually performed, a process load is reduced or a process time is shortened. Further, as another embodiment, it is conceivable that the control part 40 individually acquires the coincidence degrees between the respective correct images and the capture image, and that the control part 40 determines that the collation result is coincidence when all the coincidence degrees are equal to or greater than the first threshold value.

Further, when selecting the correct image corresponding to the angle closest to the specific angle from a plurality of the correct images, it is conceivable that the control part 40 selects a plurality of the correct images. Specifically, it is conceivable that, depending on angular intervals of a plurality of the correct images, the correct image corresponding to the angle approximate to the specific angle does not exist. Therefore, it is conceivable that the control part 40 selects, for example, two or three correct images closest to the specific angle from a plurality of the correct images. In this case, the control part 40 individually acquires the coincidence degrees between the respective selected correct images and the capture image. If all the coincidence degrees are equal to or greater than the first threshold value, the control part 40 determines that the collation result is coincidence. Thus, when compared with a case where the collation between all the correct images and the capture image is individually performed, it is possible to suppress the influence of the shadow or the unevenness of light and shade included in the correct image while reducing a process load. Thus, it is possible to enhance the inspection accuracy of the image inspection process.

Further, it is conceivable that, when the difference between the specific angle and the image capturing angle corresponding to the correct image closest to the specific angle is equal to or greater than a pre-set threshold value, the control part 40 selects a plurality of the correct images, and that, when the difference less than the threshold value, the control part 40 selects one of the correct images.

[Sixth Embodiment] In the present embodiment, similar to the fifth embodiment, descriptions are made as to variation examples of the information registration process *(see* FIG. 30) and the image inspection process (*see* FIGS. 34 and 56).

As described above, the capture image obtained by capturing a tablet with the camera 462 or 463 is registered as a correct image of the tablet in the medicine master. Further, in the image inspection process, a matching process is performed between the capture image obtained by capturing the tablet with the camera 462 or 463 and the correct image. On the other hand, in the capture image captured by the camera 462 or 463, a character thickness of the identification information included in the capture image may be changed due to, for example, the coincidence accuracy of a focus at the time of capturing the capture image. Further, when the identification information of the tablet is an engraving, the character thickness of the identification information may be changed due to the orientation of the tablet viewed from the camera 462 or 463. Therefore, there is a concern that the difference in the character thicknesses of the identification information affects the accuracy of the image inspection process.

In this regard, in the tablet packing device 4, it is conceivable that, in the information registration process (*see* FIG. 30), a plurality of the correct images can be registered with respect to each tablet in the medicine master. Specifically, each of the correct images is an image in which the character thickness of the identification information of the tablet included in the capture image is changed with respect to the capture image captured by the camera 462 or 463 and registered as the correct image.

In this regard, FIG. 60 shows the correct images P41 to P45 which are examples of a plurality of the correct images. As shown in FIG. 60, the control part 40 uses the capture image of the tablet as a correct image P41, and generates a correct image P42 in which the character thickness of the identification information in the correct image P41 is increased by two pre-set stages and a correct image P43 in which the character thickness of the identification information is increased by one pre-set stage. For example, the character thickness in the correct image P42 is four times as large as that in the correct image P41, and the character thickness in the correct image P43 is twice as large as that in the correct image P41. Further, the control part 40 uses the capture image as the correct image P41, and generates a correct image P44 in which the character thickness of the identification information in the correct image P41 is reduced by two pre-set stages and a correct image P45 in which the character thickness of the identification information is reduced by one pre-set stage. For example, the character thickness in the correct image P44 is 1/4 times as small as that in the correct image P41, and the character thickness in the correct image P45 is 1/2 times as small as that in the correct image P41. Then, the control part 40 registers the correct images P41 to P45 in the medicine master as the correct images of the tablet.

In particular, it is conceivable that the control part 40 registers, in the medicine master, a plurality of the corrected correct images which are obtained as a result of executing an illumination cancellation process, a contrast correction process, a character region extraction process and the like with respect to a plurality of the correct images. Specifically, in the illumination cancellation process, the influence of illumination at the time of capturing the capture image is removed by executing a fitting process with respect to a plurality of the correct images so that a pre-set change in luminance occurs. For example, in the fitting process, a locally bright region or a locally dark region is suppressed from a plurality of the correct images. In the contrast correction process, a range between a minimum value and a maximum value of luminance in the plurality of correct images is expanded to a range of from 0 to 255, thereby emphasizing the contrast between characters and a background. Further, in the character region extraction process, a black region is extracted from a plurality of the correct images by means of a discriminant analysis method (Otsu's threshold method). The black region is extracted as a character region of the identification information.

In the tablet packing device 4 configured as described above, in the image inspection process, the collation of the capture image and the correct image is performed using a plurality of the correct images. Specifically, in the image inspection process, the control part 40 individually executes a matching process between each of the correct images and the capture image. Then, the control part 40 specifies the highest coincidence degree detected in each of the matching processes as the collation result of the capture image, and decides the suitability of the tablet according to the coincidence degree. Thus, the influence of the difference in the character thickness of the identification information is suppressed and the inspection accuracy of the image inspection process is enhanced. Further, since a plurality of the correct images are registered in the medicine master in advance, an increase in a process load at the time of the image inspection process is suppressed.

Further, the present embodiment has been described by way of example where a plurality of the correct images having different character thicknesses are registered in the medicine master as the correct images. However, the present invention is not limited thereto. For example, it is conceivable that one correct image is registered in the medicine master, and that the control part 40 creates a plurality of capture images having different character thicknesses of the capture images from the capture images. In this case, it is conceivable that the control part 40 individually executes a matching process between each of the capture images with the correct image registered in the medicine master, specifies the highest coincidence degree as the collation result of the capture image, and decides the suitability of the tablet according to the coincidence degree.

Further, it is conceivable that a plurality of the correct images having different character thicknesses are registered in the medicine master as the correct image, and that the control part 40 generates a plurality of capture images having different character thicknesses in the capture images from the capture images. In this case, it is conceivable that the control part 40 individually executes a matching process between each of the capture images with each of the correct images, specifies the highest coincidence degree as the collation result of the capture image, and decides the suitability of the tablet according to the coincidence degree.

[Summary of the Invention] Hereinafter, the summary of the invention extracted from the respective embodiments described above are mentioned as supplementary notes. Further, it is possible to select and arbitrarily combine the configurations and processing functions described below.
[Supplementary Note 1] An inspection assistance system, comprising: an inspection display processing part configured to display, per unit of packing, a capture image of a tablet which is captured before the tablet dispensed from one or both of a tablet cassette and a manual distribution unit based on prescription data is packed into a packing material in a tablet packing device, and is configured to display a result of an inspection process which is executed based on the prescription data and an identification information of the tablet read from the capture image of the tablet.
   According to Supplementary Note 1, it is possible to assist a pharmacist's inspection task. More specifically, the capture image of the tablet is displayed per unit of packing and the result of the inspection process is displayed, thereby improving the efficiency of a pharmacist's inspection task.
[Supplementary Note 2] The inspection assistance system of Supplementary Note 1, wherein, when the result of the inspection process is an error, the inspection display processing part distinguishably displays the capture image of the tablet which causes the error. Thus, a pharmacist can easily specify and check the capture image of the tablet which causes the error.
[Supplementary Note 3] The inspection assistance system of Supplementary Note 1 or 2, further comprising: a list display processing part configured to, when the result of the inspection process is an error, display a cause of the error by a list together with a dosing date and a dosing time of the tablet causing the error. Thus, a pharmacist can easily grasp the cause of the error and the dosing date and the dosing time of the tablet by referring to the displayed list.
[Supplementary Note 4] The inspection assistance system of any one of Supplementary Notes 1 to 3, wherein, in an arrangement format in which dosing dates are displayed in one of a column and a row and dosing times are displayed in the other of the column and the row, the inspection display processing part arranges and displays, at display locations corresponding to respective combinations of the dosing dates and the dosing times, the capture images of the tablet of a unit of packing corresponding to the combinations in a display order predetermined in a direction parallel to an arrangement order of the dosing times. Thus, since the capture images of the same type of the tablet are arranged in the direction of the arrangement of the dosing dates, a pharmacist can easily check the suitability of each capture image of the tablet.
[Supplementary Note 5] The inspection assistance system of any one of Supplementary Notes 1 to 3, wherein, in an arrangement format in which tablet names are displayed in one of a column and a row and packing orders are displayed in the other of the column and the row, the inspection display processing part displays the capture images of the tablet corresponding to respective combinations of the tablet names and the packing orders. Thus, since the capture images of the same type of the tablet are arranged in the direction of the arrangement of the packing orders, a pharmacist can easily check the suitability of each capture image of the tablet.
[Supplementary Note 6] The inspection assistance system of any one of Supplementary Notes 1 to 5, wherein the inspection display processing part displays, with respect to each tablet, a plurality of capture images which include at least an original image whose the identification information of the tablet is read and correspond to different outer peripheral surfaces of the tablet captured with respect to each tablet in the tablet packing device. Thus, a pharmacist can determine the suitability of the tablet by referring to a plurality of the capture images of the tablet, for example, when it is difficult to determine the suitability of the tablet with only one capture image of the tablet.
[Supplementary Note 7] The inspection assistance system of any one of Supplementary Notes 1 to 6, wherein, when the result of the inspection process is an error, the inspection display processing part is capable of displaying, with respect to each tablet, a plurality of capture images corresponding to different outer peripheral surfaces of the tablet captured with respect to each tablet in the tablet packing device, as a capture image of the tablet causing the error. Thus, a pharmacist can determine the suitability by, for example, referring to a plurality of the capture images of the tablet.
[Supplementary Note 8] The inspection assistance system of Supplementary Note 7, further comprising: a cancellation processing part capable of cancelling the error on the tablet in response to a user's manipulation made after the capture image of the tablet causing the error is displayed. Thus, for example, when it can be checked by checking the capture images that there is no problem, a pharmacist makes the user's manipulation. Accordingly, even if an error occurs in the inspection process on the prescription data, it is possible to normally terminate the inspection of the prescription data.
[Supplementary Note 9] The inspection assistance system of Supplementary Note 8, wherein the cancellation processing part cancels the error and records, in association with the prescription data, an identification information of a user available when the user's manipulation is made. Thus, it is possible to output later the identification information of a user who cancels the error.
[Supplementary Note 10] The inspection assistance system of any one of Supplementary Notes 1 to 9, wherein the inspection display processing part displays a registration image preregistered as a correct image of the tablet, together with the capture image of the tablet. Thus, a pharmacist can easily determine the suitability of the capture image of the tablet by comparing the capture image with the registration image.
[Supplementary Note 11] The inspection assistance system of any one of Supplementary Notes 1 to 10, wherein the inspection display processing part individually displays the result of the inspection process with respect to each tablet included in the prescription data. Thus, a pharmacist can easily grasp, for example, the tablet which causes the error as a result of the inspection process.
[Supplementary Note 12] The inspection assistance system of any one of Supplementary Notes 1 to 11, wherein the inspection display processing part displays the capture image of the tablet by aligning an orientation of a character of the identification information of the tablet. Thus, a pharmacist can easily recognize the identification information of the tablet included in the capture image of the tablet.
[Supplementary Note 13] The inspection assistance system of any one of Supplementary Notes 1 to 12, wherein the inspection assistance system is connected to a plurality of medicine preparation devices used for a medicine preparation processes executed based on the prescription data, and wherein the inspection assistance system further comprises: a manipulation display processing part configured to display a manipulation section for individually re-executing a portion or all of the medicine preparation processes executed by the medicine preparation devices based on the prescription data, in a first display screen in which an inspection result of a medicine prepared by the medicine preparation devices is displayed or in a second display screen which is displayed after a predetermined manipulation is made on the first screen; and a re-execution processing part configured to, in response to a user's manipulation on the manipulation section displayed by the manipulation display processing part, cause the medicine preparation devices to re-execute a portion or all of the medicine preparation processes executed by the medicine preparation devices. Thus, in the inspection assistance system, a pharmacist can cause each of the medicine preparation devices to individually re-execute a portion or all of the medicine preparation processes. Thus, the efficiency of a pharmacist's task is improved.
[Supplementary Note 14] The inspection assistance system of Supplementary Note 13, wherein the manipulation display processing part causes the first display screen, in which the result of the inspection process is displayed by the inspection display processing part, to display the manipulation section for re-executing a portion or all of packing processes executed by the tablet packing device, and wherein the re-execution processing part causes the tablet packing device to re-execute a portion of the entirety of the packing processes executed by the tablet packing device, in response to a user's manipulation on the manipulation section displayed by the manipulation display processing part.
[Supplementary Note 15] The inspection assistance system of Supplementary Note 14, wherein the manipulation display processing part displays two or three of a first manipulation section for re-executing all the packing processes, a second manipulation section for re-executing only a portion of the packing processes which is decided to be an error in the inspection process, and a third manipulation section for re-executing a portion of the packing processes selected by a user's manipulation. Thus, a pharmacist can selectively use two or three of the first manipulation section, the second manipulation section and the third manipulation section and can cause the tablet packing device to arbitrarily execute a portion or all of the packing processes.
[Supplementary Note 16] An inspection assistance system, comprising: a first tablet packing device capable of executing a packing process of putting a tablet into a packing material based on prescription data and capable of executing a predetermined image inspection process of inspecting a suitability of the packing process; a second tablet packing device capable of executing the packing process, wherein the image inspection process is not executable or a function of the image inspection process is set invalid; and a selection processing part configured to cause the first tablet packing device to execute the packing process based on the prescription data, when it is determined based on at least one of contents of the prescription data and contents of the packing process that a pre-set first priority condition is satisfied.
[Supplementary Note 17] The inspection assistance system of Supplementary Note 16, wherein the first tablet packing device includes: an image capturing part configured to acquire a capture image of the tablet by capturing an image of the tablet before the tablet is put into the packing material; and an image inspection processing part configured to acquire an identification information of the tablet from the capture image of the tablet captured by the image capturing part and configured to execute an image inspection process of collating the identification information of the tablet and the prescription data.
[Supplementary Note 18] The inspection assistance system of Supplementary Note 17, wherein the image capturing part is configured to capture a plurality of capture images corresponding to different outer peripheral surfaces of the tablet with respect to each tablet, and wherein the image inspection processing part is configured to, according to an area of the tablet included in the capture image, specify the capture image which is used for acquiring the identification information of the tablet, among the plurality of capture images.
[Supplementary Note 19] The inspection assistance system of any one of Supplementary Notes 16 to 18, wherein the second tablet packing device includes: a tablet number detection part configured to detect the number of tablets before or after the tablets are put into the packing material; and a count inspection processing part configured to execute a count inspection process of collating the number of tablets detected by the tablet number detection part with the prescription data.
[Supplementary Note 20] The inspection assistance system of any one of Supplementary Notes 16 to 19, wherein each of the first tablet packing device and the second tablet packing device includes a tablet cassette and a manual distribution unit which are used for supplying the tablet to be packed by the packing process, and wherein the first priority condition includes at least one of a condition that the manual distribution unit is used in the packing process, a condition that the number of tablets to be packed by the packing process is equal to or greater than a pre-set threshold value, and a condition that the tablet to be packed by the packing process is a pre-set high-risk medicine.
[Supplementary Note 21] The inspection assistance system of Supplementary Note 20, wherein the selection processing part is configured to, when the first priority condition is not satisfied, select the first tablet packing device or the second tablet packing device according to a pre-set second priority condition and cause the packing process to be executed based on the prescription data, and wherein the second priority conditions includes at least one of a condition of selecting a device which has a large number of types of the tablets received in the tablet cassette among tablets included in the prescription data, a condition of selecting a device which has a small number of the packing processes which stands by currently, or a condition of selecting device arbitrarily designated by a user.
[Supplementary Note 22] The inspection assistance system of any one of Supplementary Notes 16 to 20, wherein the selection processing part is configured to, when the first priority condition is not satisfied, select the first tablet packing device or the second tablet packing device according to pre-set second priority condition and cause the packing process to be executed based on the prescription data.
[Supplementary Note 23] An inspection assistance system, comprising: an image inspection processing part configured to acquire an identification information of a tablet from a capture image of the tablet captured before the tablet is put into a packing material based on prescription data in a tablet packing device and configured to execute an image inspection process of collating the identification information of the tablet with the prescription data; and a count inspection processing part configured to execute a count inspection process of collating the prescription data with the number of tablets detected at a downstream side of a tablet image-capturable position in a dispensing path of the tablet.
[Supplementary Note 24] An inspection assistance system, comprising: an image inspection processing part configured to execute an image inspection process of collating a capture image of a tablet, which is captured before the tablet is put into a packing material based on prescription data in a tablet packing device, with a correct image preregistered in association with a type of the tablet included in the prescription data; and an inspection display processing part capable of displaying a difference between the correct image and an identification information of the tablet detected in the image inspection process executed by the image inspection processing part.
[Supplementary Note 25] The tablet packing device of Supplementary Note 24, wherein the image inspection processing part is configured to execute a first collation process of performing image collation between the capture image of the tablet and the correct image, and wherein the image inspection processing part is configured to, when a collation result obtained by the first collation process is coincidence and when a similar medicine similar to the tablet subject to the image inspection process exists, execute a second collation process of performing image collation between a region of the capture image, which corresponds to a pre-set similar portion similar to the similar medicine, and a region of the correct image which corresponds to the similar portion.
[Supplementary Note 26] A tablet packing device capable of executing a packing process of putting a tablet into a packing material based on prescription data, comprising: a medicine introduction part configured to guide the tablet to the packing material; and an image capturing part configured to, when the tablet is put into the packing material, capture an image of an image capturing range including the medicine introduction part, the tablet and the packing material.
[Supplementary Note 27] A tablet packing device capable of executing a packing process of putting a tablet into a packing material based on prescription data, comprising: a tablet rotation part configured to rotate the tablet; an image capturing processing part configured to execute an image capturing process of capturing, a plurality of times, the tablet rotated by the tablet rotation part; and a registration processing part configured to acquire an identification information of the tablet from a capture image, which satisfies a pre-set condition, among a plurality of capture images of the tablet captured by the image capturing processing part and configured to register the identification information of the tablet as a collation source data corresponding to a type of the tablet.
[Supplementary Note 28] The tablet packing device of Supplementary Note 27, further comprising: an image inspection processing part configured to execute an image inspection process of collating the identification information of the tablet, which is acquired from the capture image of the tablet captured in the packing process, with the collation source data corresponding to the type of the tablet included in the prescription data.
[Supplementary Note 29] The tablet packing device of Supplementary Note 28, wherein the identification information of the tablet is an image of a partial or entire region of the capture image of the tablet, wherein the collation source data is a correct image preregistered in association with the type of the tablet, and wherein the image inspection processing part is configured to collate the identification information of the tablet with the correct image.
[Supplementary Note 30] The tablet packing device of Supplementary Note 29 further comprising: an image capturing part including a first image capturing unit and a second image capturing unit which are capable of capturing an image of the tablet rotated by the tablet rotation part, wherein the first image capturing unit is provided in association with a case where the identification information of the tablet is an engraving and a second image capturing unit is provided in association with a case where the identification information of the tablet is a print; and a specification processing part configured to specify, in response to a user's manipulation, whether the identification information of the tablet registered by the registration processing part is an engraving or a print, wherein the image inspection processing part is configured to execute the image inspection process by using the capture image of the tablet captured by means of the first image capturing unit or the second image capturing unit corresponding to one of the engraving and the print which are specified by the specification processing part.
[Supplementary Note 31] The tablet packing device of Supplementary Note 29, further comprising: further comprising: an image capturing part including a first image capturing unit and a second image capturing unit which are capable of capturing an image of the tablet rotated by the tablet rotation part, wherein the first image capturing unit is provided in association with a case where the identification information of the tablet is an engraving and a second image capturing unit is provided in association with a case where the identification information of the tablet is a print; and a specification processing part configured to specify, in response to a user's manipulation, whether the identification information of the tablet registered by the registration processing part is an engraving or a print, wherein the image capturing processing part is configured to, at a time of capturing the capture image used when the collation source data is registered by the registration processing part, capture an image of the tablet by selecting one of the first image capturing unit and the second image capturing unit according to a specification result obtained by the specification processing part.
[Supplementary Note 32] The tablet packing device of Supplementary Note 29, further comprising: an image capturing part capable of capturing an image of the tablet rotated by the tablet rotation part; a vibration application part configured to apply vibration to the tablet; a state decision processing part configured to, based on each of the capture images of the tablet captured by the image capturing part, decide whether the tablet is rotated in a state where a side surface of the tablet directly faces the image capturing part; and a vibration application processing part configured to cause the vibration application part to apply vibration to the tablet when it is determined by the state decision processing part that the tablet is rotated in the state.
[Supplementary Note 33] The tablet packing device of any one of Supplementary Notes 28 to 32, wherein the registration processing part is configured to register, as the collation source data, a capture image selected by a user's manipulation among a plurality of the capture images of the tablet captured by the image capturing processing part.
[Supplementary Note 34] The tablet packing device of any one of Supplementary Notes 29 to 33, wherein the registration processing part is capable of receiving a user's manipulation for designating an upward position in the capture image registered as the collation source data and registering an information indicative of the upward position in the capture image as a portion of the collation source data.
[Supplementary Note 35] The tablet packing device of any one of Supplementary Notes 29 to 34, wherein the registration processing part is capable of registering an information indicative of the upward position in the capture image as a portion of the collation source data, according to a collation result between the capture image and an initial correct image which is stored in a medicine database storing in advance at least a correspondence relationship between the tablet and the initial correct image corresponding to the tablet.
[Supplementary Note 36] The tablet packing device of any one of Supplementary Notes 29 to 35, wherein the registration processing part is capable of receiving a user's manipulation for designating an upward position in the capture image registered as the collation source data and registering a correct image, which is obtained by rotating the capture image such that the upward position in the capture image is a top side, as the collation source data.
[Supplementary Note 37] The tablet packing device of any one of Supplementary Notes 29 to 36, wherein the registration processing part is capable of registering a correct image, which is obtained by rotating the capture image such that the upward position in the capture image is a top side, as the collation source data, according to a collation result between the capture image and an initial correct image which is stored in a medicine database storing in advance at least a correspondence relationship between the tablet and the initial correct image corresponding to the tablet.
[Supplementary Note 38] The tablet packing device of any one of Supplementary Notes 29 to 37, wherein the image inspection processing part is configured to execute a first collation process of performing image collation between the capture image and the collation source data, and wherein the image inspection processing part is configured to, when a collation result obtained by the first collation process is coincidence and when a similar medicine similar to the tablet subject to the image inspection process exists, execute a second collation process of performing image collation between a region of the capture image, which corresponds to a preset similar portion similar to the similar medicine, and a region of the collation source data which corresponds to the similar portion.
[Supplementary Note 39] The tablet packing device of Supplementary Note 38, wherein the first collation process is a first matching process of detecting, as a coincidence degree, a ratio of black pixels included in the capture image to the number of black pixels in the collation source data, and wherein the second collation process is a second matching process of detecting, as a coincidence degree, a proportion of a coincident region excluding a portion in which a difference is generated between the collation source data and the capture image in a collation range of the collation source data and the capture image.

## Claims

1. An inspection assistance system, comprising:
an inspection display processing part configured to display, per unit of packing, a capture image of a tablet which is captured before the tablet dispensed from one or both of a tablet cassette and a manual distribution unit based on prescription data is packed into a packing material in a tablet packing device, and configured to display a result of an inspection process which is executed based on the prescription data and an identification information of the tablet included in the capture image of the tablet.

2. The inspection assistance system of Claim 1, wherein, when the result of the inspection process is an error, the inspection display processing part distinguishably displays the capture image of the tablet which causes the error.

3. The inspection assistance system of Claim 1 or 2, further comprising:
a list display processing part configured to, when the result of the inspection process is an error, display a cause of the error by a list together with a dosing date and a dosing time of the tablet causing the error.

4. The inspection assistance system of any one of Claims 1 to 3, wherein, in an arrangement format in which dosing dates are displayed in one of a column and a row and dosing times are displayed in the other of the column and the row, the inspection display processing part arranges and displays, at display locations corresponding to respective combinations of the dosing dates and the dosing times, the capture images of the tablet of a unit of packing corresponding to the combinations in a display order predetermined in a direction parallel to an arrangement order of the dosing times.

5. The inspection assistance system of any one of Claims 1 to 3, wherein, in an arrangement format in which tablet names are displayed in one of a column and a row and packing orders are displayed in the other of the column and the row, the inspection display processing part displays the capture images of the tablet corresponding to respective combinations of the tablet names and the packing orders.

6. The inspection assistance system of any one of Claims 1 to 5, wherein the inspection display processing part displays, with respect to each tablet, a plurality of capture images which include at least an original image including the identification information of the tablet used in the inspection process and correspond to different outer peripheral surfaces of the tablet captured with respect to each tablet in the tablet packing device.

7. The inspection assistance system of any one of Claims 1 to 6, wherein, when the result of the inspection process is an error, the inspection display processing part is capable of displaying, with respect to each tablet, a plurality of capture images corresponding to different outer peripheral surfaces of the tablet captured with respect to each tablet in the tablet packing device, as a capture image of the tablet causing the error.

8. The inspection assistance system of Claim 7, further comprising:
a cancellation processing part capable of cancelling the error on the tablet in response to a user's manipulation made after the capture image of the tablet causing the error is displayed.

9. The inspection assistance system of Claim 8, wherein the cancellation processing part cancels the error and records, in association with the prescription data, an identification information of a user available when the user's manipulation is made.

10. The inspection assistance system of any one of Claims 1 to 9, wherein the inspection display processing part displays a registration image preregistered as a correct image of the tablet, together with the capture image of the tablet.

11. The inspection assistance system of any one of Claims 1 to 10, wherein the inspection display processing part individually displays the result of the inspection process with respect to each tablet included in the prescription data.

12. The inspection assistance system of any one of Claims 1 to 11, wherein the inspection display processing part displays the capture image of the tablet by aligning an orientation of a character of the identification information of the tablet.

13. The inspection assistance system of any one of Claims 1 to 12, wherein the inspection assistance system is connected to a plurality of medicine preparation devices used for a medicine preparation processes executed based on the prescription data, and
wherein the inspection assistance system further comprises:
a manipulation display processing part configured to display a manipulation section for individually re-executing a portion or all of the medicine preparation processes executed by the medicine preparation devices based on the prescription data, in a first display screen in which an inspection result of a medicine prepared by the medicine preparation devices is displayed or in a second display screen which is displayed after a predetermined manipulation is made on the first screen; and
a re-execution processing part configured to, in response to a user's manipulation on the manipulation section displayed by the manipulation display processing part, cause the medicine preparation devices to re-execute a portion or all of the medicine preparation processes executed by the medicine preparation devices.

14. The inspection assistance system of Claim 13, wherein the manipulation display processing part causes the first display screen, in which the result of the inspection process is displayed by the inspection display processing part, to display the manipulation section for re-executing a portion or all of packing processes executed by the tablet packing device, and
wherein the re-execution processing part causes the tablet packing device to re-execute a portion or all of the packing processes executed by the tablet packing device, in response to a user's manipulation on the manipulation section displayed by the manipulation display processing part.

15. The inspection assistance system of Claim 14, wherein the manipulation display processing part displays two or three of a first manipulation section for re-executing all the packing processes, a second manipulation section for re-executing only a portion of the packing processes which is decided to be an error in the inspection process, and a third manipulation section for re-executing a portion of the packing processes selected by a user's manipulation.

16. An inspection assistance system, comprising:
a first tablet packing device capable of executing a packing process of putting a tablet into a packing material based on prescription data and capable of executing a predetermined image inspection process of inspecting a suitability of the packing process;
a second tablet packing device capable of executing the packing process, wherein the image inspection process is not executable or a function of the image inspection process is set invalid; and
a selection processing part configured to cause the first tablet packing device to execute the packing process based on the prescription data, when it is determined based on at least one of contents of the prescription data and contents of the packing process that a pre-set first priority condition is satisfied.

17. The inspection assistance system of Claim 16, wherein the first tablet packing device includes:
an image capturing part configured to acquire a capture image of the tablet by capturing an image of the tablet before the tablet is put into the packing material; and
an image inspection processing part configured to acquire an identification information of the tablet from the capture image of the tablet captured by the image capturing part and configured to execute an image inspection process of collating the identification information of the tablet with the prescription data.

18. The inspection assistance system of Claim 17, wherein the image capturing part is configured to capture a plurality of capture images corresponding to different outer peripheral surfaces of the tablet with respect to each tablet, and
wherein the image inspection processing part is configured to, according to an area of the tablet included in the capture image, specify the capture image which is used for acquiring the identification information of the tablet among the plurality of capture images.

19. The inspection assistance system of any one of Claims 16 to 18, wherein the second tablet packing device includes:
a tablet number detection part configured to detect the number of tablets before or after the tablets are put into the packing material; and
a count inspection processing part configured to execute a count inspection process of collating the number of tablets detected by the tablet number detection part with the prescription data.

20. The inspection assistance system of any one of Claims 16 to 19, wherein each of the first tablet packing device and the second tablet packing device includes a tablet cassette and a manual distribution unit which are used for supplying the tablet to be packed by the packing process, and
wherein the first priority condition includes at least one of a condition that the manual distribution unit is used in the packing process, a condition that the number of tablets to be packed by the packing process is equal to or greater than a pre-set threshold value, and a condition that the tablet to be packed by the packing process is a pre-set high-risk medicine.

21. The inspection assistance system of Claim 20, wherein the selection processing part is configured to, when the first priority condition is not satisfied, select the first tablet packing device or the second tablet packing device according to a pre-set second priority condition and cause the packing process to be executed based on the prescription data, and
wherein the second priority conditions includes at least one of a condition of selecting a device which has a large number of types of the tablets received in the tablet cassette among tablets included in the prescription data, a condition of selecting a device which has a small number of the packing processes which stand by currently, or a condition of selecting device arbitrarily designated by a user.

22. The inspection assistance system of any one of Claims 16 to 20, wherein the selection processing part is configured to, when the first priority condition is not satisfied, select the first tablet packing device or the second tablet packing device according to pre-set second priority condition and cause the packing process to be executed based on the prescription data.

23. An inspection assistance system, comprising:
an image inspection processing part configured to acquire an identification information of a tablet from a capture image of the tablet captured before the tablet is put into a packing material based on prescription data in a tablet packing device and configured to execute an image inspection process of collating the identification information of the tablet with the prescription data; and
a count inspection processing part configured to execute a count inspection process of collating the prescription data with the number of tablets detected at a downstream side of a tablet image-capturable position in a dispensing path of the tablet.

24. An inspection assistance system, comprising:
an image inspection processing part configured to execute an image inspection process of collating a capture image of a tablet, which is captured before the tablet is put into a packing material based on prescription data in a tablet packing device, with a correct image preregistered in association with a type of the tablet included in the prescription data; and
an inspection display processing part capable of displaying a difference between the correct image and an identification information of the tablet detected in the image inspection process executed by the image inspection processing part.

25. The tablet packing device of Claim 24, wherein the image inspection processing part is configured to execute a first collation process of performing image collation between the capture image of the tablet and the correct image, and
wherein the image inspection processing part is configured to, when a collation result obtained by the first collation process is coincidence and when a similar medicine similar to the tablet subject to the image inspection process exists, execute a second collation process of performing image collation between a region of the capture image, which corresponds to a pre-set similar portion similar to the similar medicine, and a region of the correct image which corresponds to the similar portion.

26. A tablet packing device capable of executing a packing process of putting a tablet into a packing material based on prescription data, comprising:
a medicine introduction part configured to guide the tablet to the packing material; and
an image capturing part configured to, when the tablet is put into the packing material, capture an image of an image capturing range including the medicine introduction part, the tablet and the packing material.

27. A tablet packing device capable of executing a packing process of putting a tablet into a packing material based on prescription data, comprising:
a tablet rotation part configured to rotate the tablet;
an image capturing processing part configured to execute an image capturing process of capturing, a plurality of times, the tablet rotated by the tablet rotation part; and
a registration processing part configured to acquire an identification information of the tablet from a capture image, which satisfies a pre-set condition, among a plurality of capture images of the tablet captured by the image capturing processing part and configured to register the identification information of the tablet as a collation source data corresponding to a type of the tablet.

28. The tablet packing device of Claim 27, further comprising:
an image inspection processing part configured to execute an image inspection process of collating the identification information of the tablet, which is acquired from the capture image of the tablet captured in the packing process, with the collation source data corresponding to the type of the tablet included in the prescription data.

29. The tablet packing device of Claim 28, wherein the identification information of the tablet is an image of a partial or entire region of the capture image of the tablet,
wherein the collation source data is a correct image preregistered in association with the type of the tablet, and
wherein the image inspection processing part is configured to collate the identification information of the tablet with the correct image.

30. The tablet packing device of Claim 29, further comprising:
an image capturing part including a first image capturing unit and a second image capturing unit which are capable of capturing an image of the tablet rotated by the tablet rotation part, wherein the first image capturing unit is provided in association with a case where the identification information of the tablet is an engraving and a second image capturing unit is provided in association with a case where the identification information of the tablet is a print; and
a specification processing part configured to specify, in response to a user's manipulation, whether the identification information of the tablet registered by the registration processing part is an engraving or a print,
wherein the image inspection processing part is configured to execute the image inspection process by using the capture image of the tablet captured by means of the first image capturing unit or the second image capturing unit corresponding to one of the engraving and the print which are specified by the specification processing part.

31. The tablet packing device of Claim 29, further comprising:
an image capturing part including a first image capturing unit and a second image capturing unit which are capable of capturing an image of the tablet rotated by the tablet rotation part, wherein the first image capturing unit is provided in association with a case where the identification information of the tablet is an engraving and a second image capturing unit is provided in association with a case where the identification information of the tablet is a print; and
a specification processing part configured to specify, in response to a user's manipulation, whether the identification information of the tablet registered by the registration processing part is an engraving or a print,
wherein the image capturing processing part is configured to, at a time of capturing the capture image used when the collation source data is registered by the registration processing part, capture an image of the tablet by selecting one of the first image capturing unit and the second image capturing unit according to a specification result obtained by the specification processing part.

32. The tablet packing device of Claim 29, further comprising:
an image capturing part capable of capturing an image of the tablet rotated by the tablet rotation part;
a vibration application part configured to apply vibration to the tablet;
a state decision processing part configured to, based on each of the capture images of the tablet captured by the image capturing part, decide whether the tablet is rotated in a state where a side surface of the tablet directly faces the image capturing part; and
a vibration application processing part configured to cause the vibration application part to apply vibration to the tablet when it is determined by the state decision processing part that the tablet is rotated in the state.

33. The tablet packing device of any one of Claims 28 to 32, wherein the registration processing part is configured to register, as the collation source data, a capture image selected by a user's manipulation among a plurality of the capture images of the tablet captured by the image capturing processing part.

34. The tablet packing device of any one of Claims 29 to 33, wherein the registration processing part is capable of receiving a user's manipulation for designating an upward position in the capture image registered as the collation source data and registering an information indicative of the upward position in the capture image as a portion of the collation source data.

35. The tablet packing device of any one of Claims 29 to 34, wherein the registration processing part is capable of registering an information indicative of the upward position in the capture image as a portion of the collation source data, according to a collation result between the capture image and an initial correct image which is stored in a medicine database storing in advance at least a correspondence relationship between the tablet and the initial correct image corresponding to the tablet.

36. The tablet packing device of any one of Claims 29 to 35, wherein the registration processing part is capable of receiving a user's manipulation for designating an upward position in the capture image registered as the collation source data and registering a correct image, which is obtained by rotating the capture image such that the upward position in the capture image is a top side, as the collation source data.

37. The tablet packing device of any one of Claims 29 to 36, wherein the registration processing part is capable of registering a correct image, which is obtained by rotating the capture image such that the upward position in the capture image is a top side, as the collation source data, according to a collation result between the capture image and an initial correct image which is stored in a medicine database storing in advance at least a correspondence relationship between the tablet and the initial correct image corresponding to the tablet..

38. The tablet packing device of any one of Claims 29 to 37, wherein the image inspection processing part is configured to execute a first collation process of performing image collation between the capture image and the collation source data, and
wherein the image inspection processing part is configured to, when a collation result obtained by the first collation process is coincidence and when a similar medicine similar to the tablet subject to the image inspection process exists, execute a second collation process of performing image collation between a region of the capture image, which corresponds to a pre-set similar portion similar to the similar medicine, and a region of the collation source data which corresponds to the similar portion.

39. The tablet packing device of Claim 38, wherein the first collation process is a first matching process of detecting, as a coincidence degree, a ratio of black pixels included in the capture image to the number of black pixels in the collation source data, and
wherein the second collation process is a second matching process of detecting, as a coincidence degree, a proportion of a coincident region excluding a portion in which a difference is generated between the collation source data and the capture image in a collation range of the collation source data and the capture image.
